# EUROPEAN PATENT APPLICATION

(11) **EP 2 447 371 A1**
(43) Date of publication of application: **02.05.2012**
(21) Application number: 11181687.2
(22) Date of filing: 17.04.2006
(51) Int. Cl.: C12N 15/82, C12N 9/90, C12N 15/29, A01H 1/00

(54) **Plant promoters, terminators, genes, vectors and related transformed plants**

(30) Priority: 15.04.2005 US 671648 P
(62) Divisional of application: 06750562.8
(71) Applicant: Del Monte Fresh Produce Company, Coral Gables, FL 33134 (US)
(72) Inventor: Firoozabady, Ebrahim, Pleasant Hill, CA 94523 (US); Wintz, Hsu-ching Chen, El Cerrito, CA 94530 (US)
(74) Representative: Woolley, Lindsey Claire

(57) **Abstract**

The invention provides novel regulatory polynucleotide sequences useful in plant genetic engineering applications, e.g., polynucleotides having transcriptional promoter or terminator activity, are provided. The invention also provides novel gene and polypeptide sequences, for example, genes corresponding to pineapple carotenoid biosynthesis proteins, e.g., carotenoid isomerase (ISO), phytoene synthase (PSY) and lycopene β-cyclase, (LYC), which find use in producing plants with improved traits, e.g., improved nutritional value. In addition, related nucleic acids, e.g., vectors, and transformed plants that include one or more of these polynucleotides or polypeptides are also provided, as are related methods for producing such compositions.

## Description

### CROSS-REFERENCE TO RELATED APPLICATIONS

This application claims priority to and benefit of United States Provisional Patent Application Serial No. 60/671,648, filed on April 15, 2005, the specification of which is hereby incorporated by reference in its entirety.

### FIELD OF THE INVENTION

The present invention relates generally to the field of plant molecular biology including the regulation of gene expression in plants.

### BACKGROUND OF THE INVENTION

Isolated plant regulatory sequences, *e.g*., transcriptional promoters and terminators, are useful in genetic engineering processes that produce transgenic plants with desired phenotypes, such as plants resistance to drought, temperature extremes, pests, diseases, and herbicides, among other properties. To produce such transgenic plants, isolated plant promoters and terminators are typically inserted into vectors and operably linked to DNA sequences of interest. Plant cells are then transformed with these vectors such that the promoters and terminators control the expression of the DNA sequences in the cells. In some cases, the expression of proteins encoded by introduced DNA sequences (*e.g*., cDNA sequences comprising open reading frames) in the plants produces the desired phenotypes.

In certain cases, it is desirable to inhibit the expression of endogenous DNA sequences of the plants to produce the desired phenotypes. One exemplary and well established strategy for achieving this inhibition includes introducing promoters operably linked to antisense nucleotide sequences such that expression of the antisense sequences produces RNA transcripts that interfere with translation of the mRNA of the endogenous DNA sequences. Alternatively still, RNAi approaches can also be used to downregulate the expression of a targeted endogenous gene. RNAi strategies are also well described in the art.

The particular type of promoter selected will typically control when and where within the plant the introduced DNA sequences are expressed. When expression in specific tissues or organs is sought, tissue-preferred or tissue-specific promoters are generally used. When gene expression in response to a given stimulus is desired, inducible promoters can be selected. For example, certain tissue-specific promoters may be induced or activated by internal or external agents such as phytohormones, light, or other stimuli. When continuous expression is desired in all the cells of a plant, constitutively active promoters are generally utilized. Additional regulatory sequences upstream and/or downstream from the core promoter sequence may be included in expression constructs for transformation vectors to bring about varying levels of expression of the introduced DNA sequences in a transgenic plant.

From the foregoing, it is evident that additional plant gene regulatory sequences, *e.g*., plant promoters and terminators, and related vectors, are desirable. It is also evident that plant gene sequences that have the ability to impart a desired phenotype when expressed, overexpressed, or used to downregulate an endogenous gene product, also find use in the production of plants with improved traits. These and other features of the invention will be apparent upon complete review of the following disclosure.

### SUMMARY OF THE INVENTION

The present invention provides regulatory sequences that are used in plant genetic engineering applications. In certain aspects, for example, the invention provides promoters and/or terminators that are used in processes to transform pineapples and other plants. The invention also provides cDNA sequences that encode polypeptides that are used to achieve desired phenotypes in plants. In addition, the invention also provides related expression cassettes, vectors, and transformed plants that include these promoters and/or genes. Various methods are also provided.

In one aspect, the invention provides an isolated or recombinant nucleic acid comprising a polynucleotide sequence selected from the group consisting of: (a) a polynucleotide sequence of SEQ ID NO: 1-8, 10-12, 14, 16, 17, 93, complements thereof or unique subsequences thereof; (b) a polynucleotide sequence that hybridizes under highly stringent conditions to substantially an entire length of the polynucleotide sequence of (a); and (c) a polynucleotide sequence encoding a polypeptide comprising an amino acid sequence corresponding to: (i) SEQ ID NO: 9, 13 or 15, (ii) a conservative variant of SEQ ID NO: 9, 13 or 15, (iii) a unique subsequence of SEQ ID NO: 9, 13 or 15; or (iv) a polypeptide having at least 90% sequence identity with SEQ ID NO: 9, 13 or 15.

In some aspects, the nucleic acid of the invention, *e.g*., polynucleotide sequences of SEQ ID NO: 2-8, 10, 12 or 16 possess transcription regulatory activity, such as transcriptional promoter or terminator activity. In other aspects, the polynucleotides encode polypeptides having various activities such as carotenoid biosynthesis activity, *e.g*., carotenoid isomerase activity, phytoene synthase activity or lycopene β-cyclase activity. Polypeptide encoded by the polynucleotides are also a feature of the invention, *e.g*., the polypeptides of SEQ ID NO: 9, 13 or 15.

In other aspects, the invention provides host cell that contain nucleic acids of the invention. The host cells used are not particularly limited, for example, the host cell can be a bacterial cell such as *E. coli,* an *Agrobacterium* species, or a plant host cell.

The nucleic acids of the invention are most typically employed in larger molecules. For example, a nucleic acid of the invention can be used in an expression cassette, or can be placed into a vector of any type, *e.g.,* a plasmid, a virus or an expression vector. Any vector comprising a nucleic acid of the invention is also a feature of the invention. Methods that utilize a vector of the invention is also a feature of the invention, for example, a method to produce a transformed plant cell that harbors the vector.

In other embodiments, plant cells that harbor a nucleic acid of the invention (*e.g*., a vector) are features of the invention. More specifically, the invention provides plant cells transformed with a recombinant vector containing a nucleic acid with a polynucleotide sequence selected from: (a) a polynucleotide sequence of SEQ ID NO: 1-8, 10-12, 14, 16, 17, 93, complements thereof or unique subsequences thereof; (b) a polynucleotide sequence that hybridizes under highly stringent conditions to substantially an entire length of the polynucleotide sequence of (a); and (c) a polynucleotide sequence encoding a polypeptide comprising an amino acid sequence corresponding to: (i) SEQ ID NO: 9, 13 or 15, (ii) a conservative variant of SEQ ID NO: 9, 13 or 15, (iii) a unique subsequence of SEQ ID NO: 9, 13 or 15; or (iv) a polypeptide having at least 90% sequence identity with SEQ ID NO: 9, 13 or 15. The invention also encompasses entire plants or parts of plants (e.g., fruits) that incorporate these transformed plant cells.

The nature of the transformed plant cells is not limited, for example, the plant cell can be a pineapple cell, a monocotyledonous plant cell, or a dicotyledonous plant cell. Examples of transformed plant cells finding use with the invention include cells (or entire plants or plant parts) derived from the genera: *Ananas, Musa, Vitis, Fragaria, Lotus, Medicago, Onobrychis, Trifolium, Trigonella, Vigna, Citrus, Carica, Persea, Prunus, Syragrus, Theobroma, Coffea, Linum, Geranium, Manihot, Daucus, Arabidopsis, Brassica, Raphanus, Sinapis, Atropa, Capsicum, Datura, Hyoscyamus, Lycopersicon, Nicotiana, Solanum, Petunia, Digitalis, Majorana, Mangifera, Cichorium, Helianthus, Lactuca, Bromus, Asparagus, Antirrhinum, Heterocallis, Nemesia, Pelargonium, Panicum, Pennisetum, Ranunculus, Senecio, Salpiglossis, Cucurbita, Cucumis, Browaalia, Lolium, Malus, Apium, Gossypium, Vicia, Lathyrus, Lupinus, Pachyrhizus, Wisteria, Stizolobium, Agrostis, Phleum, Dactylis, Sorgum, Setaria, Zea, Oryza, Triticum, Secale, Avena, Hordeum, Saccharum, Poa, Festuca, Stenotaphrum, Cynodon, Coix, Olyreae, Phareae, Glycine, Pisum, Psidium, Passiflora, Cicer, Phaseolus, Lens,* and *Arachis.*

In some aspects, the transformed plant cell harbors a polynucleotide sequence encoding a polypeptide comprising an amino acid sequence corresponding to: (i) SEQ ID NO: 9, 13 or 15, (ii) a conservative variant of SEQ ID NO: 9, 13 or 15, (iii) a unique subsequence of SEQ ID NO: 9, 13 or 15; or (iv) a polypeptide having at least 90% sequence identity with SEQ ID NO: 9, 13 or 15, and further where the polypeptide encoded by the polynucleotide is expressed in the plant cell. In some embodiments, the expressed polypeptide modifies the accumulation of one or more carotenoid in the transformed plant cell, e.g., the plant cell can show increased levels of β-carotene or lycopene.

In some embodiments, the vectors of the invention can be used in homologous recombination with endogenous sequences. The polynucleotide sequences of the invention when carried on a vector can be used to target endogenous chromosomal loci for homologues recombination, resulting in a heritable change in the chromosomal sequence. Transformed plant cells that carry the recombinant chromosome are a feature of the invention.

In some embodiments, the invention provides transformed plant cells where a promoter or terminator of the invention, *e.g*., a polynucleotide sequence of SEQ ID NO: 2-8, 10, 12 and 16, complements thereof, unique subsequences thereof, or a polynucleotide sequence that hybridizes under highly stringent conditions to substantially an entire length of the polynucleotide, is operably linked to another nucleic acid that encodes an RNA molecule (for example, an antisense transcript or RNAi cassette) and/or a protein molecule (e.g., a cDNA). In some embodiments, the operably linked nucleic acid confers resistance on the transformed cell to one or more of: insects, drought, nematodes, viral disease, bacterial disease or herbicides. In some aspects, the operably linked nucleic acid contains sense sequences that correspond to at least a portion of at least one endogenous gene. Alternatively, the operably linked nucleic acid contains sense sequences that correspond to at least a portion of at least one exogenous gene. Alternatively still, the operably linked nucleic acid contains antisense sequence that corresponds to at least a portion of at least one endogenous gene. In some aspects, the operably linked nucleic acid encodes at least one polypeptide transcription factor.

In other embodiments, the invention provides various methods for producing or using the compositions of the invention. For example, the invention provides methods for producing a vector, the where the steps include operably linking an isolated or recombinant nucleic acid to an extra-chromosomal element, where the nucleic acid comprises a polynucleotide sequence selected from the group consisting of:
(a) a polynucleotide sequence of SEQ ID NO: 1-8, 10-12, 14, 16, 17, 93, complements thereof or unique subsequences thereof;
(b) a polynucleotide sequence that hybridizes under highly stringent conditions to substantially an entire length of the polynucleotide sequence of (a); and
(c) a polynucleotide sequence encoding a polypeptide comprising an amino acid sequence corresponding to: (i) SEQ ID NO: 9, 13 or 15, (ii) a conservative variant of SEQ ID NO: 9, 13 or 15, (iii) a unique subsequence of SEQ ID NO: 9, 13 or 15; or (iv) a polypeptide having at least 90% sequence identity with SEQ ID NO: 9, 13 or 15.

Most typically, the extrachromosomal element used in the methods is capable of replication in a bacterial cell or a plant cell.

### DEFINITIONS

Before describing the present invention in detail, it is to be understood that this invention is not limited to particular embodiments. It is also to be understood that the terminology used herein is for the purpose of describing particular embodiments only, and is not intended to be limiting. Units, prefixes, and symbols are denoted in the forms suggested by the International System of Units (SI), unless specified otherwise. Numeric ranges are inclusive of the numbers defining the range. As used in this specification and the appended claims, the singular forms "a", "an" and "the" also include plural referents unless the context clearly dictates otherwise. Thus, for example, reference to "a cell" also includes two or more cells (*e.g*., in the form of a tissue, a culture of cells, etc.), and the like. Further, unless defined otherwise, all technical and scientific terms used herein have the same meaning as commonly understood by one of ordinary skill in the art to which the invention pertains. The terms defined below, and grammatical variants thereof, are more fully defined by reference to the specification in its entirety.

Polynucleotide or nucleic acid: The terms "nucleic acid," "nucleic acid sequence," "nucleotide sequence," "oligonucleotide," "polynucleotide" or "nucleic acid molecule" or similar terms as used herein refer to oligomers of bases typically linked by a sugar-phosphate backbone, such as oligonucleotides or polynucleotides, and fragments or portions thereof, and to DNA or RNA of genomic or synthetic origin which can be single- or double-stranded, and represent a sense or antisense strand. The terms nucleic acid, polynucleotide and nucleotide also specifically include nucleic acids composed of bases other than the five biologically occurring bases (i.e., adenine, guanine, thymine, cytosine and uracil), and also include nucleic acids having non-natural backbone structures, such as PNA molecules. Unless otherwise indicated, a particular nucleic acid sequence of this invention encompasses complementary sequences, in addition to the sequence explicitly indicated.

Nucleic acid molecules (*e.g*., DNA or RNA) are said to have "5' ends" and "3' ends" because mononucleotides are reacted to make oligonucleotides or polynucleotides in a manner such that the 5' phosphate of one mononucleotide pentose ring is attached to the 3' oxygen of its neighbor in one direction via a phosphodiester linkage. Thus, a polynucleotide will typically have one "5' end" comprising a 5' phosphate and one "3' end" comprising a 3' oxygen. A polynucleotide sequence, even if internal to a larger nucleic acid, also can be said to have 5' and 3' directionality. In either a linear or circular DNA molecule, discrete elements are referred to as being "upstream" or 5' of the "downstream" or 3' elements. This terminology reflects the fact that transcription proceeds in a 5' to 3' fashion along the DNA strand.

Complement thereof: The term "complement thereof" refers to nucleic acid that is both the same length as, and exactly complementary to, a given nucleic acid.

Nucleic acid segment: The term "nucleic acid segment" refers to a polynucleotide or transcribable analog thereof, of at least 15 nucleotides in length, usually at least 50 nucleotides in length, more usually at least 100 nucleotides in length, generally at least 200 nucleotides in length, typically at least 300 nucleotides in length, more typically at least 400 nucleotides in length, and most typically at least 500 nucleotides in length. To illustrate, a nucleic acid segment can include a full-length gene (*e.g*., a gene that encodes a polypeptide, such as a carotenoid isomerase polypeptide or the like), or a subsequence of such a gene.

Sense nucleic acid segment: The term "sense nucleic acid segment" generally refers to a coding nucleic acid segment. In contrast, the term "antisense nucleic acid segment" typically refers a complement of a sense nucleic acid segment.

Subsequence: A "subsequence", "fragment" or "portion" is any portion of a larger nucleic acid or polypeptide. In some aspects, the subsequence (or fragment or portion) retains a critical feature or biological activity of the larger molecule. For example, a subsequence of a promoter polynucleotide sequence may also act as a promoter sequence. Similarly, subsequences of carotenoid biosynthesis enzyme of the invention may retain that retain the enzymatic activity of the larger parent molecule.

Unique subsequence: As used herein, the term unique subsequences can apply to either nucleotide sequences in nucleic acids or amino acid sequences in polypeptides, where a portion of a larger sequence is unique. Unique subsequences of the invention find a variety of uses. For example, a unique subsequence of a promoter polynucleotide sequence may also act as a promoter sequence. Unique polynucleotide subsequences of the invention are also useful as probes to identify the nucleic acids of the invention and related nucleic acids. Unique polypeptide subsequences of carotenoid biosynthesis enzymes of the invention (or indeed any polypeptide) also have use if those subsequences retain enzymatic activity.

Hybridize: As used herein, two nucleic acids are said to "hybridize" or "bind" when they associate with one another, typically in solution, typically by a base-pairing phenomenon between antiparallel nucleic acid molecules. Nucleic acids hybridize due to a variety of well characterized physico-chemical forces, such as hydrogen bonding, solvent exclusion, base stacking and the like. An extensive guide to the hybridization of nucleic acids is found in Tijssen (1993) Laboratory Techniques in Biochemistry and Molecular Biology--Hybridization with Nucleic Acid Probes part I chapter 2, "Overview of principles of hybridization and the strategy of nucleic acid probe assays," (Elsevier, New York), as well as in Ausubel (Ed.) Current Protocols in Molecular Biology, Volumes I, II, and III, 1997, which is incorporated by reference. Hames and Higgins (1995) Gene Probes 1 IRL Press at Oxford University Press, Oxford, England, (Hames and Higgins 1) and Hames and Higgins (1995) Gene Probes 2 IRL Press at Oxford University Press, Oxford, England (Hames and Higgins 2) provide details on the synthesis, labeling, detection and quantification of DNA and RNA, including oligonucleotides. Both Hames and Higgins 1 and 2 are incorporated by reference.

High sequence similarity: A region of "high sequence similarity" refers to a region that is 90% or more identical to a second selected region when aligned for maximal correspondence (*e.g*., manually or, *e.g*., using the common program BLAST set to default parameters). A region of "low sequence similarity" is 30% or less identical, more preferably, 40% or less identical to a second selected region, when aligned for maximal correspondence (*e.g*., manually or using BLAST set with default parameters).

Homologous: Nucleic acids are "homologous" when they are derived, naturally or artificially, from a common ancestral sequence. Homology is often inferred from sequence similarity between two or more nucleic acids. This occurs naturally as two or more descendent sequences deviate from a common ancestral sequence over time as the result of mutation and natural selection. Artificially homologous sequences may be generated in various ways. For example, a nucleic acid sequence can be synthesized *de novo* to yield a nucleic acid that differs in sequence from a selected parental nucleic acid sequence. Artificial homology can also be created by artificially recombining one nucleic acid sequence with another, as occurs, *e.g*., during cloning or chemical mutagenesis, to produce a homologous descendent nucleic acid. Artificial homology may also be created using the redundancy of the genetic code to synthetically adjust some or all of the coding sequences between otherwise dissimilar nucleic acids in such a way as to increase the frequency and length of highly similar stretches of nucleic acids while minimizing resulting changes in amino acid sequences to the encoded gene products. Typically, such artificial homology is directed to increasing the frequency of identical stretches of sequence of at least three base pairs in length. More preferably, it is directed to increasing the frequency of identical stretches of sequence of at least four base pairs in length. It is generally assumed that two nucleic acids have common ancestry when they demonstrate sequence similarity. However, the exact level of sequence similarity necessary to establish homology varies in the art. In general, for purposes of this disclosure, two nucleic acid sequences are deemed to be homologous when they share enough sequence identity to permit direct recombination to occur between the two sequences, that is, anywhere along the two sequences.

Stringent hybridization: As used herein, "stringent hybridization" conditions or "stringent conditions" in the context of nucleic acid hybridization are sequence dependent, and are different under different environmental parameters. An extensive guide to hybridization of nucleic acids is found in Tijssen (1993), *supra.* Generally, "highly stringent" hybridization and wash conditions are selected to be at least about 5° C lower than the thermal melting point (Tₘ) for the specific sequence at a defined ionic strength and pH. The Tₘ is the temperature (under defined ionic strength and pH) at which 50% of the target sequence hybridizes to a perfectly matched probe. Very stringent conditions are selected to be equal to the Tₘ point for a particular nucleic acid of the present invention, this occurs, *e.g*., when a copy of a nucleic acid is created using the maximum codon degeneracy permitted by the genetic code. Stringent hybridization conditions are sequence-dependent and will be different in different circumstances. Longer sequences hybridize specifically at higher temperatures.

An example of stringent hybridization conditions for hybridization of complementary nucleic acids which have more than 100 complementary residues on a filter in a Southern or northern blot is 50% formalin with 1 mg of heparin at 42°C, with the hybridization being carried out overnight. An example of highly stringent wash conditions is 0.15M NaCl at 72°C for about 15 minutes. An example of stringent wash conditions is a 0.2x SSC wash at 65°C for 15 minutes (*see,* Sambrook et al., Molecular Cloning: A Laboratory Manual, 3rd Ed., Cold Spring Harbor Laboratory Press, Cold Spring Harbor, N.Y. (2001), for a description of SSC buffer). Often, a high stringency wash is preceded by a low stringency wash to remove background probe signal. An example of a medium stringency wash for a duplex of, *e.g*., more than 100 nucleotides, is 1x SSC at 45°C for 15 minutes. An example of a low stringency wash for a duplex of, *e.g*., more than 100 nucleotides, is 4-6x SSC at 40°C for 15 minutes. In general, a signal to noise ratio of 2x (or higher, *e.g*., 5X, 10X, 20X, 50X, 100X or more) than that observed for control probe in the particular hybridization assay indicates detection of a specific hybridization. For example, the control probe can be a homologue to a relevant nucleic acid, as noted herein. Nucleic acids that do not hybridize to each other under stringent conditions are still substantially identical if the polypeptides which they encode are substantially identical. This occurs, *e.g*., when a copy of a nucleic acid is created using the maximum codon degeneracy permitted by the genetic code.

In some embodiments, stringent hybridization conditions include, *e.g*., 2.0X SSPE (comprising 0.36 M NaCl, 20mM NaH₂PO₄*H₂O, 2 mM EDTA, pH 7.4) and 0.5% SDS at a temperature of 55°C and a pH of 7.4. An optimal SSPE range includes, *e.g*., 1.8 (higher stringency) - 2.2X (lower stringency). Varying the percentage of SDS included does not seem to affect stringency. An optimal temperature range includes, *e.g*., 54-56°C. Assay results typically include light/low signals for high stringency conditions (*e.g*., 57°C or above), and additional non-specific signal generally occurs (as well as darker signal) for the low stringency conditions (*e.g*., 53°C or below). An optimal pH range includes, *e.g*., 7.2-7.6. A high stringency condition at, *e.g*., pH 8.0 or above typically produces a lighter signal, whereas a low stringency condition at, *e.g*., pH 6.5 or below typically produces a darker signal with an increased level of cross-hybridization.

Correspond: Two nucleic acids "correspond" when they have the same sequence, or when one nucleic acid is complementary to the other, or when one nucleic acid is a subsequence of the other, or when one sequence is derived, by natural or artificial manipulation from the other.

Encoding: The term "encoding" refers to a polynucleotide sequence that can be translated into one or more amino acids. The term does not require a start or stop codon. An amino acid sequence can be encoded in any reading frame provided by a polynucleotide sequence.

Gene: As used herein, the term "gene" most generally refers to a combination of polynucleotide elements, that when operatively linked in either a native or recombinant manner, provide some product or function. The term "gene" is to be interpreted broadly herein, encompassing mRNA, cDNA, cRNA and genomic DNA forms of a gene. In some cases, a gene is heritable. In some aspects, genes comprise coding sequences (*e.g*., an "open reading frame" or "coding region") necessary for the production of a polypeptide, while in other aspects, genes do not encode a polypeptide. Examples of genes that do not encode polypeptides include ribosomal RNA genes (rRNA) and transfer RNA (tRNA) genes.

The term "gene" can optionally encompass non-coding regulatory sequences that reside at a genetic locus. For example, in addition to a coding region of a nucleic acid, the term "gene" also encompasses the transcribed nucleotide sequences of the full-length mRNA adjacent to the 5' and 3' ends of the coding region. These noncoding regions are variable in size, and typically extend on both the 5' and 3' ends of the coding region. The sequences that are located 5' and 3' of the coding region and are contained on the mRNA are referred to as 5' and 3' untranslated sequences (5' UT and 3' UT). Both the 5' and 3' UT may serve regulatory roles, including translation initiation, post-transcriptional cleavage and polyadenylation. The term "gene" encompasses mRNA, cDNA and genomic forms of a gene.

In some aspects, the genomic form or genomic clone of a gene includes the sequences of the transcribed mRNA, as well as other non-transcribed sequences which lie outside of the transcript. The regulatory regions which lie outside the mRNA transcription unit are sometimes called "5' or 3' flanking sequences." A functional genomic form of a gene typically contains regulatory elements necessary for the regulation of transcription.

Promoter: The term "promoter" is usually used to describe a DNA region, typically but not exclusively 5' of the site of transcription initiation, sufficient to confer accurate transcription initiation. In some embodiments, a promoter is constitutively active, while in alternative embodiments, the promoter is conditionally active (*e.g*., where transcription is initiated only under certain physiological conditions). The promoter nucleic acid typically contains regions of DNA that are involved in recognition and binding of RNA polymerase and other proteins or factors to initiate transcription. A "plant promoter" is a promoter capable of initiating transcription in plant cells.

Terminator: As used herein, the term "terminator" or "termination sequence" generally refers to a 3' flanking region of a gene that contains nucleotide sequences which regulate transcription termination and typically confer RNA stability. More generally, the term "regulatory element" refers to any genetic element that controls some aspect of the expression of nucleic acid sequences.

Pineapple gene promoter activity: The term "pineapple gene promoter activity" refers to the ability of a region of DNA to initiate the transcription (serve as a promoter or an initiation site for transcription) of a pineapple gene in a pineapple cell or plant. A region of DNA (*e.g*., a promoter, etc.) with pineapple gene promoter activity may also have the ability to initiate transcription of other nucleic acid segments in other plants.

Operatively linked: As used herein, the terms "in operable combination," "in operable order," "operatively linked," "operatively joined" and similar phrases, when used in reference to nucleic acids, refer to the linkage of nucleic acid sequences placed in functional relationships with each other. For example, an operatively linked promoter sequence, open reading frame and terminator sequence results in the accurate production of an RNA molecule. In some aspects, operatively linked nucleic acid elements result in the transcription of an open reading frame and ultimately the production of a polypeptide (*i.e*., expression of the open reading frame).

Construct: As used herein, the term "construct" is used in reference to any extra-chromosomal polynucleotide or other molecule that can transfer nucleic acid segment(s) into a cell, and is typically capable of replication in a cell. The term "vector" or "vehicle" is sometimes used interchangeably with "construct." A vector optionally comprises parts which mediate vector propagation and manipulation (*e.g*., sequences necessary for replication, genes imparting drug or antibiotic resistance, a multiple cloning site, operably linked promoter/enhancer elements which enable the expression of a cloned gene, etc.). Exemplary vectors include (*i.e*., are derived from) plasmids, bacteriophages, or plant or animal viruses. A "cloning vector" or "shuttle vector" or "subcloning vector" contains operably linked parts that facilitate subcloning steps (*e.g*., a multiple cloning site containing multiple restriction endonuclease sites).

Expression vector: The term "expression vector" as used herein refers to a recombinant vector comprising operably linked polynucleotide sequences that facilitate expression of a coding sequence in a particular host organism (*e.g*., a plant or bacterial expression vector). Polynucleotide sequences that facilitate expression typically include, *e.g*., a promoter, transcription termination sequences (*i.e*., terminator sequences), and a ribosome binding site, often along with other sequences.

Encode: As used herein, the term "encode" refers to any process whereby the information in a polymeric macromolecule or sequence string is used to direct the production of a second molecule or sequence string that is different from the first molecule or sequence string. As used herein, the term is used broadly, and can have a variety of applications. In some aspects, the term "encode" describes the process of semi-conservative DNA replication, where one strand of a double-stranded DNA molecule is used as a template to encode a newly synthesized complementary sister strand by a DNA-dependent DNA polymerase.

In another aspect, the term "encode" refers to any process whereby the information in one molecule is used to direct the production of a second molecule that has a different chemical nature from the first molecule. For example, a DNA molecule can encode an RNA molecule (*e.g*., by the process of transcription incorporating a DNA-dependent RNA polymerase enzyme). Also, an RNA molecule can encode a polypeptide, as in the process of translation. When used to describe the process of translation, the term "encode" also extends to the triplet codon that encodes an amino acid. In some aspects, an RNA molecule can encode a DNA molecule, *e.g*., by the process of reverse transcription incorporating an RNA-dependent DNA polymerase. In another aspect, a DNA molecule can encode a polypeptide, where it is understood that "encode" as used in that case incorporates both the processes of transcription and translation.

Heterologous: As used herein, the terms "heterologous" or "exogenous" as applied to polynucleotides or polypeptides refers to molecules that have been rearranged or artificially supplied to a biological system and are not in a native configuration (*e.g*., with respect to sequence, genomic position or arrangement of parts) or are not native to that particular biological system. The terms indicate that the relevant material originated from a source other than the naturally occurring source, or refers to molecules having a non-natural configuration, genetic location or arrangement of parts. The terms "exogenous" and "heterologous" are sometimes used interchangeably with "recombinant."

Recombinant: The term "recombinant" in reference to a nucleic acid or polypeptide indicates that the material (*e.g*., a recombinant nucleic acid, gene, polynucleotide, polypeptide, etc.) has been altered by human intervention. Generally, the arrangement of parts of a recombinant molecule is not a native configuration, or the primary sequence of the recombinant polynucleotide or polypeptide has in some way been manipulated. The alteration to yield the recombinant material can be performed on the material within or removed from its natural environment or state. For example, a naturally occurring nucleic acid becomes a recombinant nucleic acid if it is altered, or if it is transcribed from DNA which has been altered, by means of human intervention performed within the cell from which it originates. A gene sequence open reading frame is recombinant if that nucleotide sequence has been removed from it natural context and cloned into any type of artificial nucleic acid vector. The term recombinant can also refer to an organism that harbors recombinant material. Protocols and reagents to produce recombinant molecules, especially recombinant nucleic acids, are common and routine in the art (see, *e.g*., Maniatis et al. (eds.), Molecular Cloning: A Laboratory Manual, Cold Spring Harbor Laboratory Press, NY, [1982]; Sambrook et al. (eds.), Molecular Cloning: A Laboratory Manual, Second Edition, Volumes 1-3, Cold Spring Harbor Laboratory Press, NY, [1989]; and Ausubel et al. (eds.), Current Protocols in Molecular Biology, Vol. 1-4, John Wiley & Sons, Inc., New York [1994]).

Native or endogenous: In contrast to a heterologous or exogenous molecule, a "native" or "endogenous" molecule is native to the biological system, species or chromosome under study. A "native" or "endogenous" gene is a gene that does not contain nucleic acid elements encoded by sources other than the chromosome on which it is normally found in nature. An endogenous gene, transcript or polypeptide is encoded by its natural chromosomal locus, and not artificially supplied to the cell.

Isolated: A nucleic acid, protein or other component is "isolated" when it is partially or completely separated from components with which it is normally associated (other proteins, nucleic acids, cells, synthetic reagents, etc.).

Enriched: As used herein, a nucleic acid, protein or other component is "enriched" when it is relative fraction (*i.e*., proportion) in a treated heterogeneous mixture is increased compared to its relative fraction in the heterogeneous mixture prior to the treatment (*e.g*., prior to a purification step).

Host cell: The term "host cell" typically refers to a cell that contains a heterologous nucleic acid, such as a vector, and supports the replication and/or expression of the nucleic acid. Host cells may be prokaryotic cells such as *E. coli* or *Agrobacterium,* or eukaryotic cells such as yeast, insect, amphibian or mammalian cells. Preferably, host cells are plant cells. In the context of the invention, one particularly preferred host cell is a pineapple host cell.

Bacteria: As used herein, the terms "bacteria" and "eubacteria" refer to prokaryotic organisms that are distinguishable from Archaea and eukaryotes (*e.g*., plants). "Prokaryote" refers to organisms belonging to the Kingdom Monera (also termed Procarya). Prokaryotic organisms are generally distinguishable from eukaryotes by their unicellular organization, asexual reproduction by budding or fission, the lack of a membrane-bound nucleus or other membrane-bound organelles, a circular chromosome, the presence of operons, the absence of introns, message capping and poly-A mRNA, and other biochemical characteristics, such as a distinguishing ribosomal structure. The Prokarya include subkingdoms Eubacteria (*e.g*., *E. coli* and *Agrobacterium*) and Archaea (sometimes termed "Archaebacteria"). Cyanobacteria (the blue green algae) and mycoplasma are sometimes given separate classifications under the Kingdom Monera.

Derived from: As used herein, the term "derived from" refers to a component that is isolated from or made using a specified molecule or organism, or information from the specified molecule or organism. For example, a polypeptide that is derived from a second polypeptide can include an amino acid sequence that is identical or substantially similar to the amino acid sequence of the second polypeptide. In the case of polypeptides, the derived species can be obtained by, for example, naturally occurring mutagenesis, artificial directed mutagenesis or artificial random mutagenesis. Mutagenesis of a polypeptide typically entails manipulation of the polynucleotide that encodes the polypeptide.

Similarly, the term "derived from" can apply to polynucleotides. A polynucleotide that is derived from a source polynucleotide can include a nucleotide sequence that is identical or substantially similar to the source nucleotide sequence. In the case of polynucleotides, the derived species can be obtained by, for example, mutagenesis. In some aspects, a derived polynucleotide is generated by placing a source polynucleotide into a heterologous context, *i.e*., into a context that is different from its native or endogenous context. For example, a gene promoter can be derived from an endogenous gene promoter by removing that endogenous promoter domain and placing it in operable combination with different nucleotide sequences with which it is not normally associated.

Reporter: As used herein, the term "reporter" or equivalent terms refers in a general sense to any component that can be readily detected in a system under study, where the detection of the reporter correlates with the presence or absence of some other molecule or property, or can be used to identify, select and/or screen targets in a system of interest.. The choice of the most suitable reporter to use for a particular application depends on the intended use, and other variables known to one familiar with the art. In some aspects, a reporter is a reporter gene.

A wide variety of reporter molecules and genes are known in the art. Each reporter has a particular assay for the detection of that reporter. Some reporter detection assays can be enzymatic assays (*e.g*., β-glucuronidase, or GUS), while other assays can be immunological in nature (*e.g*., ELISA or immunohistochemical analysis), or colorimetric, for example. Further still, a reporter can include a protein, *e.g*., an enzyme, that confers antibiotic resistance or sensitivity (*e.g*., β-lactamase, chloramphenicol acetyltransferase (CAT), and the like), a fluorescent marker (*e.g*., a green fluorescent protein such as GFP, YFP, EGFP, RFP, etc.), a luminescent marker (*e.g*., a firefly luciferase protein), an affinity based screening marker, an enzymatic activity such as *lacZ* (β-galactosidase), or other positive or negative selectable marker genes such as ADH (alcohol dehydrogenase), his3, ura3, leu2, lys2, or the like.

Transcription factor: The term "transcription factor" refers to any factor, typically a protein, that controls, regulates or influences the process of transcription.

Expression: The term "expression" refers to the transcription and accumulation of sense mRNA or antisense RNA derived from polynucleotides. Expression may also refer to translation of mRNA into a polypeptide, *e.g*., a carotenoid biosynthesis polypeptide. In certain embodiments, for example, selected polypeptides are expressed in preselected plant storage organs, such as roots, seeds, fruits, etc., leading to enhanced accumulation of one or more carotenoids (*e.g*., naturally produced carotenoids) or other compounds in that plant storage organ. Accordingly, the term "fruit-specific expression" refers to the expression of, *e.g*., introduced polynucleotides that is substantially limited to fruit tissues of the transformed plants.

Expression cassette: The term "expression cassette" refers to a DNA segment that comprises a coding region (*e.g*., a polynucleotide of interest, typically comprising a coding region open reading frame) operably linked to suitable control sequences (*e.g*., a promoter and/or terminator sequences) capable of effecting expression of the coding region in a compatible host.

Polypeptide: A polypeptide is any oligomer of amino acids (natural or unnatural, or a combination thereof), of any length, typically but not exclusively joined by covalent peptide bonds. A polypeptide can be from any source, *e.g*., a naturally occurring polypeptide, a polypeptide produced by recombinant molecular genetic techniques, a polypeptide from a cell or translation system, or a polypeptide produced by cell-free synthetic means. A polypeptide is characterized by its amino acid sequence, *e.g*., the primary structure of its component amino acids. As used herein, the amino acid sequence of a polypeptide is not limited to full-length sequences, but can be partial or complete sequences. Furthermore, it is not intended that a polypeptide be limited by possessing or not possessing any particular biological activity. As used herein, the term "protein" is synonymous with polypeptide. The term "peptide" refers to a small polypeptide, for example but not limited to, from 2-25 amino acids in length.

Conservative variant: As used herein, the term "conservative variant" refers to a component, *e.g*., a conservative variant enzyme, that differs from but functionally performs identically or similar to the component from which it was derived. For example, a pineapple carotenoid isomerase (ISO) and a conservative variant ISO will differ in primary amino acid sequence, but will both catalyze the same reaction. The conservative variant can have, *e.g*., one variation, two variations, three variations, four variations, or five or more variations in sequence.

Identical: The terms "identical" or "identity" in the context of two or more nucleic acid or polypeptide sequences, refer to two or more sequences or subsequences that are the same or have a specified percentage of amino acid residues or nucleotides that are the same, when compared and aligned for maximum correspondence, as measured using a sequence comparison algorithms or by visual inspection.

Substantially identical: The phrase "substantially identical" in the context of two nucleic acids or polypeptides, refers to two or more sequences or subsequences that have at least 60%, preferably 80%, most preferably 90-95% nucleotide or amino acid residue identity, when compared and aligned for maximum correspondence, as measured using, *e.g*., a sequence comparison algorithm or by visual inspection. Typically, the substantial identity exists over a region of the sequences that is at least about 50 residues in length, more preferably over a region of at least about 100 residues, and most preferably the sequences are substantially identical over at least about 150 residues. In some embodiments, the sequences are substantially identical over the entire length of the coding regions.

"Substantially an entire length of a polynucleotide or amino acid sequence" refers to at least 70%, generally at least 80%, or typically 90% or more of a sequence.

Caroteniod: The term "carotenoid" refers to naturally occurring organic pigments that are found in plants and some other photosynthetic organisms like algae, some types of fungi and some bacteria. There are over 600 known carotenoids split generally into two classes, xanthophylls and carotenes. Carotenoids are generally characterized by a large (35-40 carbon atoms) polyene chain, sometimes terminated by ring structures. Carotenoids where some of the double bonds have been oxidized are known as xanthophylls (*e.g*., lutein and zeaxanthin); un-oxidized carotenoids are known as carotenes (*e.g*., α-carotene, β-carotene and lycopene). Carotenoids have a wide variety of physiological functions.

Carotenoid biosynthesis polynucleotide: The term "carotenoid biosynthesis polynucleotide" refers to a nucleic acid that encodes a polypeptide having carotenoid biosynthetic activity (*e.g*., a carotenoid biosynthetic polypeptide, etc.).

Carotenoid biosynthesis polypeptide: The term "carotenoid biosynthesis polypeptide" refers to a biocatalyst or enzyme that catalyzes at least one step in a carotenoid biosynthesis pathway. Carotenoid biosynthesis polypeptides include, *e.g*., geranylgeranyl pyrophosphate synthases, isopentenyl diphosphate isomerases, phytoene synthases, phytoene desaturases, ζ-carotene desaturases, lycopene β-cyclases, lycopene ε-cyclases, β-carotene hydroxylases, ε-hydroxylases, and the like.

Carotenoid isomerase activity: The term "carotenoid isomerase activity" refers to catalytic action that results in the isomerization of poly-*cis*-carotenoids to all-*trans-*carotenoids.

Lycopene β-cyclase activity: The term "lycopene β-cyclase activity" refers to catalytic action that results in the cyclization of lycopene (ψ,ψ-carotene) to yield β-carotene or α-carotene.

Monocot: The term "monocot" or "monocotyledonous" refers to plants having a single cotyledon. Exemplary monocots include pineapple, maize, rice, wheat, oat, and barley.

Dicot: The term "dicot" or "dicotyledonous" refers to plants that produce an embryo with two cotyledons. Exemplary dicots include tobacco, bell pepper, cotton, soybean, and peanut.

Plant: The term "plant" includes whole plants, plant organs (*e.g*., leaves, stems, roots, etc.), seeds, plant germplasm and plant cells and progeny of the same. The type of plants that can be used as described herein generally include higher plants amenable to transformation techniques, including both monocotyledonous and dicotyledonous plants.

Transformation: The term "transformation" refers to the transfer or introduction of a nucleic acid segment into a cell, tissue or organism, for example, into a plant cell, plant tissue or an intact plant, whether the introduction results in genetically stable inheritance of the nucleic acid segment or only a transient presence of the nucleic acid segment in the genome of the plant or plant cell. Plant cells or plants that include the introduced nucleic acid segments are referred to as "transgenic," "recombinant," or "transformed" plant cells or plants.

### BRIEF DESCRIPTION OF THE FIGURES

**FIG. 1** provides various polynucleotide and polypeptide sequences that find use with the invention.

**FIG. 2** provides primer nucleotide sequences and other sequences that find use with the invention.

**FIG. 3** provides a schematic of the binary vector pHCW-5, which is used to transfer nucleotide sequences to plant cells (*i.e*., plant transformation) from an *Agrobacterium tumefaciens* host.

**FIG. 4** provides a table showing the results of a GUS expression activity assay that assesses the activity of various promoter sequences in different pineapple tissues.

**FIG. 5** provides a table showing the results of a GUS expression activity assay that assesses the activity of various promoter constructs in Del Monte Gold MD-2 pineapple leaf explants.

**FIG. 6** provides a table showing the results of a GUS expression assay that assesses the activity of various promoter constructs in MD-2 pineapple leaf explants.

### DETAILED DESCRIPTION OF THE INVENTION

The present invention provides a variety of novel polynucleotides and polypeptides useful for producing transgenic plants, and for producing transgenic plants having improved nutritional or aesthetic value, or improved in any physiological aspect. Generally, the invention provides two types of polynucleotides, which are either regulatory sequences (*e.g*., promoter sequences and terminator sequences) which can be used to express any gene of interest, or protein coding sequences, or portions of protein coding cDNAs, that find use in the upregulation or downregulation of the respective genes to produce fruits with improved aesthetics or nutritional quality. The invention also provides vectors comprising these nucleic acids, host cells and transgenic plants comprising these sequences, and methods for producing and using the same. Aspects of the invention are also described in the Examples provided below.

### NOVEL REGULATORY NUCLEIC ACIDS

The present invention provides a variety of novel plant regulatory polynucleotides useful in genetic engineering applications for expressing any particular gene of interest, most typically in a plant host cell (*e.g*., as in a transgenic plant). The regulatory sequences of the invention are transcriptional regulatory sequences, (*e.g*., promoter sequences and terminator sequences) which can be used to express any gene of interest.

### Promoter Sequences

In some aspects, the invention provides novel promoter sequences that find use in the transcriptional initiation of recombinant nucleic acids. The use of recombinant promoter sequences is well established in the art, and furthermore, the mechanism of promoter action is also well understood. However, it is appreciated that an understanding of the molecular mechanisms underlying promoter activity are not required to make or use the present invention.

In order to generate promoters that can be used to drive the recombinant expression of any particular gene of interest, a number of novel promoter sequences were isolated from different pineapple genes using various isolation methods (see Examples). These particular genes were chosen for promoter isolation in view of their known expression patterns. For example, it is known that the ubiquitin genes and the epoxide hydrolase genes are highly conserved across species and are widely expressed in a number of different plant tissues

Epoxide hydrolases (EHS) catalyse the hydrolysis of epoxides to the corresponding diols. In nature, these enzymes have three main functional roles: detoxification, synthesis of signaling molecules and metabolism which allows some bacteria to use epoxides or their alkene and halohydrin precursors as carbon sources. It is known that epoxide hydrolases expression is enhanced in pineapple fruits and roots (Neuteboom et al., Plant Science 163:1021-1035 [2002]). In this invention, it is contemplated that an isolated recombinant EHS promoter will have activity in pineapple, and can be advantageously used to drive the expression of operatively coupled nucleotide sequences, especially in pineapple fruit.

Bromelain inhibitor (BI) is a cysteine proteinase inhibitor isolated from pineapple stem. Bromelain inhibitor gene expression was analyzed by RT-PCR in various pineapple tissues. The results showed that the gene is expressed at high levels in pineapple fruit, but expression was not detectable in shoots. Thus, it was contemplated that an isolated recombinant bromelain inhibitor gene promoter can be used to drive the expression of operatively coupled nucleotide sequences, and advantageously drive the expression in a "fruit-specific" manner in the pineapple plant.

Thus, these promoters make good candidates for use in recombinant gene expression and transgenic plant constructions.

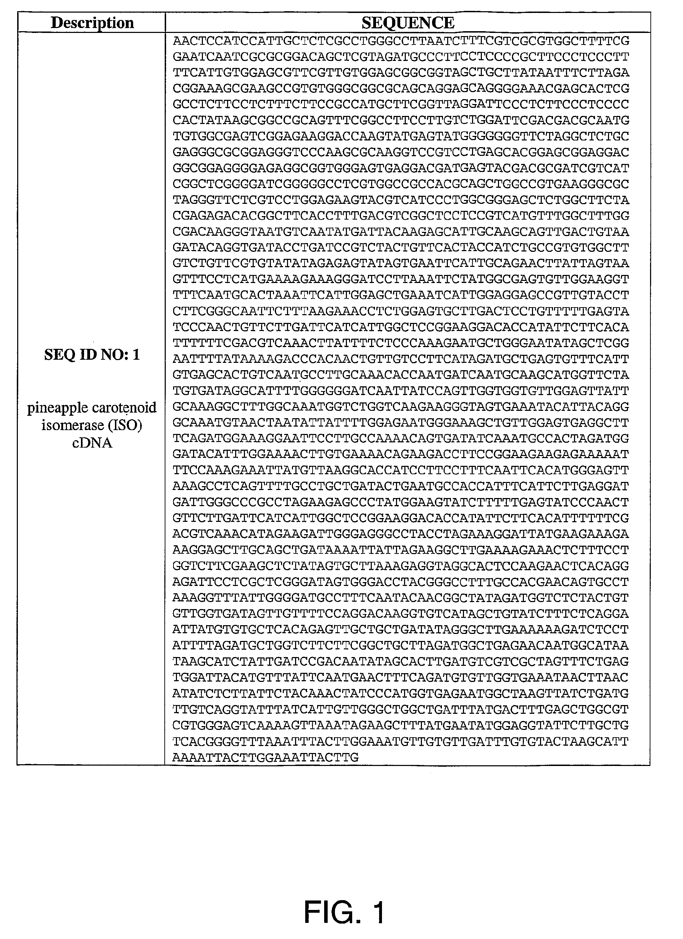

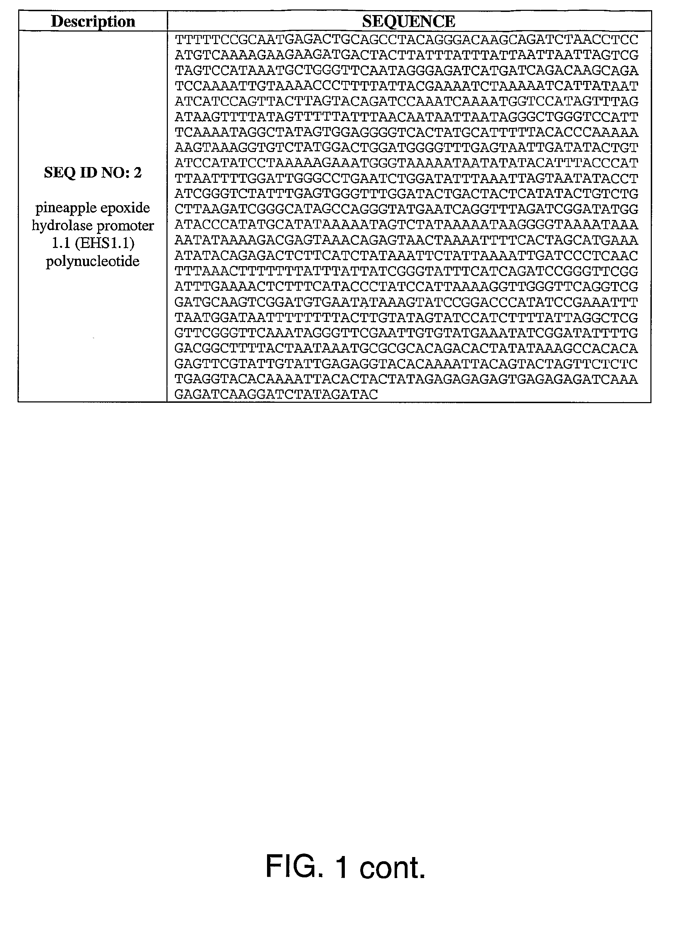

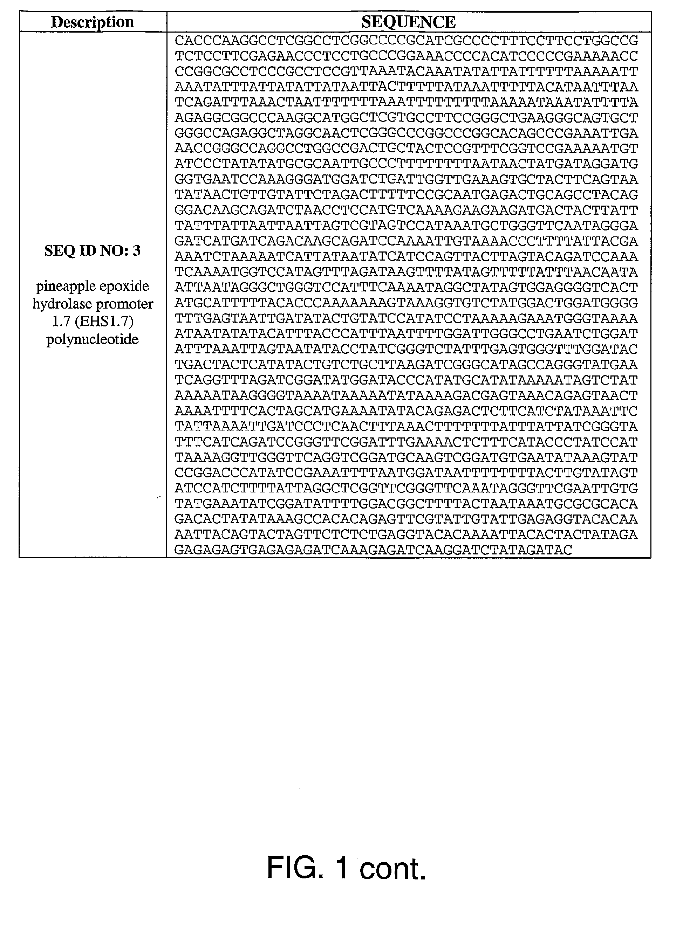

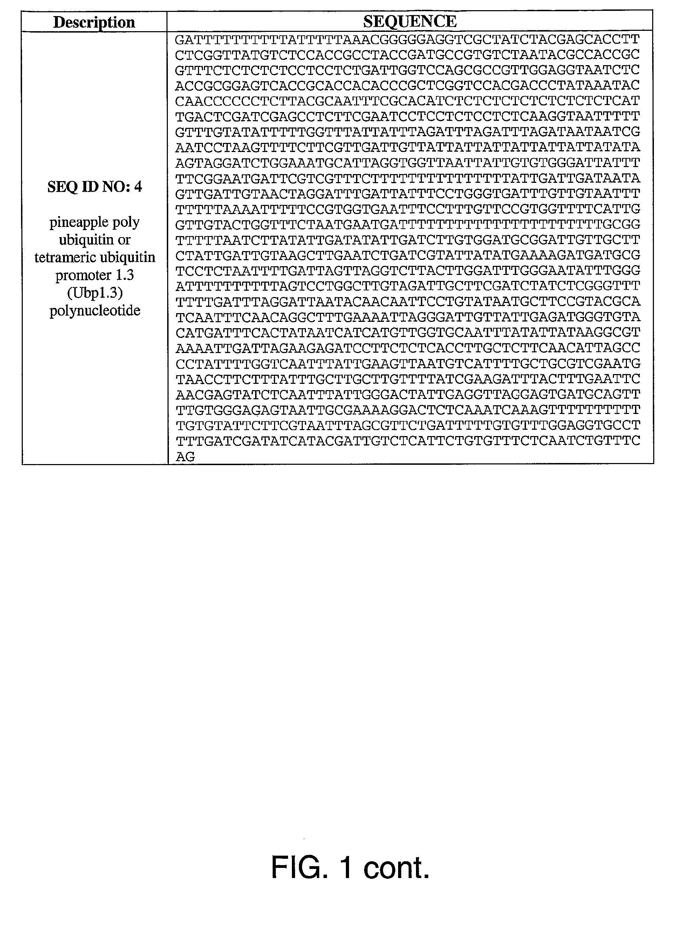

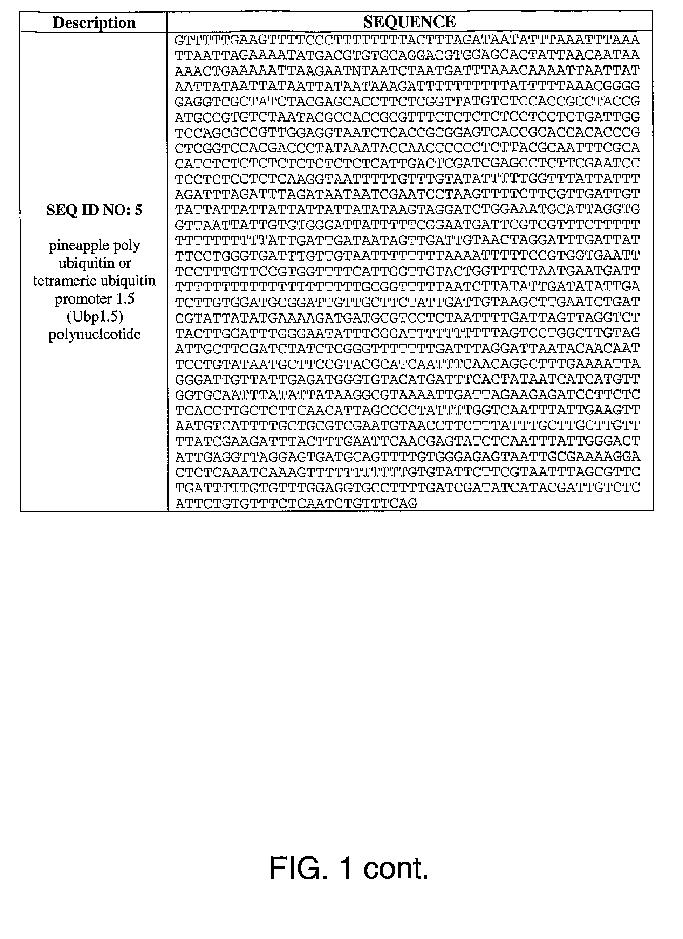

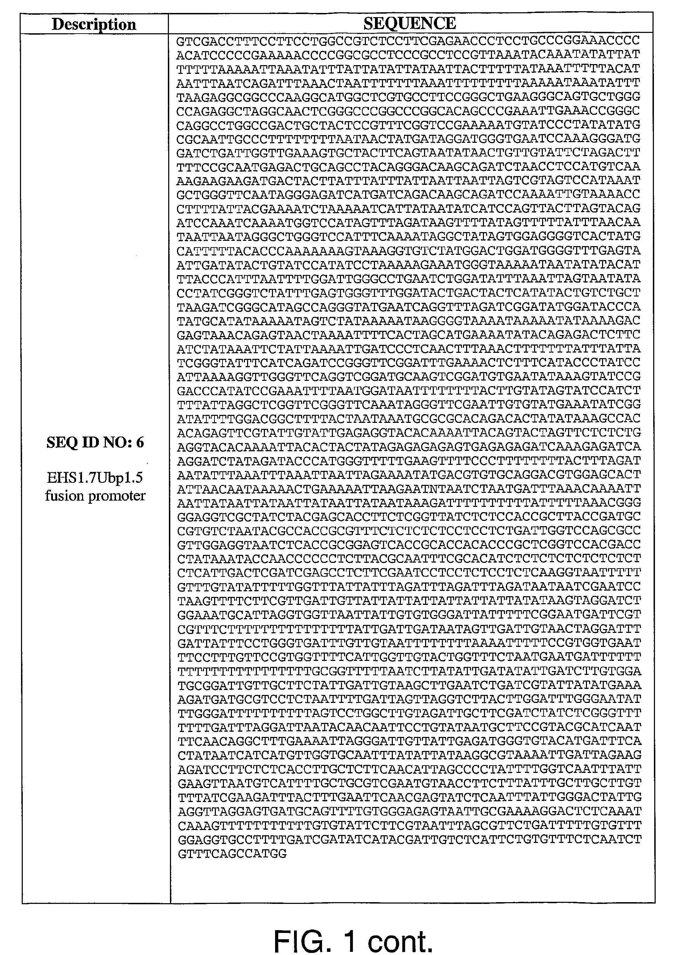

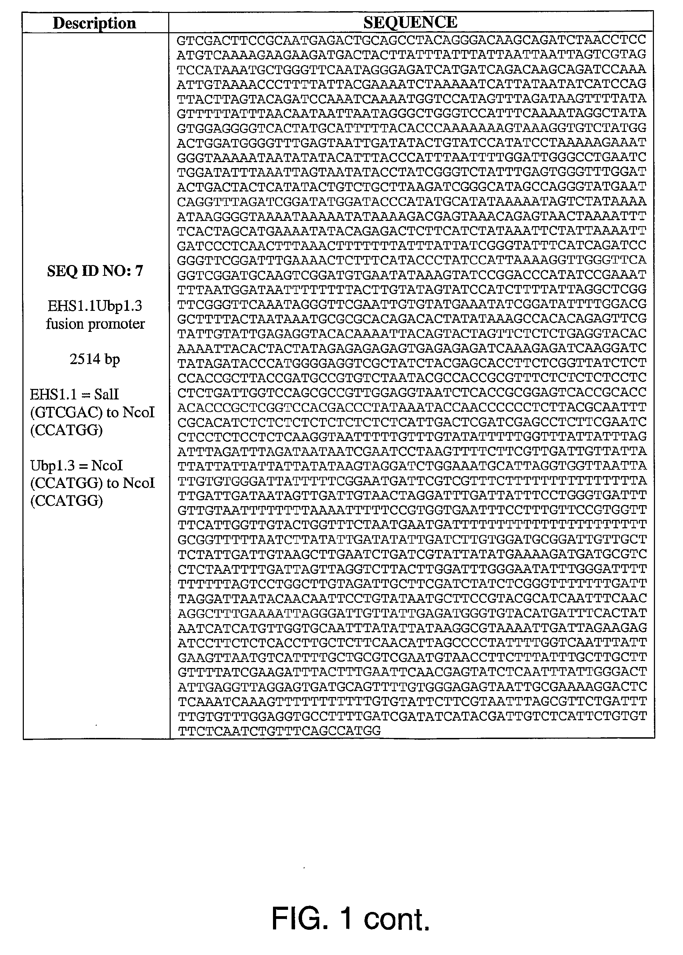

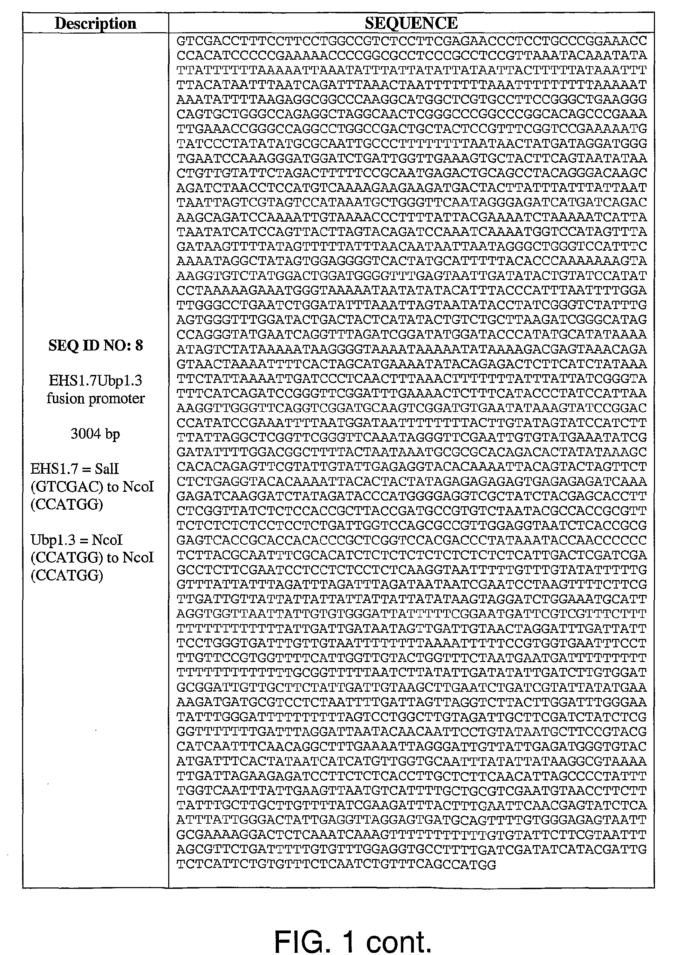

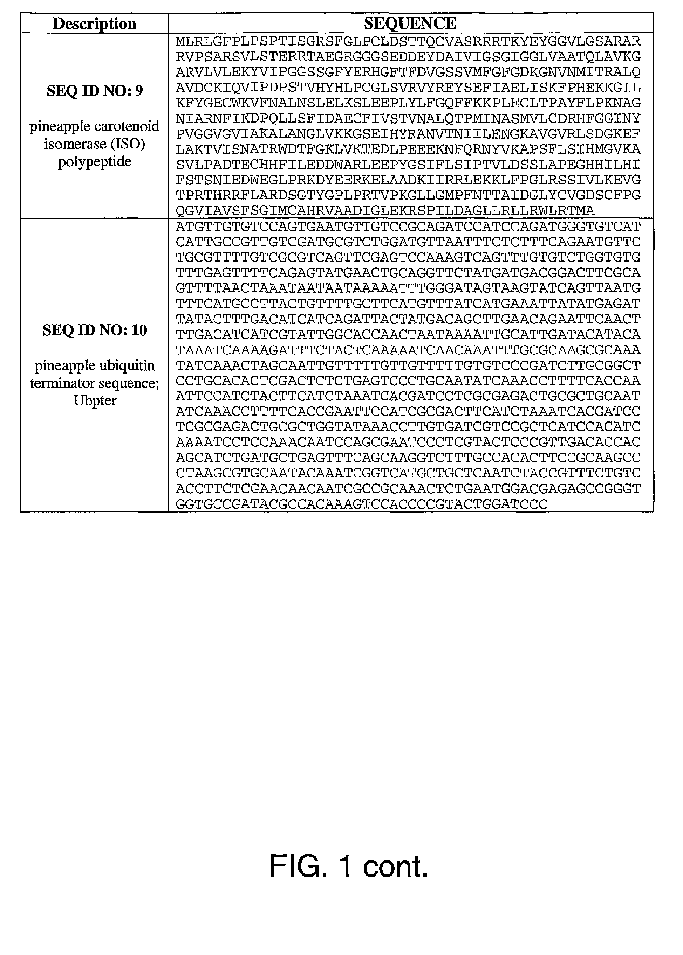

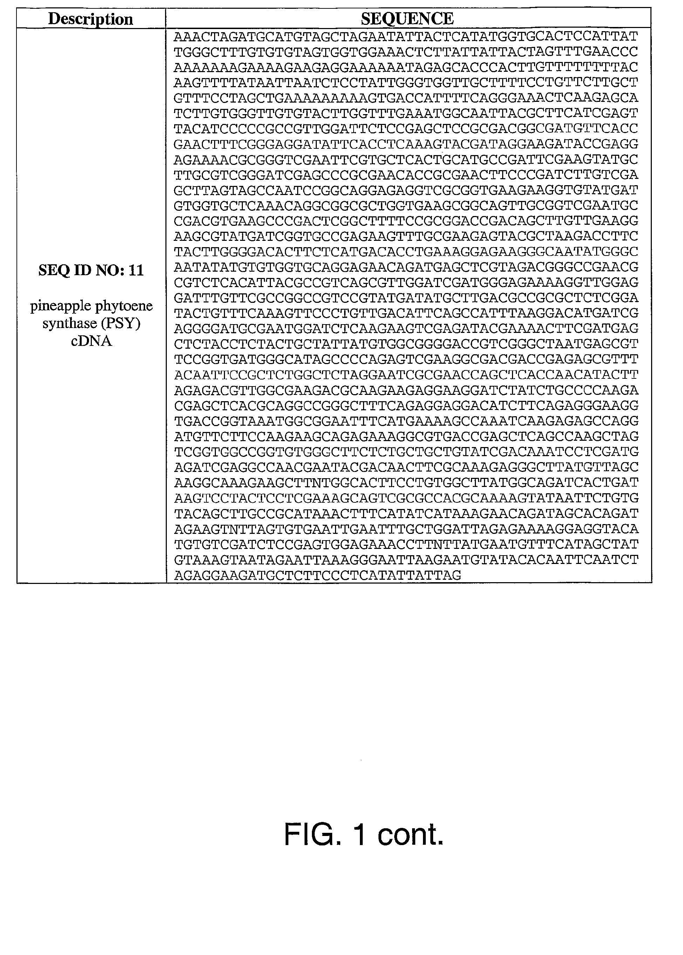

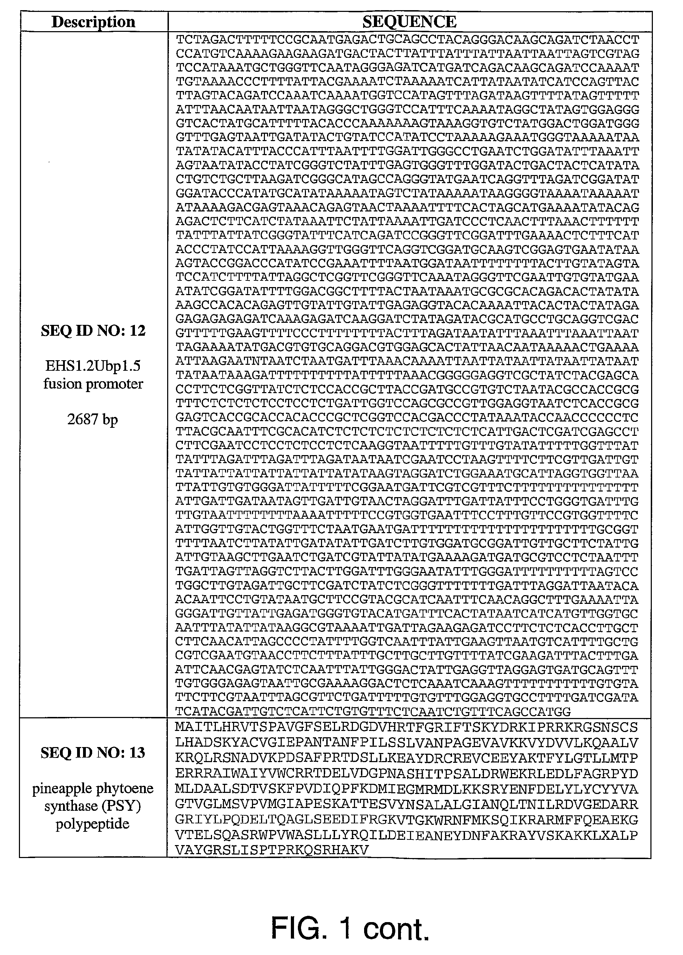

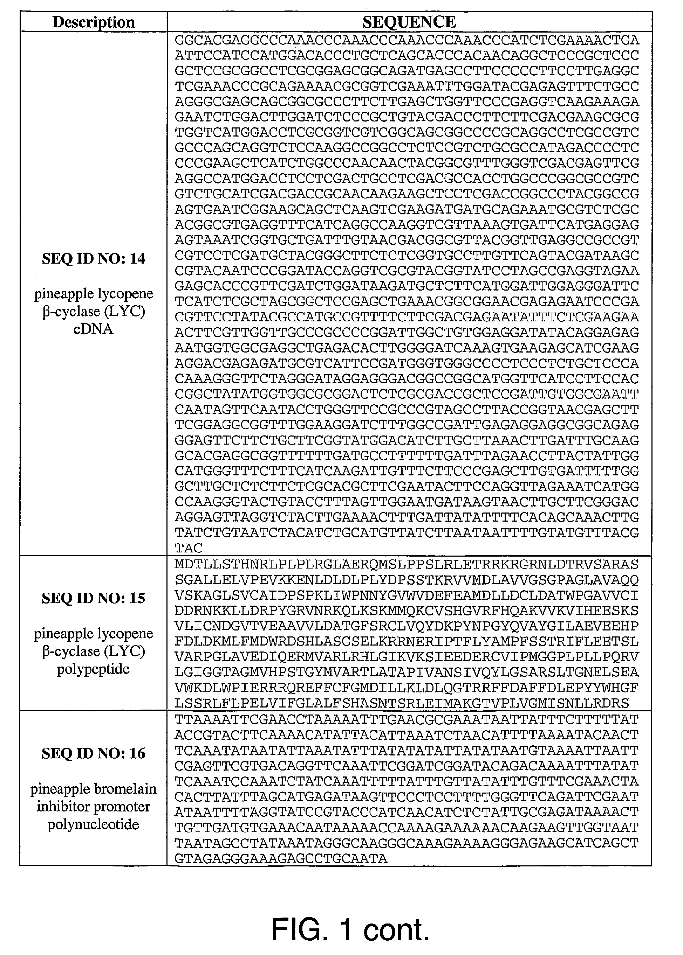

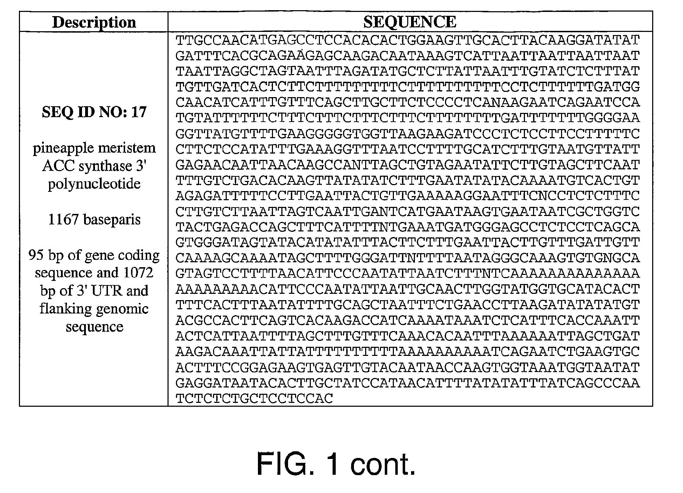

Promoter sequences provided by the invention are provided in FIG. 1, and are summarized in Table 1. All nucleic acid sequences provided herein are represented using symbols recommended by IUPAC-IUB Joint Commission on Biochemical Nomenclature (JCBN), Abbreviations and Symbols for Nucleic Acids, Polynucleotides and their Constituents (see, *e.g*., J. Mol. Biol., 55:299-310 (1971), which is incorporated by reference.

**TABLE 1**

| **Name** | **Description** | **SEQ ID NO** |
|---|---|---|
| **PROMOTERS** | | |
| **EHS1.1** | pineapple epoxide hydrolase promoter 1.1 kB (EHS1.1) | **2** |
| **EHS1.7** | pineapple epoxide hydrolase promoter 1.7 kB (EHS1.7) | **3** |
| **Ubp1.3** | pineapple poly ubiquitin or tetrameric ubiquitin promoter 1.3 kB (Ubp1.3) | **4** |
| **Ubp1.5** | pineapple poly ubiquitin or tetrameric ubiquitin promoter 1.5 kB (Ubp1.5) | **5** |
| **EHS1.7Ubp1.5** | EHS and Ubp fusion promoter | **6** |
| **EHS1.1Ubp1.3** | EHS and Ubp fusion promoter | **7** |
| **EHS1.7Ubp1.3** | EHS and Ubp fusion promoter | **8** |
| **EHS1.2Ubp1.5** | EHS and Ubp fusion promoter | **12** |
| **bromelain inhibitor promoter** | pineapple bromelain inhibitor promoter | **16** |
| | | |

| **TERMINATORS** | | |
|---|---|---|
| **Ubpter** | pineapple ubiquitin gene terminator sequence | **10** |
| **ACC-3'-ter** | pineapple meristem ACC synthase 3' terminator region | **17** |

The invention also provides unique subsequences of the promoters of the invention (*e.g*., unique subsequences of SEQ ID NOS: 2-5 and 16). Unique promoter subsequences retain the ability to regulate transcription (*e.g*., promote transcription initiation). It is well within the skill level of one familiar with the art to test for promoter activity to determine which promoter subsequences retain promoter activity.

Also as provided by the invention, fusions of any combination of these promoters is contemplated, and indeed, demonstrated (see Examples). For example, fusions of the EHS and Ubp gene promoters can be made and used to drive gene expression (see, SEQ ID NOS: 6, 7, 8 and 12. It is not intended that fusion promoters of the invention be limited to any particular combinations (*e.g*., limited to SEQ ID NOS: 6, 7, 8 and 12). Indeed, any single promoter of the invention can be combined with any other promoter of the invention. Alternatively, any promoter of the invention can be used as a fusion with any other promoter known in the art to create new fusion promoters. Subsequences of promoters can also be used in fusion promoter constructions, where subsequences containing less than the full lengths of SEQ ID NOS: 6, 7, 8 or 12 are used in fusion promoter constructs.

In some aspects, the promoters of the invention can show activity in multiple plant tissues such as leaves, stem, roots and fruit. In some aspects, promoters of the invention can demonstrate differential expression patterns where the promoter may show greater activity in one type of plant tissue relative to its activity observed in a different tissue. In some aspects, promoters that show good expression in fruit (*e.g*., pineapple fruit) are most advantageous.

In some aspects, promoters that show activity in fruit but no activity or reduced activity in other tissues is most preferable. Conversely, in other aspects, it is most advantageous that a promoter be active in a tissue such as leaf, stem or roots, but have no activity or reduced activity in the fruit. Such is the case where it is desirable to express a gene product that results in pest resistance in the leaves, stem or roots, but not express that gene product in the edible portion of the plant (*i.e*., the fruit).

It is not intended that the novel promoter sequences of the invention be limited to use in any particular species or variety of pineapple, nor to any particular plant species, nor indeed, to any particular monocotyledonous or dicotyledonous plant, as it is demonstrated herein that the promoters of the invention are active in a wide variety of pant species.

### Terminator Sequences

The invention provides a novel terminator sequences that find use in proper transcriptional processing of recombinant nucleic acids. Although terminator sequences do not by themselves initiate gene transcription, their presence is necessary for optimal and accurate processing and termination of the RNA transcript, and result in message stability. The use of recombinant terminator sequences is established in the art. It is appreciated that an understanding of the molecular mechanisms underlying promoter terminator sequence activity are not required to make or use the present invention.

In order to generate a terminator that can be used to optimize the expression of any particular recombinant gene of interest, novel terminator sequences were isolated from pineapple. These were terminator sequences are Ubpter (**FIG. 1**, SEQ ID NO: 10), isolated from the pineapple ubiquitin gene (see Example 3) and ACC-3'-ter (**FIG. 1**, SEQ ID NO: 17), isolated from the pineapple meristem ACC synthase 3' domain (Example 8).

Because ubiquitin is highly conserved across species and is widely expressed in a number of different plant tissues, the pineapple ubiquitin terminator sequence Ubpter was a good candidate for the isolation of the terminator sequence for use in recombinant gene expression and transgenic plant constructions. It is not intended that use of the Ubpter or ACC-3'-ter sequences for the expression of recombinant gene products be limited to any particular host cell or variety of pineapple, nor to any particular plant species, nor indeed, to any particular monocotyledonous plant, as it is contemplated, and indeed demonstrated herein, that this sequence can also function in dicotyledonous plants.

The invention also provides unique subsequences of Ubpter and ACC-3'-ter sequences of the invention. Unique Ubpter and ACC-3'-ter subsequences retain the ability to regulate transcription by contributing to accurate transcription termination and RNA stability. It is well within the skill level of one familiar with the art to test for terminator activity to determine which unique Ubpter and ACC-3'-ter subsequences retain terminator activity.

### NOVEL EXPRESSED NUCLEIC ACIDS

In some aspects, the invention provides novel nucleotide sequences that correspond to expressed gene sequences. These novel expressed gene sequences have various uses, including the expression of recombinant gene products (*e.g*., recombinant polypeptides), or for the construction of expression cassettes the result in the targeted down-regulation of endogenous genes (*e.g*., by antisense expression, RNAi or other types of gene silencing mechanisms). The invention provides isolated nucleic acid molecules comprising sequences from pineapple carotenoid biosynthesis genes and corresponding polypeptides. In addition, the invention also provides various vectors and transformed plants that include these pineapple carotenoid biosynthesis genes. In other embodiments, the invention also provides nucleic acids for the targeted down-regulation of specific endogenous genes.

### Carotenoid Biosynthesis Genes

As known in the art, the manipulation of carotenoid (*e.g*., β-carotene, lycopene and phytoene) biosynthesis in plants can result in plants with improved properties, *e.g*., fruit with improved nutritional qualities. That is to say, fruits having elevated levels of β-carotene and/or lycopene have improved nutritional value as plants would exhibit higher levels of β-carotene, lycopene, lutein, or other caretenoids in their edible parts such as fruit (Rosati et al., The Plant J., 24:413 [2000]).

One approach to controlling carotenoid biosynthesis is to regulate the expression of the proteins that are responsible for controlling the enzymatic production of the carotenoids. For example, upregulation of carotenoid isomerase (ISO) and/or phytoene synthase (PSY) results in upregulation of β-carotene and/or lycopene accumulation. Also, downregulation of lycopene β-cyclase (LYC) also results in lycopene accumulation (see, Hirschberg et al., Pure & Applied Chem., 69(10):2151-2158 [1997]).

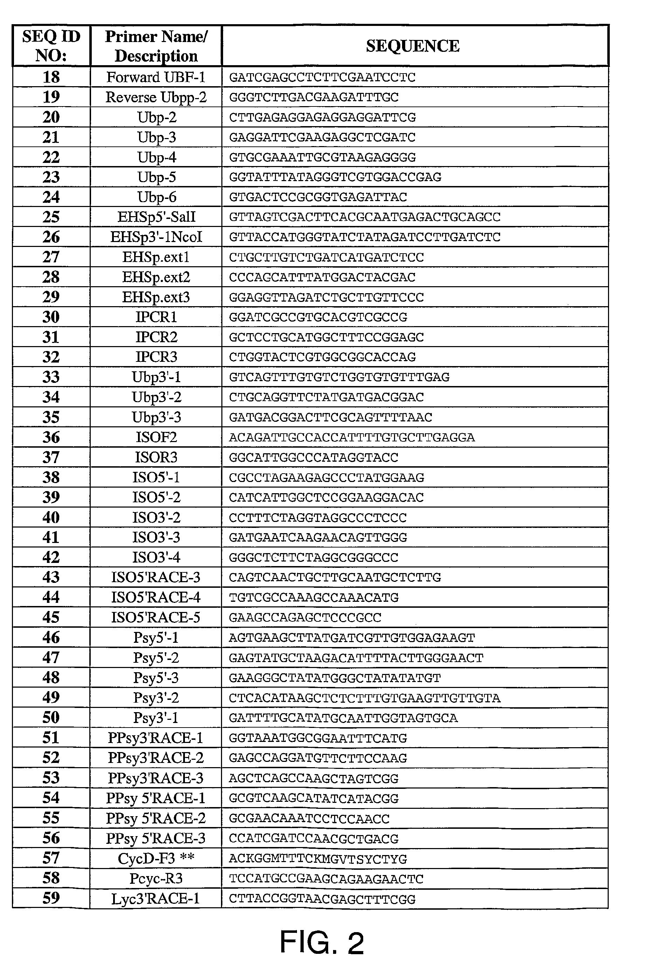

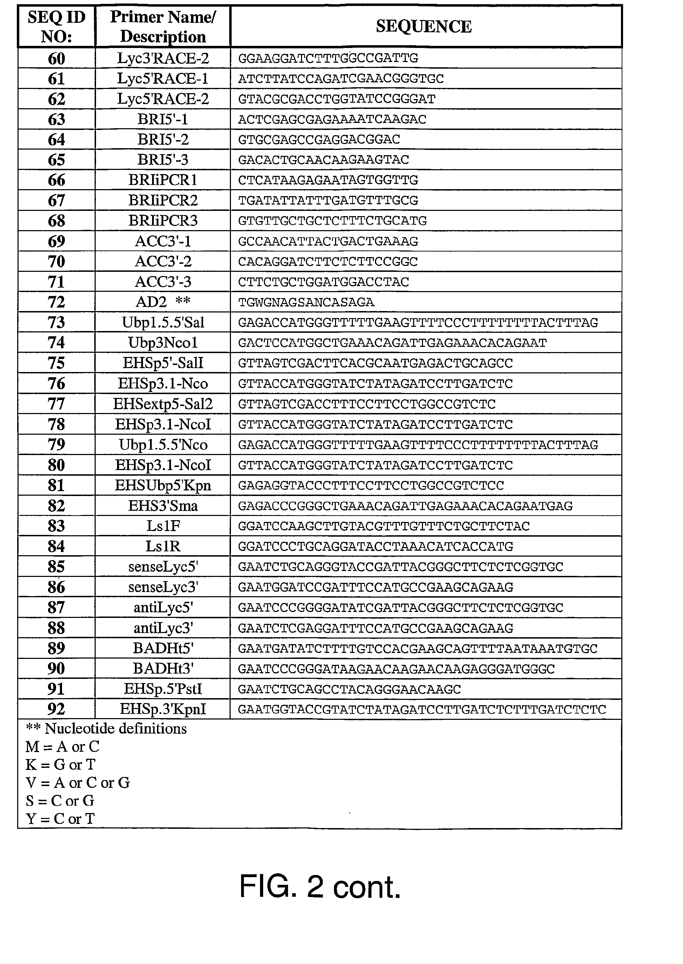

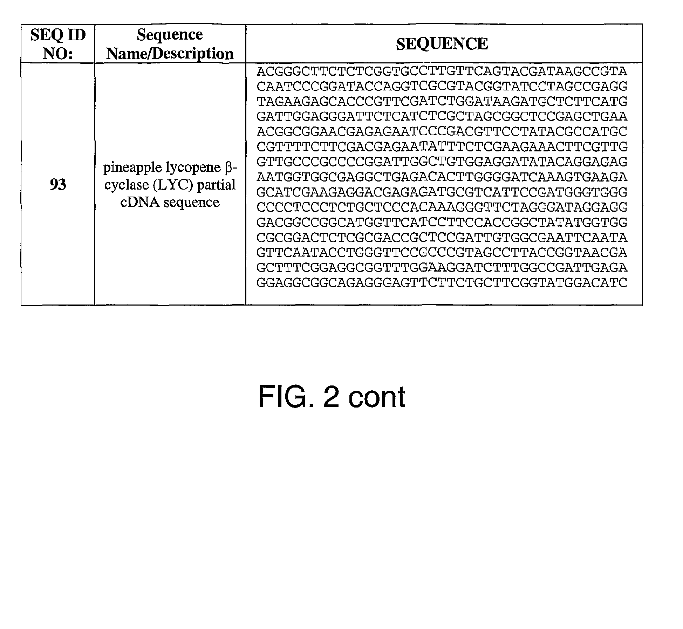

To this end, the cloning of pineapple carotenoid biosynthesis genes was undertaken, as described in detail in the Examples section. More specifically, the invention provides cDNA sequences or partial cDNA sequences that encode the pineapple carotenoid isomerase (ISO) cDNA, the pineapple phytoene synthase (PSY) cDNA, and the pineapple lycopene β-cyclase (LYC) cDNA. Examples of these coding sequences are provided in **FIGS.1** and **2****.** These cDNAs contain the open reading frames for the respective proteins. These cDNA sequences are shown Table 2.

**TABLE 2**

| **Polynucleotide Description** | **SEQ ID NO** |
|---|---|
| pineapple carotenoid isomerase (ISO) cDNA sequence | **1** |
| pineapple phytoene synthase (PSY) cDNA sequence | **11** |
| pineapple lycopene β-cyclase (LYC) cDNA sequence | **14** |
| pineapple ACC synthase partial cDNA 3' region | **17** |
| pineapple lycopene β-cyclase (LYC) partial cDNA sequence | **93** |

The invention also provides pineapple carotenoid biosynthesis polypeptides that are encoded by the respective open reading frames. Examples of these polypeptide amino acid sequences are provided in **FIG.1** and Table 3. The amino acid sequences are represented herein using symbols recommended by IUPAC-IUB Joint Commission on Biochemical Nomenclature (JCBN), Nomenclature and Symbolism for Amino Acids and Peptides (see, *e.g*., J. Biol. Chem., 260:14-42 (1985), which is incorporated by reference.

**TABLE 3**

| **Polypeptide Description** | **SEQ ID NO** |
|---|---|
| pineapple carotenoid isomerase (ISO) amino acid sequence | **9** |
| pineapple phytoene synthase (PSY) amino acid sequence | **13** |
| pineapple lycopene β-cyclase (LYC) amino acid sequence | **15** |

It is contemplated that carotenoid accumulation can be stimulated in plant tissues by the production of a transgenic plant that overexpresses the ISO or PSY polypeptides (SEQ ID NOS: 9 and 13, respectively). Any suitable plant expression vector known in the art can be used in conjunction with the ISO and/or PSY cDNAs (SEQ ID NOS: 1 and 11, respectively) to construct a transgenic plant that overexpresses these polypeptides, thereby producing a plant that displays elevated carotenoid accumulation. It is not intended that the expression of ISO or PSY be limited to any particular promoter or expression system. Indeed, any suitable commercial expression system can be used. In some aspects, a novel promoter and/or terminator of the invention (see, *e.g*., Table 1) can also be used for the expression of novel polypeptides of the invention, *e.g*, but not limited to ISO and PSY polypeptides. Furthermore, overexpression of lycopene β-cyclase can be used for enhancement of β-carotene (precursor of vitamin A), lutein or other carotenoids in plants. Lutein has nutritional value and other health benefits.

It is also contemplated that carotenoid accumulation can be stimulated in plant tissues by the downregulation of endogenous lycopene β-cyclase (LYC) expression by using the pineapple lycopene β-cyclase cDNA sequence provided by the invention in FIG. 1 and 2 and SEQ ID NOS: 14 and 93. For example, the cDNA sequence of SEQ ID NO: 14, 93, or any suitable fragment thereof, can be used in an RNAi expression system to downregulate endogenous lycopene β-cyclase, thereby producing a plant that displays elevated carotenoid accumulation. Other expression inhibition systems are also known in the art, for example, based on RNA silencing and/or antisense technology. It is not intended that the use of the pineapple lycopene β-cyclase cDNA sequences of SEQ ID NOS: 14 or 93 be limited to any particular gene suppression system. It is noted that in any RNAi, RNA silencing or antisense technology, it is not required that the targeted sequences be protein-coding open-reading frame. Any portion of a cDNA, including the 5' and 3' untranslated regions (UTR) in the cDNA can be a candidate target for the inactivation mechanism.

### Aminocyclopropane Carboxylic Acid (ACC) Synthase Gene

Ethylene plays a critical role in plant development, including seed germination, fruit ripening, leaf and flower senescence and abscission. The pathway for endogenous ethylene biosynthesis includes the formation of S-adenosyl-L-methionine (AdoMet) by S-adenosyl-L-methionine synthetase. AdoMet is subsequently converted by *S*-adenosyl-L-methionine methylthio-adenosine-lyase (ACC synthase; EC 4.4.1.14) to the nonprotein amino acid 1-aminocyclopropane-l carboxylic acid (ACC), the immediate precursor of ethylene in higher plants. Physiological analysis has suggested that ACC synthase activity is the key regulatory step in the pathway of ethylene production.

It is well known that endogenous ethylene is often deleterious to crops, including pineapple. ACC synthase genes may be used as targets for the generation of transgenic plants in which endogenous expression of ACC synthase is inhibited to effect suppression of ethylene production (see, *e.g*., United States Patent No. 6,194,639, entitled "ACC synthase genes from pineapple," to Botella et al., filed May 1, 1997).

For this purpose, a portion of the pineapple ACC synthase gene was cloned from pineapple (see Example 8 and SEQ ID NO: 17). It is contemplated that ACC synthase expression can be suppressed in plant tissues by using the pineapple ACC synthase cDNA sequence provided by the invention in **FIG. 1** and SEQ ID NO: 17. For example, this cDNA sequence of SEQ ID NO: 17, or any suitable fragment thereof, can be used in an RNAi expression system to downregulate the expression of endogenous ACC synthase, thereby producing a plant that displays improved characteristics such as delayed flowering. Other expression inhibition systems are also known in the art, for example, based on RNA silencing and/or antisense technology. It is not intended that the use of the pineapple ACC synthase cDNA sequence of SEQ ID NO: 17 be limited to any particular gene suppression system.

It is noted that in any RNAi, RNA silencing or antisense technology, it is not required that the targeted sequences be protein-coding open-reading frame. Any portion of a cDNA, including the 5' or 3' untranslated regions (UTR) in the cDNA can be a candidate target for the inactivation mechanism.

### SEQUENCE VARIANTS

Numerous nucleic acid and polypeptide sequences are within the scope of the present invention. The promoter or carotenoid biosynthetic sequences of the invention are typically identical to or show substantial sequence identity to at least portions of the nucleotide sequences depicted in SEQ ID NOS: 1-8, 10, or 11. A promoter or carotenoid biosynthetic nucleic acid of the invention generally hybridizes to a nucleic acid having a sequence as shown in SEQ ID NOS: 1-8, 10, or 11 under stringent or highly stringent conditions.

Typically, the promoters or carotenoid biosynthetic nucleic acid segments of the invention are between about 100 nucleotides and about 5000 nucleotides in length, typically between about 200 and about 4000 nucleotides in length, and more typically between about 500 and about 3000 nucleotides in length.

### Silent Variations

It will be appreciated by those skilled in the art that due to the degeneracy of the genetic code, a multitude of nucleic acid sequences from, *e.g*., the pineapple ISO, PSY and LYC loci may be produced, some of which may bear minimal sequence homology to the nucleic acid sequences explicitly disclosed herein. For instance, inspection of a codon table (see, *e.g*., Table 4) shows that codons AGA, AGG, CGA, CGC, CGG, and CGU all encode the amino acid arginine. Thus, at every position in the nucleic acids of the invention where an arginine is specified by a codon, the codon can be altered to any of the corresponding codons described above without altering an encoded polypeptide. It is understood that U in an RNA sequence corresponds to T in a DNA sequence.

**TABLE 4**

| **Amino Acids and Abbreviations** | | | **Codons** | | | | | |
|---|---|---|---|---|---|---|---|---|
| Alanine | Ala | A | GCA | GCC | GCG | GCU | | |
| Cysteine | Cys | C | UGC | UGU | | | | |
| Aspartic acid | Asp | D | GAC | GAU | | | | |
| Glutamic acid | Glu | E | GAA | GAG | | | | |
| Phenylalanine | Phe | F | UUC | UUU | | | | |
| Glycine | Gly | G | GGA | GGC | GGG | GGU | | |
| Histidine | His | H | CAC | CAU | | | | |
| Isoleucine | Ile | I | AUA | AUC | AUU | | | |
| Lysine | Lys | K | AAA | AAG | | | | |
| Leucine | Leu | L | UUA | UUG | CUA | CUC | CUG | CUU |
| Methionine | Met | M | AUG | | | | | |
| Asparagine | Asn | N | AAC | AAU | | | | |
| Proline | Pro | P | CCA | CCC | CCG | CCU | | |
| Glutamine | Gln | Q | CAA | CAG | | | | |
| Arginine | Arg | R | AGA | AGG | CGA | CGC | CGG | CGU |
| Serine | Ser | S | AGC | AGU | UCA | UCC | UCG | UCU |
| Threonine | Thr | T | ACA | ACC | ACG | ACU | | |
| Valine | Val | V | GUA | GUC | GUG | GUU | | |
| Tryptophan | Trp | W | UGG | | | | | |
| Tyrosine | Tyr | Y | UAC | UAU | | | | |

Such "silent variations" are one species of "conservatively modified variations", discussed below. One of skill will recognize that each codon in a nucleic acid (except AUG, which is ordinarily the only codon for methionine) can be modified by standard techniques to encode a functionally identical polypeptide. Accordingly, each silent variation of a nucleic acid, which encodes a polypeptide is implicit in any described sequence. The invention provides each and every possible variation of nucleic acid sequence of, *e.g*., the pineapple ISO. PSY and LYC loci that could be made by selecting combinations based on possible codon choices. These combinations are made in accordance with the standard triplet genetic code (*e.g*., as set forth in Table 4) as applied to the nucleic acid sequences of these open reading frames. All such variations of every nucleic acid herein are specifically provided and described by consideration of the sequence in combination with the genetic code.

### Conservative Variations

Owing to the degeneracy of the genetic code, "silent substitutions" (*i.e*., substitutions in a nucleic acid sequence which do not result in an alteration in an encoded polypeptide) are an implied feature of *every* nucleic acid sequence that encodes an amino acid sequence. Similarly, "conservative amino acid substitutions," where one or a limited number of amino acids in an amino acid sequence are substituted with different amino acids with highly similar properties, are also readily identified as being highly similar to a disclosed construct. Such conservative variations of each disclosed sequence are a feature of the present invention.

"Conservative variations" of a particular nucleic acid sequence refers to those nucleic acids which encode identical or essentially identical amino acid sequences, or, where the nucleic acid does not encode an amino acid sequence, to essentially identical sequences. One of skill will recognize that individual substitutions, deletions or additions which alter, add or delete a single amino acid or a small percentage of amino acids (typically less than 5%, more typically less than 4%, 2% or 1%) in an encoded sequence are "conservatively modified variations" where the alterations result in the deletion of an amino acid, addition of an amino acid, or substitution of an amino acid with a chemically similar amino acid. Thus, "conservative variations" of a listed polypeptide sequence of the present invention include substitutions of a small percentage, typically less than 5%, more typically less than 2% or 1%, of the amino acids of the polypeptide sequence, with an amino acid of the same conservative substitution group. Finally, the addition of sequences which do not alter the encoded activity of a nucleic acid molecule, such as the addition of a non-functional sequence, is a conservative variation of the basic nucleic acid.

In some aspects, conservative variants are generated by substituting one amino acid residue in a polypeptide for another amino acid residue having similar chemical properties (*e.g*., aromatic side chains or positively charged side chains), and therefore does not substantially change the functional properties of the polypeptide molecule. Conservative substitution tables providing functionally similar amino acids are well known in the art. Table 5 provides a table of conservative amino acid substitutions, indicating groups that contain natural amino acids of like chemical properties, where substitutions within a group is a "conservative substitution."

**TABLE 5 Conservative Amino Acid Substitutions**

| **Nonpolar and/or Aliphatic Side Chains** | **Polar, Uncharged Side Chains** | **Aromatic Side Chains** | **Positively Charged Side Chains** | **Negatively Charged Side Chains** |
|---|---|---|---|---|
| Glycine | Serine | | | |
| Alanine | Threonine | | | |
| Valine | Cysteine | Phenylalanine | Lysine | Aspartate |
| Leucine | Methionine | Tyrosine | Arginine | Glutamate |
| Isoleucine | Asparagine | Tryptophan | Histidine | |
| Proline | Glutamine | | | |

### ISOLATION OF PINEAPPLE GENES AND REGULTORY SEQUENCES

The isolation of transcriptional promoters, transcriptional terminators, protein coding sequences (*e.g*., cDNA), or other nucleic acids may be accomplished by essentially any technique known to those of skill in the art. For instance, oligonucleotide probes based on known sequences can be used to identify the desired gene in a cDNA or genomic DNA library. Probes may be used to hybridize with genomic DNA or cDNA sequences to isolate homologous genes in the same or different species. Alternatively, antibodies raised against an enzyme can be used to screen an mRNA expression library for the corresponding coding sequence. The isolation of nucleic acid segments is further illustrated in the examples provided below.

Techniques for nucleic acid manipulation of genes such as subcloning nucleic acid sequences encoding polypeptides into expression vectors, labelling probes, DNA hybridization, and the like are also described generally in, *e.g*., Berger and Kimmel, Guide to Molecular Cloning Techniques, Methods in Enzymology, Vol. 152, Academic Press, Inc. (1999)("Berger"); Sambrook et al., Molecular Cloning--A Laboratory Manual, 2nd Ed., Vol. 1-3, Cold Spring Harbor Laboratory (2000) ("Sambrook"); and Current Protocols in Molecular Biology, Ausubel et al. (Eds.), Current Protocols, a joint venture between Greene Publishing Associates, Inc. and John Wiley & Sons, Inc., (supplemented through 2000) ("Ausubel"), which are each incorporated by reference.

Alternatively, the nucleic acids of interest (*e.g*., promoters, terminators, genes encoding desired polypeptides, etc.) can be amplified from nucleic acid samples using amplification techniques. For instance, polymerase chain reaction (PCR) technology can be used to amplify the sequences of desired genes directly from genomic DNA, from cDNA, from genomic libraries or cDNA libraries. PCR and other *in vitro* amplification methods may also be useful, for example, to clone nucleic acid sequences that code for proteins to be expressed, to make nucleic acids to use as probes for detecting the presence of the desired mRNA in samples, for nucleic acid sequencing, or for other purposes.

Examples of techniques sufficient to direct persons of skill through *in vitro* amplification methods, including the polymerase chain reaction (PCR), the ligase chain reaction (LCR), Qβ-replicase amplification and other RNA polymerase mediated techniques (*e.g*., NASBA) are found in Berger, Sambrook, and Ausubel, as well as Mullis et al. (1987) U.S. Pat. No. 4,683,202; PCR Protocols A Guide to Methods and Applications (Innis et al., eds.), Academic Press Inc. (1990)(" Innis"); Arnheim & Levinson (1990) Chemical and Engineering News 36-47; Kwoh et al. (1989) Proc. Natl. Acad. Sci. USA 86:1173; Guatelli et al. (1990) Proc. Natl. Acad. Sci. USA 87:1874; Lomell et al. (1989) J. Clin. Chem. 35:1826; Landegren et al., (1988) Science 241:1077-1080; Van Brunt (1990) Biotechnology 8:291-294; Wu and Wallace, (1989) Gene 4:560; Barringer et al. (1990) Gene 89:117, and Sooknanan and Malek (1995) Biotechnology 13:563-564. Additional methods of cloning *in vitro* amplified nucleic acids are also described in U.S. Pat. No. 5,426,039 to Wallace et al. Methods of amplifying large nucleic acids by PCR are summarized in Cheng et al. (1994) Nature 369:684-685 and the references cited therein, in which PCR amplicons of up to 40 kb are generated.

Polynucleotides may also be synthesized by well-known techniques as described in the technical literature. See, *e.g*., Carruthers et al. (1982) Cold Spring Harbor Symp. Quant. Biol. 47:411-418, and Adams et al. (1983) J. Am. Chem. Soc. 105:661. Double stranded DNA segments may then be obtained either by synthesizing the complementary strand and annealing the strands together under appropriate conditions, or by adding the complementary strand using DNA polymerase with an appropriate primer sequence.

Oligonucleotides for use as probes, *e.g.,* in *in vitro* amplification methods, for use as gene probes are typically synthesized chemically according to the solid phase phosphoramidite triester method described by Beaucage and Caruthers (1981) Tetrahedron Letts. 22(20):1859-1862, *e.g.*, using an automated synthesizer, as described in Needham-VanDevanter et al. (1984) Nucleic Acids Res. 12:6159-6168. Oligonucleotides for use in the nucleic acid constructs or vectors that are utilized in certain embodiments of the invention can also be custom made and ordered from a variety of commercial sources known to persons of skill.

### CONSTRUCTION OF EXPRESSION CASSETTES AND VECTORS

Essentially any known vector or vector system can be used to create a transformed plant or plant cell of the invention. Any vector or vector system known in the art can be used with the invention. For example, vectors in the form of plasmids or plasmid systems (*e.g*., binary systems, trinary systems, shuttle vector systems, etc.) can e used. Certain exemplary plasmid systems that are optionally adapted for use in the present invention are described in, *e.g*., U.S. Patent Serial Nos. 5,977,439 to Hamilton (issued November 2, 1999), 5,929,306 to Torisky et al. (issued July 27, 1999), 5,149,645 to Hoekema et al. (issued September 22, 1992), 6,165,780 to Kawasaki (issued December 26, 2000), 6,147,278 to Rogers et al. (issued November 14, 2000), 4,762,785 to Comai (issued August 9, 1988), and 5,068,193 to Comai (issued November 26, 1991), which are each incorporated by reference. The construction of vectors is described further in the examples provided below.

In some embodiments, the vector or vector system used comprises a novel regulatory sequence of the invention (*e.g*., a promoter or terminator sequence of Table 1), in which case the operably joined expressed sequence of interest is not particularly limited.

In other aspects, the vector or vector system used comprises a novel cDNA or partial cDNA sequence of the invention (e.g., a cDNA selected from an ISO cDNA, a PSY cDNA, an LYC cDNA, or a partial cDNA from the pineapple ACC synthase gene 3' region, as listed in Table 2), or a portion thereof, in either sense or antisense orientation), in which case the operably joined promoter and/or terminator sequences used are not particularly limited. In certain embodiments, nucleic acid segments that encode polypeptides (*e.g*., a polynucleotide having a sequence that corresponds to SEQ ID NO: 1 and/or 11, that encode the polypeptides of SEQ ID NO: 9 and/or 13) are operatively linked to promoter sequences in the vectors.

In some embodiments, the vector or vector system used comprises both a novel regulatory sequence of the invention (*e.g*., a promoter and/or terminator sequence of Table 1) and a novel cDNA or partial cDNA sequence of the invention (e.g., a cDNA selected from an ISO cDNA, a PSY cDNA or a LYC cDNA, as shown in Table 2, or a portion thereof, in either sense or antisense orientation).

The nucleic acid segments optionally utilized herein, *e.g*., in the form of expression cassettes typically include in the 5' to 3' direction of transcription, a transcriptional and translational initiation region, a DNA sequence of interest (*e.g*., a gene encoding a polypeptide), and a transcriptional and translational termination region functional in the particular plant being transformed. The termination region may be native with the transcriptional initiation region, may be native with the DNA sequence of interest, or may be derived from another source. Convenient termination regions are available from the Ti-plasmid of *A*. *tumefaciens,* such as the octopine synthase and nopaline synthase termination regions. See also, Guerineau et al., (1991), Mol. Gen. Genet., 262:141-144; Proudfoot, (1991), Cell, 64:671-674; Sanfacon et al., (1991), Genes Dev., 5:141-149; Mogen et al., (1990), Plant Cell, 2:1261-1272; Munroe et al., (1990), Gene, 91:151-158; Ballas et al., (1989), Nucleic Acids Res., 17:7891-7903; and Joshi et al., (1987), Nucleic Acid Res., 15:9627-9639), which are each incorporated by reference.

In certain embodiments, the nucleic acid segments of interest will be targeted to plastids, such as chloroplasts, for expression. In this manner, where the nucleic acid segment is not directly inserted into the plastid, the expression cassette will additionally contain a gene encoding a transit peptide to direct the nucleic acid of interest to the plastid. Such transit peptides are known in the art. See, *e.g*., Von Heijne et al. (1991) Plant Mol. Biol. Rep. 9:104-126; Clark et al. (1989) J. Biol Chem. 264:17544-17550; della-Cioppa et al. (1987) Plant Physiol. 84:965-968; Romer et al. (1993) Biochem. Biophys. Res Commun. 196:1414-1421; and, Shah et al. (1986) Science 233:478-481, which are each incorporated by reference. Plant genes useful in the invention may utilize native or heterologous transit peptides.

Constructs optionally utilized in performing the methods of the invention may also include any other necessary regulators such as plant translational consensus sequences (Joshi, C. P., (1987), Nucleic Acids Research, 15:6643-6653), introns (Luehrsen and Walbot, (1991), Mol. Gen. Genet., 225:81-93) and the like, operably linked to the nucleotide sequence of interest.

In some embodiments, 5' leader sequences are included in expression cassette constructs utilized in performing the methods of the invention. Such leader sequences can act to enhance translation. Translation leaders are known in the art and include: picornavirus leaders, for example, EMCV leader (*Encephalomyocarditis 5'* noncoding region) (Elroy-Stein et al. (1989) PNAS USA 86:6126-6130); *potyvirus* leaders, for example, TEV leader (Tobacco Etch Virus) (Allison et al., (1986); MDMV leader (Maize Dwarf Mosaic Virus); Virology, 154:9-20), and human immunoglobulin heavychain binding protein (BiP), (Macejak, D. G., and Sarnow, P., (1991), Nature, 353:90-94; untranslated leader from the coat protein mRNA of alfalfa mosaic virus (AMV RNA 4), (Jobling, S. A., and Gehrke, L., (1987), Nature, 325:622-625; tobacco mosaic virus leader (TMV), (Gallie, D. R. et al., (1989), Molecular Biology of RNA, pages 237-256; and maize chlorotic mottle virus leader (MCMV) (Lommel, S. A. et al., (1991), Virology, 81:382-385. See also, Della-Cioppa et al., (1987), Plant Physiology, 84:965-968, which are each incorporated by reference.

Depending upon where the DNA sequence of interest is to be expressed, it may be desirable to synthesize the sequence with plant preferred codons, or alternatively with chloroplast preferred codons. Plant preferred codons may be determined from the codons of highest frequency in the proteins expressed in the largest amount in the particular plant species of interest. See, European Patent Application Nos. 0359472 and 0385962; International Application No. WO 91/16432; Perlak et al. (1991) Proc. Natl. Acad. Sci. USA 88:3324-3328; and Murray et al. (1989) Nucleic Acids Res. 17: 477-498, which are each incorporated by reference. In this manner, the nucleotide sequences can be optimized for expression in plants of interest. It is recognized that all or any part of the gene sequence may be optimized or synthetic. That is, synthetic or partially optimized sequences may also be used. For the construction of chloroplast preferred genes, see *e.g*., U.S. Pat. No. 5,545,817, which is incorporated by reference.

In preparing expression cassettes, the various nucleic acid fragments may be manipulated, so as to provide for the nucleic acid sequences in the proper orientation and, as appropriate in the proper reading frame. Towards this end, adapters or linkers may be employed to join the nucleic acid fragments or other manipulations may be involved to provide for convenient restriction sites, removal of superfluous nucleic acid segments, removal of restriction sites, or the like. For this purpose, *in vitro* mutagenesis, primer repair, restriction, annealing, resection, ligation, or the like may be employed, where insertions, deletions or substitutions, *e.g*., transitions and transversions, may be involved.

The vectors optionally utilized in performing the methods described herein may include expression control elements, such as promoters (*e.g*., a polynucleotide having a sequence that corresponds to SEQ ID NO: 2-8 or 10, etc.). Polypeptide coding nucleic acids (*e.g*., a polynucleotide having a sequence that corresponds to SEQ ID NO: 1 or 11, or another nucleic acid of interest) are operatively linked to the expression vector to permit the promoter sequence to direct RNA polymerase binding and expression of the polypeptide coding gene. Useful in expressing the polypeptide coding gene are promoters which are inducible, viral, synthetic, constitutive as described in, *e.g*., Poszkowski et al. (1989) EMBO J. 3:2719 and Odell et al. (1985) Nature 313:810 (1985), and temporally regulated, spatially regulated, and spatiotemporally regulated as described in, *e.g*., Chua et al. (1989) Science 244:174-181.

The choice of which expression vector and, *e.g*., to which preselected organ-enhanced promoter a polypeptide coding gene is operatively linked typically depends on the functional properties desired, *e.g*., the location and timing of protein expression. A vector that is useful in practicing the present invention integrates into the genome of the plant of interest, is capable of directing the replication, and also the expression of the polypeptide coding gene included in the nucleic acid segment to which it is operatively linked. It is well known in the art that the entire expression vector does not integrate into the host plant genome, but only a portion integrates. Nonetheless, the vector will be said to integrate for ease of expression.

In some embodiments, as mentioned herein, a construct utilized in performing the methods of the invention may include elements in addition to the conjoined nucleic acid sequences, such as promoters, enhancer elements, and signaling sequences. Exemplary promoters include the CaMV promoter, a promoter from the ribulose-1,5-bisphosphate carboxylase-oxygenase small subunit gene, a ubiquitin promoter, a rolD promoter, and others referred to herein or otherwise known to those of skill in the art. Exemplary enhancer elements are described in, *e.g*., U.S. Pat. No. 6,271,444, which issued Aug. 7, 2001 to McBride et al. Exemplary signaling sequences include, but are not limited to, nucleic acid sequences encoding tissue-specific transit peptides, such as chloroplast transit peptides (see, *e.g*., Zhang et al. (2002) Trends Plant Sci 7(1):14-21).

In certain embodiments, a strongly or weakly constitutive plant promoter can be employed which will direct expression of the encoded sequences in all tissues of a plant. Such promoters are active under most environmental conditions and states of development or cell differentiation. Examples of constitutive promoters include the 1'- or 2'-promoter derived from T-DNA of *Agrobacterium tumefaciens,* and other transcription initiation regions from various plant genes known to those of skill. In situations in which overexpression of a gene is undesirable, a weak constitutive promoters can be used for lower levels of expression. In instances where high levels of expression are sought, a strong promoter, *e.g*., a t-RNA or other pol III promoter, or a strong pol II promoter, such as the cauliflower mosaic virus promoter, can be used.

Alternatively, a plant promoter may be under environmental control. Such promoters are referred to here as "inducible" promoters. Examples of environmental conditions that may effect transcription by inducible promoters include pathogen attack, anaerobic conditions, or the presence of light.

In some embodiments, promoters incorporated into a construct optionally used to perform the methods of the present invention are "tissue-specific" and, as such, under developmental control in that the desired gene is expressed only in certain tissues, such as fruit-tissues. In embodiments in which one or more nucleic acid sequences endogenous to the plant are incorporated into a construct, the endogenous promoters (or variants thereof) from these genes can be employed for directing expression of the genes in the transformed plant. Tissue-specific promoters can also be used to direct expression of heterologous structural genes, including the artificially evolved nucleic acids.

In addition to the promoters noted above, promoters of bacterial origin which operate in plants include the octopine synthase promoter, the nopaline synthase promoter and other promoters derived from native Ti plasmids (see, Herrara-Estrella et al. (1983) Nature 303:209-213). Viral promoters include the 35S and 19S RNA promoters of cauliflower mosaic virus (Odell et al. (1985) Nature 313:810-812). Other plant promoters include the ribulose-1,3-bisphosphate carboxylase small subunit promoter and the phaseolin promoter. The promoter sequence from the E8 gene and other genes may also be used. The isolation and sequence of the E8 promoter is described in detail in Deikman and Fischer (1988) EMBO J. 7:3315-3327.

In preparing constructs utilized in performing the methods of the invention, sequences other than the promoter and the conjoined nucleic acid segment can also be employed. If normal polypeptide expression is desired, a polyadenylation region at the 3'-end of the coding region can be included. The polyadenylation region can be derived from the natural gene, from a variety of other plant genes, or from T-DNA.

Typical vectors useful for expression of genes in plants are well known in the art and include vectors derived from the tumor-inducing (Ti) plasmid of *Agrobacterium tumefaciens* described by Rogers et al. (1987) Meth. in Enzymol., 153:253-277 (1987). These vectors are plant integrating vectors in that on transformation, the vectors integrate a portion of vector DNA into the genome of the plant. For integrating vectors based on the Ti plasmid, the region integrated into the host plant chromosomes is that between the right and left borders of the Ti plasmid.

Exemplary *A*. *tumefaciens* vectors useful herein are plasmids pKYLX6 and pKYLX7 of Schardl et al. (1987) Gene 61:1-11 and Berger et al. (1989) Proc. Natl. Acad. Sci. U.S.A., 86:8402-8406. Plasmid pKYLX6 is an *E. coli* vector designed for intermediate constructs, whereas plasmid pKYLX7 is an *A*. *tumefaciens* vector designed for integration of cloned genes. Modified vectors pKYLX61 and pKYLX71 contain HindIII, XhoI, BamHI, PstI and SstI sites in place of the original HindIII-SstI fragment multiple cloning site region. Another useful vector herein is plasmid pBI101.2 that is available from Clontech Laboratories, Inc., Palo Alto, Calif. Plasmids pKYLX7, pKYLX71 and pB7101.2 are binary vectors that are used in *A*. *tumefaciens* with another vector having a vir gene. Additional details relating to binary vectors are described in, *e.g*., Hellens et al. (2000) "pGREEN: a versatile and flexible Ti vector for Agrobacterium-mediated plant transformation," Plant Molecular Biology 42:819-832. Other vectors systems are also optionally utilized herein including, *e.g*., trinary vector systems. Another plant transformation system is based on *Agrobacterium rhizogenes* that induces hairy roots rather than a tumor on transformation. See, *e.g*., International Publication No. WO 88/02405 (published Apr. 7, 1988) describes the use of *A*. *rhizogenes* strain A4 and its Ri plasmid along with *A*. *tumefaciens* vectors pARC8 or pARC16 to transform plants. Agrobacterium-mediated transformation is described further below.

Retroviral expression vectors are also optionally adapted for use in performing the methods described herein. The term "retroviral expression vector," as used herein, refers to a DNA molecule that includes a promoter sequence derived from the long terminal repeat (LTR) region of a retrovirus genome. Because some of the nucleic acid segment expression products that may be utilized herein are associated with food production and coloration, the retroviral expression vector is preferably replication-incompetent in eukaryotic cells. The construction and use of retroviral vectors has been described by, *e.g*., Verma in International Publication No. WO 87/00551, and in Cocking et al. (1987) Science 236:1259-62, which are both incorporated by reference.

In some embodiments, the vector used to express a polypeptide coding gene includes a plant selectable marker that confers a selectable phenotype on the transformed cell. The selectable plant marker gene on the DNA segment to be inserted will usually encode a function, which permits the survival and emergence of transformed organogenic cells or tissue in a selective medium. Usually, the selectable marker gene will encode antibiotic resistance, with suitable genes including those coding for resistance to the antibiotic spectinomycin (*e.g*., the aadA gene), the streptomycin phosphotransferase (SPT) gene coding for streptomycin resistance, the neomycin phosphotransferase (NPTII) gene encoding kanamycin or geneticin resistance, the hygromycin phosphotransferase (HPT) gene coding for hygromycin resistance, genes coding for resistance to herbicides which act to inhibit the action of acetolactate synthase (ALS), in particular the sulfonylurea-type herbicides (*e.g*., the acetolactate synthase (ALS) gene containing mutations leading to such resistance in particular the S4 and/or Hra mutations), genes coding for resistance to herbicides which act to inhibit action of glutamine synthase, such as phosphinothricin or basta (*e.g*., the bar gene), or other such genes known in the art. The bar gene encodes resistance to the herbicide basta, the nptII gene encodes resistance to the antibiotics kanamycin and geneticin, and the ALS gene encodes resistance to the herbicide chlorsulfuron. Selection based on resistance to sulfonylurea-type herbicides is preferred. Selectable markers based on the green fluorescent protein (GFP) or ß-glucuronidase (GUS) are also optionally used and are described further in, *e.g*., Mantis et al. (2000) "Comparing the utility of ß-glucuronidase and green fluorescent protein for detection of weak promoter activity in Arabidopsis thaliana," Plant Molecular Biology Reporter 18:319-330, which is incorporated by reference.

Methods to select transformed plant cells incorporating a desired resistance gene are well known in the art. For example, if the marker is sulfonylurea resistance, the selection medium generally contains a sulfonylurea-type herbicide at an appropriate concentration (*e.g*., chlorsulfuron in the range of about 1-1000 µg/l, and more typically in the range of about 5-100 µg/l). For selection of geneticin resistant plant cells or tissue, which contain the NPTII gene, geneticin is typically included in the medium at 10-50 mg/l. Spectinomycin resistant plant cells or tissue containing the aadA gene are typically selected on medium containing 200-1000 mg/l spectinomycin.

In certain embodiments, transformed cells and plants are selected according to visual differentiation. For example, since many carotenoids are colored, these carotenoid products can be visualized and determined by their characteristic spectra and other analytic methods. Therefore, genes encoding carotenoid biosynthetic enzymes may be used as marker genes to allow for visual selection of transformants. In particular, such transformed cells generally display colors ranging from yellow to orange to red as a result of the increased carotenoid levels. In some embodiments, other analytical techniques can be used to select transformed cells including, *e.g*., mass spectrometry, thin layer chromatography (TLC), high pressure liquid chromatography (HPLC), capillary electrophoresis (CE), NMR spectroscopy, and conventional hybridization techniques.

### METHODS OF PRODUCING TRANSFORMED PLANT CELLS, CELL CULTURE, AND EXPLANT SOURCES

The present invention also relates to methods of genetically transforming cells and plants. Exemplary plant traits that can be modified using the methods described herein include fruit quality (*e.g*., sweetness, acidity, texture, condition, color (*e.g*., shell or tissue color or the like), etc.), fruit ripening characteristics, nutritional value (*e.g*., modified carotenoid levels, etc.), among many other traits typically of interest to consumers. Optionally, other agronomic traits such as, improved flowering control, improved resistance to drought, improved resistance to bacterial diseases, improved resistance to viral diseases, and/or improved resistance to insects, nematodes, and herbicides are also engineered into the cells and plants of the invention.

In certain embodiments, the methods of the invention include the use of suitable explant material, which is genetically transformed by contacting the explant material with *Agrobacterium* cells. The *Agrobacterium* cells mediate the transfer of nucleic acid segments, *e.g*., that encode polypeptides, into plant cells. Other techniques for delivering nucleic acid segments into cells or plants are also optionally utilized, e.g, biolistics, as described and demonstrated in the Examples. The invention also provides culture media (see, Example 15) suitable for the steps of inducing the formation of organogenic or other cells for co-cultivation with *Agrobacterium* cells. Methods of transforming plants are also illustrated in the examples provided below.

To further illustrate, in some embodiments, nucleic acid segments that encode polypeptides (*e.g*., plant polypeptides, bacterial polypeptides, etc.) are introduced into plant cells. In certain of these embodiments, the polypeptide is heterologous to the cells. In some embodiments, the polypeptide is homologous to an endogenous polypeptide of the cells. Essentially any nucleic acid segment is optionally utilized to transform the organogenic cells according to the methods described herein. Accordingly, no attempt is made to identify all of the known nucleic acids that can be utilized. However, to illustrate, some carotenogenesis-related nucleic acid segments that are optionally introduced into cells or plants typically encode, *e.g*., isopentenyl diphosphate isomerases, geranylgeranyl pyrophosphate synthases, phytoene synthases, phytoene desaturases, ζ-carotene desaturases, lycopene β-cyclases, lycopene ε-cyclases, β-carotene hydroxylases, ε-hydroxylases, and/or the like. Additional details relating to these carotenogenesis-related nucleic acid segments are described in, *e.g*., International Patent Application Publication No. WO 2004/052085, entitled "TRANSGENIC PINEAPPLE PLANTS WITH MODIFIED CAROTENOID LEVELS AND METHODS OF THEIR PRODUCTION," filed December 5, 2003 by Young et al. and International Patent Application Publication No. WO 2004/053082, entitled "ORGANOGENIC TRANSFORMATION AND REGENERATION," filed December 8, 2003 by Firoozabady, which are both incorporated by reference. Other exemplary nucleic acid segments optionally comprise or encode, *e.g*., an ACC synthase, an ACC oxidase, a malic enzyme, a malic dehydrogenase, a glucose oxidase, a chitinase, a defensin, an expansin, a hemicellulase, a xyloglucan transglycosylase, an apetala gene, a leafy gene, a knotted-related gene, a homeobox gene, an Etr-related gene, a ribonuclease, and/or the like.

In one aspect, the invention provides a method of producing transformed plant cells that includes culturing at least one non-apical meristemic cell to produce one or more organogenic cells, and introducing at least one nucleic acid segment into the organogenic cells to produce one or more transformed organogenic cells. In another aspect, the invention relates to a method of producing transformed plant cells that includes culturing at least one meristemic cell to produce at least one shoot. In addition, the method also includes culturing at least one explant from the shoot to produce one or more organogenic cells, and introducing at least one nucleic acid segment into the organogenic cells to produce one or more transformed organogenic cells. The methods described herein also typically further include generating at least one plant from the transformed organogenic cells.

In some embodiments, the methods of the invention include culturing meristemic cells (*e.g*., non-apical meristemic cells). In certain embodiments, for example, meristemic cells are derived from monocotyledonous plants, whereas in others, meristemic cells are derived from dicotyledonous plants. To further illustrate, meristemic cells are optionally derived from plants selected from the genera: *Ananas, Musa, Vitis, Fragaria, Lotus, Medicago, Onobrychis, Trifolium, Trigonella, Vigna, Citrus, Carica, Persea, Prunus, Syragrus, Theobroma, Coffea, Linum, Geranium, Manihot, Daucus, Arabidopsis, Brassica, Raphanus, Sinapis, Atropa, Capsicum, Datura, Hyoscyamus, Lycopersicon, Nicotiana, Solanum, Petunia, Digitalis, Majorana, Mangifera, Cichorium, Helianthus, Lactuca, Bromus, Asparagus, Antirrhinum, Heterocallis, Nemesia, Pelargonium, Panicum, Pennisetum, Ranunculus, Senecio, Salpiglossis, Cucurbita, Cucumis, Browaalia, Lolium, Malus, Apium, Gossypium, Vicia, Lathyrus, Lupinus, Pachyrhizus, Wisteria, Stizolobium, Agrostis, Phleum, Dactylis, Sorgum, Setaria, Zea, Oryza, Triticum, Secale, Avena, Hordeum, Saccharum, Poa, Festuca, Stenotaphrum, Cynodon, Coix, Olyreae, Phareae, Glycine, Pisum, Psidium, Passiflora, Cicer, Phaseolus, Lens, Arachis*, or the like.

To further exemplify, essentially any pineapple variety may be transformed according to the methods described herein. For example, pineapple merstemic cells are optionally obtained from varieties that are typically used for human consumption, including those of the Smooth Cayenne group, the Spanish group (*e.g*., Red Spanish), the Perolera group, the Pernambuco group, and the Primavera group. The most important variety for use in the production of canned pineapple, other processed pineapple products, and fresh pineapple is typically Smooth Cayenne. Within many of these varieties there are a large number of clones which have been established in different geographical areas, and which are adapted to production in those locations. Among the Smooth Cayenne clones are the Champaka clones which have been used extensively for production of canned and fresh pineapple.

In certain embodiments, rapidly growing shoot cultures are produced *in vitro,* then explants such as leaf pieces, petioles, cotyledons, stem sections, peduncles, etc. are cultured to produce meristemic or organogenic cells, *e.g*., in pretreatment processes that prepare cell and/or tissues for cocultivation with *Agrobacterium* cells. Initial explants can be any meristemic region of a plant, including either the main or axillary meristems (apices) of the plant prior to, *e.g*., flower formation, and the main or axillary meristems of, *e.g.,* the crown of the fruit in pineapple or another plant. These regions can be excised from the plant and sterilized by standard methods as described herein and well known to those of ordinary skill in the art to establish sterile cultures in an artificial medium. Such cultures can be maintained for an extended period of time (*e.g*., weeks, months or years) by a series of propagation steps. Suitable media for establishment and maintenance of *in vitro* shoot cultures are described in, e.g., DeWald et al. (1988) Plant Cell Reports, 7:535-537; Wakasa et al. (1978) Japan J Breed 28:113-121; Mathews and Rangan (1981) Scientia Hort 14:227-234; Srinivasa et al. (1981) Scientia Hort 15: 23S-238; Fitchet (1990) Acta Hort 275:267-274; Bordoloi and Sarma (1993) J Assam Science Society 35:41-45; and Firoozabady and Moy (2004) "Regeneration of pineapple plants via somatic embryogenesis and organogenesis," In Vitro Cellular and Developmental Biology - Plant 40(1). Additional details relating to culturing plant cells, including pretreatment processes, are provided below in the examples.

One skilled in the art will recognize that many different types of cells can be used as target cells for the delivery of nucleic acid segments and selection of transformation events. For example, nucleic acid segments can be delivered to the cells of the leaf, leaf base, or stem sections as they undergo organogenesis. In particular, nucleic acid segments can be delivered to organogenic cells after the organogenic material has been maintained and proliferated *in vitro* for a selected period of time. In certain embodiments, as referred to above, the plant cells which are the target for nucleic acid segment delivery are first obtained from the basal portion of leaves (i.e., leaf base) or sections of the stem of shoots grown *in vitro,* and proliferated in culture prior to the nucleic acid segment delivery step. As used herein, the term "leaf base" refers to that portion of the leaf that is connected to the stem of a shoot.

Additional details relating to cell culture are described in, *e.g*., Published International Application No. WO 01/33943, entitled "A METHOD OF PLANT TRANSFORMATION," by Graham et al., which published May 17, 2001, U.S. Pat. No. 5,908,771, entitled "METHOD FOR REGENERATION OF SALVIA SPECIES," which issued June 1, 1999 to Liu et al., U.S. Pat. No. 6,242,257, entitled "TISSUE CULTURE PROCESS FOR PRODUCING A LARGE NUMBER OF VIABLE COTTON PLANTS IN VITRO," which issued June 5, 2001 to Tuli et al., Croy (Ed.) Plant Molecular Biology Labfax, Bios Scientific Publishers Ltd. (1993), Jones (Ed.) Plant Transfer and Expression Protocols, Humana Press (1995), and in the references cited therein.

Nucleic acid segments can be introduced into cells in a number of art-recognized ways. In overview, suitable methods of transforming plant cells include microinjection (Crossway et al. (1986) BioTechniques 4:320-334), electroporation (Riggs et al. (1986) Proc. Natl. Acad. Sci. USA 83:5602-5606, Agrobacterium-mediated transformation (Hinchee et al. (1988) Biotechnology 6:915-921), ballistic particle acceleration or microprojectile bombardment (Sanford et al. U.S. Pat. No. 4,945,050; and McCabe et al. (1988) Biotechnology 6:923-926), pollen-mediated delivery (Zhou et al. (1983) Methods Enzymol. 101:433, De Wet et al. (1985) in The Experimental Manipulation of Ovule Tissues, Chapman et al. (Eds.), Longman, p. 197; Hess (1987) Intern. Rev. Cytol. 107:367; and Luo et al. (1989) Plant Mol. Biol. Rep. 7:69), direct nucleic acid transfer to protoplasts of pineapple cells (Caboche et al. (1984) Comptes Rendus Acad. Sci. 299, series 3:663), microinjection (Crossway et al. (1986) Mol. Gen. Genet. 202:179 and Reich et al. (1986) Bio/technol. 4:1001), macroinjection of inflorescence (De la Pena et al. (1987) Nature 325:274), whisker-mediated impregnation (Dunahay (1993) Biotechniques 15:452-460 and Frame et al. (1994) The Plant Journal 6:941-948), laser perforation (Weber (1988) Naturwissenschaften 75:35), and ultrasonification (Zhang et al. (1991) Bio/technol. 9:994).

To further illustrate, *Agrobacterium*-mediated transfer is a widely applicable system for introducing genes into plant cells because the nucleic acid segments can be introduced into whole plant tissues, thereby bypassing the need for regeneration of an intact plant from a protoplast. The use of *Agrobacterium*-mediated expression vectors to introduce DNA into plant cells is well known in the art. See, *e.g*., the methods described by Fraley et al. (1985) Biotechnology, 3:629 and Rogers et al. (1987) Methods in Enzymology, 153:253-277. Further, the integration of T-DNA is a relatively precise process resulting in few rearrangements. The region of DNA to be transferred is defined by the border sequences, and intervening DNA or nucleic acid segment is usually inserted into the plant genome as described by Spielmann et al. (1986) Mol. Gen. Genet., 205:34 and Jorgensen et al. (1987) Mol. Gen. Genet. 207:471.

Many *Agrobacterium* transformation vectors are capable of replication in *E*. *coli* as well as *Agrobacterium*, allowing for convenient manipulations as described by Klee et al., in Plant DNA Infectious Agents, Hohn and Schell, (Eds.), Springer-Verlag (1985) pp. 179-203.

Moreover, technological advances in vectors for *Agrobacterium*-mediated gene transfer have improved the arrangement of genes and restriction sites in the vectors to facilitate construction of vectors capable of expressing various polypeptide coding genes. For example, the vectors described by Rogers et al. (1987) Methods in Enzymology, 153:253, have convenient multi-linker regions flanked by a promoter and a polyadenylation site for direct expression of inserted polypeptide coding genes and are suitable for present purposes. Suitable vectors are described in greater detail herein.

In certain embodiments of the invention, heterologous nucleic acid segments are introduced using *Agrobacterium* strains carrying the exogenous DNA in a T-DNA element. The recombinant T-DNA element can either be part of a Ti-plasmid that contains the virulence functions necessary for DNA delivery from *Agrobacterium* cells to plant cells, or the T-DNA element can be present on a plasmid distinct from another plasmid carrying the virulence functions (referred to as binary vectors). A variety of these binary vectors, capable of replication in both *E. coli* and *Agrobacterium,* are described in the references cited above. In certain methods of co-cultivation, *Agrobacterium* is grown to a concentration of 2-7 x 10⁸ cells/ml and is diluted to 1-6 x 10⁸ cells/ml, preferably 2-5 x 10⁸ cells/ml before co-cultivation. *Agrobacterium* is typically co-cultivated with plant tissues for about 1-7 days, and more typically for about 2-3 days with, *e.g*., certain plant tissues, such as pineapple tissues.

Suitable *Agrobacterium* strains include *Agrobacterium tumefaciens* and *Agrobacterium rhizogenes.* While wild-type strains may be used, "disarmed" derivatives of both species, in which the tumor-inducing sequences of the Ti plasmid have been removed, are preferred. Suitable *Agrobacterium tumefaciens* strains include, *e.g*., EHA101, as described by Hood et al. ((1986) J. Bacteriol., 168:1291-1301), LBA4404, as described by Hoekema et al. ((1983) Nature, 303:179-80), and C58(pMP90), as described by Koncz and Schell ((1986) Mol. Gen. Genet., 204:383-96). A preferred *Agrobacterium rhizogenes* strain is 15834, as described by Birot et al. (Biochem, 25: 323-35).

The plant cells and tissues and the *Agrobacterium* cells carrying the nucleic acid segment are co-cultivated in a suitable co-cultivation medium to allow transfer of the T-DNA to plant cells. After the *Agrobacterium* strain carrying the nucleic acid segment has been prepared, it is usually cultured prior to incubation with the cells. *Agrobacterium* can be cultured on solid or liquid media according to methods well known to those of skill in the art. See, *e.g*., U.S. Pat. No. 5,262,316, which is incorporated by reference.

As additional options, transformation of plant protoplasts can be achieved using methods based on calcium phosphate precipitation, polyethylene glycol treatment, electroporation, and combinations of these treatments. See, *e.g.*, Potrykus et al. (1985) Mol. Gen. Genet., 199:183; Lorz et al. (1985) Mol. Gen. Genet., 199:178; Fromm et al. (1986) Nature, 319:791; Uchimiya et al. (1986) Mol. Gen. Genet., 204:204; Callis et al. (1987) Genes and Development, 1:1183; Marcotte et al. (1988) Nature, 335:454; Wang et al. (1992) Bio/Technology, 10:691-696; and Fennell et al. (1992) Plant Cell Reports, 11:567-570, which are each incorporated by reference.

To transform plant species that cannot be successfully regenerated from protoplasts, other ways to introduce nucleic acid segments into intact cells or tissues can be utilized. For example, "particle gun" or high-velocity microprojectile technology can be utilized. Using such technology, nucleic acid segments are carried through the cell wall and into the cytoplasm on the surface of small metal particles as described in Klein et al. (1987) Nature, 327:70; Klein et al. (1988) Proc. Natl. Acad. Sci. U.S.A., 85:8502; and McCabe et al. (1988) Biotechnology, 6:923; and Vasil et al. (1992) Bio/Technology, 9:667-674. The metal particles penetrate through several layers of cells and thus allow for the transformation of cells within tissue explants. Transformation of tissue explants eliminates the need for passage through a protoplast stage and thus speeds the production of transgenic plants.

Nucleic acid segments are also optionally introduced into plants in performing the methods of the invention by direct nucleic acid transfer into pollen as described by Zhou et al. (1983) Methods in Enzymology 101:433; Hess (1987) Intern Rev. Cytol. 107:367; Luo et al. (1988) Plant Mol. Biol. Reporter 6:165. Expression of polypeptide coding genes can be obtained by injection of the nucleic acid segment into reproductive organs of a plant as described by Pena et al. (1987) Nature 325:274.

Alternatively, a plant plastid can be transformed directly in performing the methods described herein. Stable transformation of chloroplasts has been reported in higher plants, see, *e.g*., Svab et al. (1990) Proc. Nat'l. Acad. Sci. USA 87:8526-8530; Svab et al. (1993) Proc. Nat'l Acad. Sci. USA 90:913-917; Staub et al. (1993) Embo J. 12:601-606. The method utilizes particle gun delivery of nucleic acid segments containing a selectable marker and targeting of the nucleic acid to the plastid genome through homologous recombination. In such methods, plastid gene expression can be accomplished by use of a plastid gene promoter or by trans-activation of a silent plastid-borne transgene positioned for expression from a selective promoter sequence such as that recognized by T7 RNA polymerase. The silent plastid gene is activated by expression of the specific RNA polymerase from a nuclear expression construct and targeting of the polymerase to the plastid by use of a transit peptide. Tissue-specific expression may be obtained in such a method by use of a nuclear-encoded and plastid-directed specific RNA polymerase expressed from a suitable plant tissue specific promoter. Such a system has been reported in McBride et al. (1994) Proc. Natl. Acad. Sci. USA 91:7301-7305, which is incorporated by reference.

After delivery of nucleic acid segments, plant cells (*e.g*., embryogenic plants, organogenic plant cells, etc.) are typically transferred to media that include a selective agent (*e.g*., an herbicide or the like) that is capable of preventing the growth of cells that have not received a gene (*e.g*., a selectable marker) whose expression product is capable of preventing the action of the selective agent to thereby select for transformed plant cells. In certain embodiments, for example, tissues are exposed to sublethal levels of selective agents for about 2-12 weeks, and then to lethal levels of selective agents for about 4-30 weeks in a step-wise selection process. Selectable markers are described further herein. In certain embodiments, plant cells are transferred to a recovery medium that comprises counter-selective agents (*e.g*., antibiotics, etc.), *e.g.,* to kill *Agrobacterium* cells for a period of about 1-15 days, *e.g*., prior to or concurrently with being transferred to media comprising a selective agent. After a period of culture, plant cells that continue to grow normally are separated from cells whose growth has been slowed or terminated.

The regeneration of plants from either single plant protoplasts or various explants is well known in the art. See, *e.g*., Weissbach et al. (Eds.), Methods for Plant Molecular Biology, Academic Press, Inc. (1988). In certain embodiments of the invention, the regeneration and growth process includes the steps of selecting transformed cells and shoots, rooting the transformed shoots, and growing the plantlets in soil. To illustrate, the regeneration of plants containing a gene introduced by *Agrobacterium* from leaf explants can be achieved as described by Horsch et al. (1985) Science, 227:1229-1231. In this procedure, transformants are grown in the presence of a selection agent and in a medium that induces the regeneration of shoots in the plant species being transformed as described by Fraley et al. (1983) Proc. Natl. Acad. Sci. U.S.A., 80:4803. This procedure typically produces shoots within two to four weeks and these transformed shoots are then transferred to an appropriate root-inducing medium containing the selective agent and an antibiotic to prevent bacterial growth. In pineapples, for example, leaf bases may be used to produce organogenic materials (Firoozabady and Moy (2004) "Regeneration of pineapple plants via somatic embryogenesis and organogenesis," In Vitro Cellular and Developmental Biology - Plant 40(1), which is incorporated by reference), and then these materials may be exposed to *Agrobacterium* to produce, upon selection, transgenic organogenic materials. These materials then are induced to produce shoots and complete plants. Typically, transformed shoots that rooted in the presence of the selective agent to form plantlets are then transplanted to soil or other media to allow the production of additional roots.

Additional details relating to plant regeneration, micropropagation, and other aspects that are adapted for use in the methods of the present invention are provided in, *e.g*., Firoozabady et al., "Transformation and regeneration of pineapple," Plant Cell, Tissue and Organ Culture 84(1):1-16 (2005); U.S. Patent Nos. 5,952,543; 5,591,616 and 6,037,522, International Application Publication Numbers WO01/33943 and WO01/12828, and European Patent Application Nos. 604662 (A1) and 672752 (A1), each of which are incorporated by reference. See also, Kyte et al., Plants from Test Tubes: An Introduction to Micropropagation, Timber Press, Inc. (1996), Hudson et al., Hartmann and Kester's Plant Propagation : Principles and Practices 7th Ed., Pearson Education (2001), Bajaj (Ed.) High-Tech and Micropropagation I, Springer-Verlag New York, Inc. (1992), Jain, In Vitro Haploid Production in Higher Plants, Kluwer Academic Publishers (1996), and Debergh et al. (Eds.), Micropropagation: Technology and Application, Kluwer Academic Publishers (1991), which are each incorporated by reference.

Optionally, polypeptides produced in transformed cells or plants can be recovered and purified from transformed cell cultures or transformed plant tissues (e.g., fruit tissues or the like) by any of a number of methods well known in the art, including ammonium sulfate or ethanol precipitation, acid extraction, anion or cation exchange chromatography, phosphocellulose chromatography, hydrophobic interaction chromatography, affinity chromatography, hydroxylapatite chromatography, and lectin chromatography. In some cases the protein will need to be refolded to recover a functional product. In addition to the references noted supra, a variety of purification methods are well known in the art, including, e.g., those set forth in Sandana, Bioseparation of Proteins, Academic Press, Inc. (1997); and Bollag et al., Protein Methods, 2nd Ed., Wiley-Liss, NY (1996); Walker, The Protein Protocols Handbook, Humana Press, NJ (1996); Harris and Angal, Protein Purification Applications: A Practical Approach, IRL Press (1990); Scopes, Protein Purification: Principles and Practice, 3rd Ed., Springer Verlag (1993); and Janson et al., Protein Purification: Principles, High Resolution Methods and Applications, 2nd Ed., Wiley-VCH (1998), which are each incorporated by reference.

### PLANT TRAIT MODULATION STRATEGIES

The nucleic acid segments introduced into plant cells as described herein may contain one or more genes that are chosen to provide new plant traits, to enhance an existing plant trait, or to otherwise modify expression of phenotypes exhibited by the plant. Such traits include herbicide resistance, pesticide resistance, disease resistance, environmental tolerance (e.g., heat, cold, drought, salinity), morphology, growth characteristics, nutritional content, taste, yield, horticultural characteristics, consumer (quality) traits, and the like.

Functional genes to be introduced may be structural genes, which encode polypeptides that impart the desired phenotype. Alternatively, functional genes may be regulatory genes that play roles in transcriptional and/or translational control to suppress, enhance, or otherwise modify the transcription and/or expression of endogenous genes within the plants. In some embodiments, for example, introduced nucleic acid segments encode polypeptide transcription factors, which when expressed in transformed cells effect elevated expression of targeted genes. In other embodiments, introduced nucleic acid segments encode promoters and/or enhancers, which nucleic acid segments homologously recombine with promoters and/or enhancers of endogenous genes to increase or decrease expression of the genes as desired.

To further illustrate, various nucleic acid constructs can be used in a number of techniques to suppress expression of endogenous plant genes, e.g., sense or antisense suppression or ribozymes. Anti-sense RNA inhibition of gene expression has been shown; see, *e.g.*, Sheehy et al. (1988) Proc. Nat. Acad. Sci. USA 85:8805-8809, and Hiatt et al. U.S. Pat. No. 4,801,340. For examples of the use of sense suppression to modulate expression of endogenous genes see, Napoli et al. (1990) The Plant Cell 2:279-289, and U.S. Pat. No. 5,034,323.

Catalytic RNA molecules or ribozymes can also be used to inhibit gene expression, which is optionally used to effect the accumulation of selected products that are upstream in a given biochemical pathway from the gene that is inhibited. It is possible to design ribozymes that specifically pair with virtually any target RNA and cleave the phosphodiester backbone at a specific location, thereby functionally inactivating the target RNA. In carrying out this cleavage, the ribozyme is not itself altered, and is thus capable of recycling and cleaving other molecules, making it a true enzyme. The inclusion of ribozyme sequences within antisense RNAs confers RNA-cleaving activity upon them, thereby increasing the activity of the constructs.

A number of classes of ribozymes have been identified, which are optionally adapted for use in performing the methods described herein. One class of ribozymes is derived from a number of small circular RNAs which are capable of self-cleavage and replication in plants. The RNAs replicate either alone (viroid RNAs) or with a helper virus (satellite RNAs). Examples include RNAs from avocado sunblotch viroid and the satellite RNAs from tobacco ringspot virus, lucerne transient streak virus, velvet tobacco mottle virus, solanum nodiflorum mottle virus, and subterranean clover mottle virus. The design and use of target RNA-specific ribozymes is described in Haseloff et al. (1988) Nature 334:585-591.

For antisense suppression, the introduced sequence also need not be full length relative to either the primary transcription product or fully processed mRNA. Generally, higher homology can be used to compensate for the use of a shorter sequence. Furthermore, the introduced sequence need not have the same intron or exon pattern, and homology of non-coding segments may be equally effective. Normally, a sequence of between about 30 or 40 nucleotides and about 2000 nucleotides should be used, though a sequence of at least about 100 nucleotides is preferred, a sequence of at least about 200 nucleotides is more preferred, and a sequence of at least about 500 nucleotides is especially preferred.

To further illustrate, RNA interference (RNAi), also known as Post-Transcriptional Gene Silencing (PTGS), is also optionally utilized to modulate traits in plants. RNAi is a cellular mechanism that selectively negates the effect of a target gene by destroying messenger RNA. By destroying the targeted mRNA, protein synthesis is interrupted, thereby effectively "silencing" the target gene. In certain embodiments, this process is initiated by double-stranded RNA (dsRNA), where one strand is substantially identical to the target mRNA sequence. Accordingly, in some embodiments of the invention, nucleic acid segments introduced into plant cells as described herein trigger the production of double stranded dsRNA, which is then cleaved into small interfering RNA (siRNA) as part of the RNAi process. This results in the destruction of the target mRNA, thereby effectively silencing expression of the target gene. Additional details relating to RNAi are described in, *e.g.*, U.S. Pat. No. 6,573,099, entitled "GENETIC CONSTRUCTS FOR DELAYING OR REPRESSING THE EXPRESSION OF A TARGET GENE," which issued June 3, 2003 to Graham, and in, *e.g.*, Arenz et al. (2003) "RNA interference: from an ancient mechanism to a state of the art therapeutic application?" Naturwissenschaften. 90(8):345-59, Wang et al. (2003) "RNA interference: antiviral weapon and beyond," World J Gastroenterol. 9(8):1657-61, and Lavery et al. (2003) "Antisense and RNAi: powerful tools in drug target discovery and validation" Curr Opin Drug Discov Devel. 6(4):561-9. Custom nucleic acid segments that can be utilized to effect target gene silencing are also commercially available from various suppliers, such as Ambion, Inc. (Austin, TX, USA), Benitec Australia Limited (St Lucia, AU), and the like.

Often the functional genes to be introduced will be modified from their native form. For example, sense and anti-sense constructs referred to above often have all or a portion of the transcript of the native gene operably linked to a promoter sequence at the 5' end of the transcribable segment, and operably linked to the 3' sequence of another gene (including polyadenylation sequences) at the 3' end of the transcribable segment. As is apparent to those skilled in the art, the promoter sequence could be one of the many plant active sequences already described. Alternatively, other plant-active promoter sequences can be derived specifically to be linked to the transcribable segment. The promoter can be endogenous to pineapple, or can be from an exogenous source such as a cauliflower mosaic virus 35S promoter (Odell et al. (1985) Nature 313:810-812), the ubiquitin 1 promoter (Christiensen et al. (1992) Plant Mol. Biol. 18:675-689), or the Smas promoter (Ni et al. (1995) Plant J. 7:661-676). The 3' end sequence to be added can be derived from, preferably, the nopaline synthase or octopine synthase genes, or alternatively from another plant gene, or less preferably from any other eukaryotic gene.

As described herein, the production of carotenoids can be elevated in cells and plants transformed with nucleic acid segments (*e.g.*, a first gene of interest) that, *e.g.*, encode carotenoid biosynthetic enzymes. Optionally, once this biosynthetic activity has been increased by expression of these introduced carotenoid biosynthesis genes, the pathway can be diverted for the production and accumulation of specific carotenoids. The diversion typically includes the use of at least one second gene of interest. To illustrate, the second gene can encode an enzyme to force the production of a particular carotenoid or alternatively can encode a gene to stop the pathway for the accumulation of a particular carotenoid. To force the production of a particular carotenoid, expression of a carotenoid biosynthesis gene in the pathway for the desired carotenoid is used. Genes native or exogenous to the target plant are optionally used in these methods, including, *e.g.*, carotenoid biosynthesis genes from sources other than plants, such as bacteria, including *Erwinia* and *Rhodobacter* species. Exemplary carotenoid biosynthesis nucleic acid segments or genes that can be utilized for these purposes are described further above. To stop the pathway in order to accumulate a particular carotenoid compound, the second gene will provide for inhibition of transcription of a gene (*e.g.*, native or exogenous) to the target plant in which the enzyme encoded by the inhibited gene is capable of modifying the desired carotenoid compound. Inhibition may be achieved by transcription of the gene to be inhibited in either the sense (cosuppression) or antisense orientation of the gene. Other sense and antisense strategies for modulating carotenoid accumulation in plants are referred to above.

To further illustrate, but not to limit the present invention, to alter, *e.g.*, the carotenoid composition of a plant towards the accumulation of higher levels of β-carotene derived carotenoids, such as zeaxanthin, zeaxanthin diglucoside, canthaxanthin, and astaxanthin, inhibition of lycopene ε-cyclase can be achieved to prevent accumulation of α-carotene and other carotenoids that are derivative from α-carotene, such as lutein. In addition to the inhibition of lycopene ε-cyclase, increased expression of a second gene may be utilized for increased accumulation of a particular β-carotene derived carotenoid. For example, increased β-carotene hydroxylase expression is useful for production of zeaxanthin, whereas increased β-carotene hydroxylase and keto-introducing enzyme expression is useful for production of astaxanthin. Alternatively, to accumulate lycopene, the inhibition of lycopene β-cyclase or of lycopene ε-cyclase and lycopene β-cyclase can be effected to reduce conversion of lycopene to α- and β-carotene.

A variety of genes are optionally used as to divert carotenoid biosynthesis in cells and plants as desired. These include, but are not limited to, β-carotene hydroxylase or crtZ (Hundle et al. (1993) FEBS Lett. 315:329-334, Accession No. M87280) for the production of zeaxanthin; genes encoding keto-introducing enzymes, such ascrtW (Misawa et al. (1995) J. Bacteriol. 177:6575-6584, WO 95/18220, WO 96/06172) or β-C-4-oxygenzse (crtO; Harker et al. (1997) FEBS Lett. 404:129-134) for the production of canthaxanthin; crtZ and crtW or crtO for the production of astaxanthin; ε-cyclase and ε-hydroxylase for the production of lutein; ε-hydroxylase and crtZ for the production of lutein and zeaxanthin; antisense lycopene ε-cyclase (Accession No. U50738) for increased production of β-carotene; antisense lycopene ε-cyclase and lycopene β-cyclase (Hugueney et al. (1995) Plant J. 8:417-424, Cunningham Jr et al. (1996) Plant Cell 8:1613-1626, Scolnik et al. (1995) Plant Physiol. 108:1343, Accession Nos. X86452, L40176, X81787, U50739 and X74599) for the production of lycopene; antisense plant phytoene desaturase for the production of phytoene; and the like.

In this manner, the pathway can be modified for the high production of any particular carotenoid compound of interest. Such compounds include, but are not limited to, α-cryptoxanthin, β-cryptoxanthin, ζ-carotene, phytofluene, neurosporane, etc. Using the methods of the invention, any compound of interest in the carotenoid pathway can be produced at high levels in selected storage organs, such as the fruit of plants.

Optionally, the pathway can also be manipulated to decrease levels of, for example, a particular carotenoid by, *e.g.*, transforming the plant cell with antisense DNA sequences which prevents the conversion of the precursor compound into the particular carotenoid being regulated.

Although some of the description herein relates to altering the accumulation of carotenoids in plants for purposes of clarity of illustration, it will be appreciated that the general strategies described herein can be readily adapted to modulate other plant traits by persons skilled in the art. Accordingly, the present invention is not limited to modulated carotenoid accumulation in plants.

### EXAMPLES

The following examples are offered to illustrate, but not to limit the claimed invention. One of skill will recognize a variety of non-critical parameters that may be altered without departing from the scope of the claimed invention. It is understood that the examples and embodiments described herein are for illustrative purposes only and that various modifications or changes in light thereof will be suggested to persons skilled in the art and are to be included within the spirit and purview of this application and scope of the appended claims.

### EXAMPLE 1

### Isolation of the Ubiquitin Promoter from Pineapple

### ISOLATION OF PINEAPPLE UBIQUITIN PROMOTER (UBP) INTRON SEQUENCE

### Primer design

**Step I.1** In order to isolate the intron sequence of the ubiquitin promoter, two primers were designed based on the published sequence of the pineapple ubiquitin gene (*Ananas comosus* tetrameric ubiquitin mRNA, complete coding sequence; see GenBank Accession No. AY098526).

| **Primer** | **Sequence** | **SEQ ID NO** |
|---|---|---|
| Forward primer UBF-1 | GATCGAGCCTCTTCGAATCCTC | 18 |
| Reverse primer Ubpp-2 | GGGTCTTGACGAAGATTTGC | 19 |

### Genomic DNA isolation

**Step I.2** Genomic DNA was extracted from Del Monte Gold™ MD-2 variety pineapple shoots using DNeasy plant genomic DNA isolation kit from QIAGEN^{®} according to the manufacture's procedure.

### Genomic PCR reaction

Step I.3 Genomic DNA was amplified by PCR using 50 ng of MD-2 genomic DNA and the UBF-1 and Ubpp-2 primers. The PCR reaction was initiated by combining the following components:

| | |
|---|---|
| UBF-1 primer (10 µM stock): | 0.4 µl |
| Ubpp-2 primer (10 µM stock): | 0.4 µl |
| MD-2 genomic DNA: | 1.0 µl |
| 4dNTPs (2.5 mM ea): | 1.6 µl |
| Eppendorf^{®} 10X reaction buffer: | 2.0 µl |
| 5X Master Taq mix | 4.0 µl |
| Eppendorf^{®} Taq DNA polymerase: | 0.2 µl |
| Water to 20 µl. | |

**Step I.4** The PCR thermocycling program was as follows:
Denature 4 minutes at 95°C.
95°C for 30 seconds
55°C for 30 seconds
72°C for 1 min 30 seconds
36 cycles

### Cloning and sequencing of the PCR fragment.

**Step I.5** The PCR amplification product was purified from 0.8% agarose gel electrophoresis, using QIAGEN^{®} gel purification kit (QIAGEN^{®}, Inc., CA, USA). The fragment was then cloned into the pGEM-Teasy vector (Promega™ Corp., WI, USA). Sequencing was performed using universal M13-forward and M13-reverse primers.

### ISOLATION OF UBP PROMOTER SEQUENCE UPSTREAM OF THE UBP PROMOTER INTRON

### Primer design

**Step I.6** Two primers were designed based on the published sequence of the ubiquitin gene from pineapple (*Ananas comosus* tetrameric ubiquitin mRNA, complete coding sequence, GenBank Accession No. AY098526) to isolate the sequence upstream of the intron.

| **Primer** | **Sequence** | **SEQ ID NO** |
|---|---|---|
| Ubp-2 | CTTGAGAGGAGAGGAGGATTCG | **20** |
| Ubp-3 | GAGGATTCGAAGAGGCTCGATC | **21** |

### Isolation of Ubp intron upstream sequence by chromosome walking

**Step I.7** The Del Monte Gold™ MD-2 variety genomic DNA was digested with three restriction enzymes: Kpn I, Sac I and Pst I. The digested genomic DNA was used as a template along with Ubp-2 and Ubp-3 primers for the "chromosome walking" experiment using the TOPO^{®} Walker Kit" (Invitrogen™), according to the manufacture's protocol.

**Step I.8** The PCR program used for the "chromosome walking" experiment was:
Denature for 4 minutes at 95°C.
95°C for 30 seconds
58°C for 30 seconds
72°C for 1 min
36 cycles

### Cloning and sequencing of the PCR amplicon

**Step I.9** The cloning and sequencing of the PCR product was as described in Step 1.5 above. Sequencing of this pineapple ubiquitin promoter PCR product as described in Steps I. 1 through 1.8 yielded a 1352 basepair nucleic acid, as provided in **FIG. 1** and SEQ ID NO: 4.

### ISOLATION OF EXTENDED UBP PROMOTER SEQUENCE

### Primer design

**Step I.10** Three primers for chromosome walking were designed based on the sequence obtained from Step I.9 above. These were:

| **Primer** | **Sequence** | **SEQ ID NO** |
|---|---|---|
| Ubp-4 | GTGCGAAATTGCGTAAGAGGGG | **22** |
| Ubp-5 | GGTATTTATAGGGTCGTGGACCGAG | **23** |
| Ubp-6 | GTGACTCCGCGGTGAGATTAC | **24** |

### Isolation of extended Ubp promoter sequence by chromosome walking

**Step I.11** The MD-2 genomic DNA was digested with Apa I, Kpn I, Sac I and Sph I respectively. The digested genomic DNA was used as a template along with Ubp-4, Ubp-5 and Ubp-6 primers for "chromosome walking" using TOPO^{®} Walker Kit" (Invitrogen™) according to the manufacture's protocol.

### Cloning and sequencing of the PCR fragment.

**Step I.12** The cloning and sequencing of the PCR product was as described in Step 1.5 above. Sequencing of this pineapple ubiquitin promoter PCR product generated in Steps I.10 through I.12 yielded a 1526 basepair nucleic acid, as provided in **FIG. 1** and SEQ ID NO: 5.

### EXAMPLE 2

### Isolation of Epoxide Hydrolase (EHS) Promoter from Pineapple

### ISOLATION OF PINEAPPLE EHS PROMOTER 1.1 KB SEQUENCE

### Primer design

**Step II.1** Based on known EHS cDNA sequence, EHS promoter sequence was obtained by inverse PCR (iPCR), using techniques known in the art. Based on this EHS promoter sequence, two primers containing engineered restriction sites were designed to be used in genomic DNA isolation. These are shown below, where the restriction sites are underlined.

| **Primer** | **Sequence** | **SEQ ID NO** |
|---|---|---|
| Forward primer EHSp5'-SalI | GTTAGTCGACTTCACGCAATGAGACTGCAGCC | **25** |
| Reverse primer EHSp3'-1NcoI | GTTACCATGGGTATCTATAGATCCTTGATCTC | **26** |

### Genomic DNA isolation

**Step II.2** Genomic DNA was isolated as described in Step 1.2.

### Genomic PCR reaction

**Step II.3** PCR was performed using 50 ng of MD-2 genomic DNA and the EHSp5'-SalI and EHSp3'-1NcoI primers in the reaction condition as described in Step 1.3

**Step II.4** The PCR program was executed as described in Step 1.4.

### Cloning and sequencing of the PCR fragment.

**Step II.5** The PCR reaction product was cloned and sequenced as described in Step I.5. Sequencing of this pineapple EHS promoter PCR product generated a 1172 basepair nucleic acid (termed EHS 1.1), as provided in **FIG.1** and SEQ ID NO: 2.

### ISOLATION OF EHS PROMOTER 1.7 KB SEQUENCE USING INVERSE PCR METHOD

### Primer Design

**Step II.7** For the purpose of obtaining further promoter sequence, six primers were designed based on the EHS1.1 promoter sequence of SEQ ID NO: 2, obtained in Step II.5. These primers were:

| **Primer** | **Sequence** | **SEQ ID NO** |
|---|---|---|
| Forward primer EHSp.ext1 | CTGCTTGTCTGATCATGATCTCC | **27** |
| Forward primer EHSp.ext2 | CCCAGCATTTATGGACTACGAC | **28** |
| Forward primer EHSp.ext3 | GGAGGTTAGATCTGCTTGTTCCC | **29** |
| Reverse primer IPCR1 | GGATCGCCGTGCACGTCGCCG | **30** |
| Reverse primer IPCR2 | GCTCCTGCATGGCTTTCCGGAGC | **31** |
| Reverse primer IPCR3 | CTGGTACTCGTGGCGGCACCAG | **32** |

### Genomic DNA isolation, digestion and ligation

**Step II.8** Genomic DNA was extracted as described as in Step 1.2. DNA was digested with KpnI and then religated in a standard ligation reaction as follows:

| | |
|---|---|
| KpnI digested genomic DNA | 1 µg |
| 10X ligase buffer (New England BioLabs^{®}, Inc.) | 10 µl |
| T4 DNA Ligase (New England BioLabs^{®}, Inc) | 10 µl |
| Add water to 100 µl. | |
| Incubate at room temperature for 3 hours. | |

### PCR reaction

**Step II.9** PCR was performed using 50-100 ng of KpnI/religated genomic DNA and the three forward and three reverse primers in the reaction conditions as described in Step 1.3 and using the PCR program described in Step 1.4.

### Cloning and sequencing of the PCR fragment.

**Step II.10** The PCR reaction product was cloned and sequenced as described in Step I.5. This sequencing revealed 1693 basepairs of promoter sequence, termed EHS1.7. This sequence is provided in **FIG. 1** and SEQ ID NO: 3.

### EXAMPLE 3

### Isolation of Ubiquitin Terminator Sequence from Pineapple

### ISOLATION OF UBIQUITIN TERMINATOR SEQUENCE BY CHROMOSOME WALKING

Primer Design - Three primers were designed based on the published sequence of the ubiquitin gene from pineapple (*Ananas comosus* tetrameric ubiquitin mRNA, complete coding sequence, GenBank Accession No. AY098526). These primers were:

| **Primer** | **Sequence** | **SEQ ID NO** |
|---|---|---|
| Ubp3'-1 | GTCAGTTTGTGTCTGGTGTGTTTGAG | **33** |
| Ubp3'-2 | CTGCAGGTTCTATGATGACGGAC | **34** |
| Ubp3'-3 | GATGACGGACTTCGCAGTTTTAAC | **35** |

Genomic DNA Isolation - Genomic DNA was extracted from MD-2 pineapple shoots as described as in Step I. 2.

Chromosome Walking - Preparation of MD-2 genomic DNA template and chromosome walking experiment was performed as described in Step I.7. The PCR for the chromosome walking is performed as described in Step I.8. A nucleic acid of approximatley 0.9 kb was subcloned and sequenced as described in Step I.5.

This sequencing revealed 940 basepairs of sequence preceeding the "TAA" stop codon of the pineapple ubiquitin gene coding sequence. This sequence was termed the Ubiquitin terminator sequence, and is provided in **FIG. 1** and in SEQ ID NO: 10.

### EXAMPLE 4

### Isolation of carotenoid isomerase cDNA (ISO) from Pineapple

### ISOLATION OF ISO cDNA FROM PINEAPPLE FRUIT

Primer Design - Two primers were designed for cDNA amplification based on the sequence of the conserved regions of ISO genes in different plant species *(Lycopersicon esculentum,* GenBank Accession No. AF416727; *Arabidopsis thaliana,* GenBank Accession No. NM100559; and *Citrus limon,* GenBank Accession No. AB114667). See also, Isaacson et al., "Cloning of tangerine from tomato reveals a carotenoid isomerase essential for the production of beta-carotene and xanthophylls in plants," Plant Cell 14(2):333-342 (2002) and Eckardt "Tangerine Dreams: Cloning of Carotenoid Isomerase from Arabidopsis and Tomato," The Plant Cell 14(2):289-292 (2002), both of which are incorporated by reference in their entirety.

These primers for cDNA amplification were:

| **Primer** | **Sequence** | **SEQ ID NO** |
|---|---|---|
| Forward primer ISOF2 | ACAGATTGCCACCATTTTGTGCTTGAGGA | **36** |
| Reverse primer ISOR3 | GGCATTGGCCCATAGGTACC | **37** |

RNA Isolation - Total RNA was extracted from pineapple fruit (5 days before harvest) or shoots with TRI-Reagent^{®} solution (SIGMA^{®}, St. Louis, MO, USA). The extraction was performed according to the manufacture's instructions.

Reverse Transcription Reaction - Reverse transcription was carried out using Invitrogen™ SuperScript™ III according to the manufacturer's instructions. The reaction contained 0.4 µg of total RNA and 1 µl of 10 µM oligo (dT)₂₆ primer in 20 µl of final reaction volume.

PCR Reaction - PCR was performed using the first strand cDNA synthesized in the reverse transcription reaction described in the previous step as a template and ISOF2 and ISOR3 primers in a PCR reaction using conditions described in Step I.3. The PCR program was performed as described in Step 1.4.

Cloning and Sequencing - The PCR fragment was subcloned and sequenced as described in Step 1.5.

### ISOLATION OF ISO 3' AND 3' UTR cDNA USING 3'-RACE

Primer Design - Two primers suitable for 3' RACE (Rapid Amplification of cDNA Ends) were designed based on the sequence obtained in the previous step. These primers were:

| **Primer** | **Sequence** | **SEQ ID NO** |
|---|---|---|
| ISO5'-1 | CGCCTAGAAGAGCCCTATGGAAG | **38** |
| ISO5'-2 | CATCATTGGCTCCGGAAGGACAC | **39** |

First and Second PCR Reactions - The first strand cDNA synthesized in in the reverse transcription reaction described above was used as a template and a set of ISO5'-1 and oligo (dT)26 primers was used for the first PCR reaction. A second PCR reaction was performed using ISO5'-2 and oligo (dT)₂₆ as primers, and the first PCR product as a template. The PCR reactions were performed as in Step I.3. The PCR program was performed as described in Step I.4. The 3'-RACE PCR product was then subcloned and sequenced as described in I.5.

### ISOLATION OF ISO 5' cDNA USING 5'-RACE

Primer Design - Three primers were designed based on the sequence obtained in the previous ampification step. These primer were:

| **Primer** | **Sequence** | **SEQ ID NO** |
|---|---|---|
| ISO3'-2 | CCTTTCTAGGTAGGCCCTCCC | **40** |
| ISO3'-3 | GATGAATCAAGAACAGTTGGG | **41** |
| ISO3'-4 | GGGCTCTTCTAGGCGGGCCC | **42** |

Reverse Transcription Reaction - Reverse transcription was carried out using the ISO3'-2 primer and Invitrogen™ SuperScript™ III according to the manufacturer's instructions. The reaction contained 0.4 µg of total RNA and 1 µl of 10 µM oligo (dT)₂₆ primer in 20 µl of final reaction volume.

A-Tailing Reaction - The cDNA obtained from the previous step was subjected to an A-tailing reaction by using terminal transferase (TdT, New England BioLabs) and dATP in the following reaction mix as per the manufacture's instructions.

| | |
|---|---|
| cDNA template | 5 µl |
| 10X Buffer (New England BioLabs^{®}, Inc.) | 2 µl |
| 2.5 mM CoCl₂ (New England BioLabs^{®}, Inc.) | 2 µl |
| 0.1 mM dATP | 1 µl |
| terminal transferase (TdT; New England BioLabs^{®}, Inc) | 1 µl |
| Add water to 20 µl. | |

First and Second PCR Reactions - The A-tailed cDNA obtained in the previous step was used as a template and a set of ISO3'-3 and oligo (dT)₂₆ primers was used for the first PCR reaction. A second PCR reaction was performed using ISO3'-4 and oligo (dT)26 as primers, and the first PCR product as a template. The PCR reactions were carried out as described in Step I.3, and the PCR program was performed as in Step I.4. The 5'-RACE PCR product was subcloned and sequenced as in Step I.5.

### ISOLATION OF COMPLETE ISO 5' cDNA USING GeneRacer™

Primer Design - Three primers were designed based on the sequence obtained from the previous step. These primers were:

| **Primer** | **Sequence** | **SEQ ID NO** |
|---|---|---|
| ISO5'RACE-3 | CAGTCAACTGCTTGCAATGCTCTTG | **43** |
| ISO5'RACE-4 | TGTCGCCAAAGCCAAACATG | **44** |
| ISO5'RACE-5 | GAAGCCAGAGCTCCCGCC | **45** |

RNA Preparation and Reverse Transcription Reaction - Oligo(dT) was ligated to the total RNA isolated from pineapple fruit or leaves according to the manufacture's instructions (GeneRacer™ Kit, Invitrogen™). Reverse transcription was carried under conditiosn described above using the ISO5'-RACE-3 primer.

First and Second PCR Reactions - The cDNA obtained in the previous step was used as a template and a set of ISO5'-RACE-4 and 5' primer (from GeneRacer™) was used for the first PCR reaction. A second PCR reaction was performed using ISO5'RACE-5 and a 5' nested primer (from GeneRacer™) as primers, and using the firstPCR product as a template. The PCR program was performed as described in Step I.4. The 5'RACE PCR product was purified, subcloned and sequenced as in Step I.5.

This sequencing identified a 2372 basepair nucleic acid encoding a putative pineapple carotenoid isomerase. This nucleotide sequence is provided in **FIG. 1** and SEQ ID NO: 1. The amino acid translation of the open reading frame contained in this nucleic acid is provided in **FIG.1** and SEQ ID NO: 9.

### EXAMPLE 5

### Isolation of Phytoene Synthase (Psy) cDNA from Pineapple

### ISOLATION OF PSY cDNA FROM PINEAPPLE

Primer Design - Primers were designed based on the sequence of conserved regions of phytoene synthase (Psy) genes from different plant spieces, including *Lycopersicon esculentum,* GenBank Accession No. Y00521 and *Citrus unshiu*, GenBank Accession No. AF220218. These primers were:

| **Primer** | **Sequence** | **SEQ ID NO** |
|---|---|---|
| Forward primer Psy5'-1 | AGTGAAGCTTATGATCGTTGTGGAGAAGT | **46** |
| Forward primer Psy5'-2 | GAGTATGCTAAGACATTTTACTTGGGAACT | **47** |
| Forward primer Psy5'-3 | GAAGGGCTATATGGGCTATATATGT | **48** |
| Reverse primer Psy3'-2 | CTCACATAAGCTCTCTTTGTGAAGTTGTTGTA | **49** |
| Reverse primer Psy3'-1 | GATTTTGCATATGCAATTGGTAGTGCA | **50** |

The initial isolation of the Psy cDNA used the identical strategy and protocols for RNA isolation, reverse transcription, PCR amplification, clonign and sequencing as described in Example 4 for the cloning of the ISO cDNA.

### ISOLATION OF PINEAPPLE PSY 3' AND 3' UTR cDNA USING 3'RACE

Three primers were designed based on the Psy cDNA sequence obtained above. These primers were:

| **Primer** | **Sequence** | **SEQ ID NO** |
|---|---|---|
| PPsy3'RACE-1 | GGTAAATGGCGGAATTTCATG | **51** |
| PPsy3'RACE-2 | GAGCCAGGATGTTCTTCCAAG | **52** |
| PPsy3'RACE-3 | AGCTCAGCCAAGCTAGTCGG | **53** |

The generation of 3' RACE products for the Psy gene used the identical 3' RACE strategy and protocols as described in Example 4 for the cloning of the ISO 3' sequences.

### ISOLATION OF PSY 5' cDNA USING 5'RACE METHOD

Three primers were designed based on the Psy cDNA sequence obtained above. These primers were:

| **Primer** | **Sequence** | **SEQ ID NO** |
|---|---|---|
| PPsy 5'RACE-1 | GCGTCAAGCATATCATACGG | **54** |
| PPsy 5'RACE-2 | GCGAACAAATCCTCCAACC | **55** |
| PPsy 5'RACE-3 | CCATCGATCCAACGCTGACG | **56** |

The generation of 5' RACE products for the Psy gene used the identical 5' RACE strategy and protocols as described in Example 4 for the cloning of the ISO 5' sequences.

### ISOLATION OF COMPLETE PSY 5' cDNA USING GeneRacer™

The generation of a complete Psy gene used the identical strategy and protocols for reverse transcription, PCR reactions and cloning/sequencing as described in Example 4 for the ISO gene sequences.

The sequencing of this product identified a 1781 base pair nucleic acid encoding a putative pineapple phytoene synthase. This nucleotide sequence is provided in FIG. 1 and SEQ ID NO: 11. The amino acid translation of the open reading frame contained in this nucleic acid is provided in **FIG. 1** and SEQ ID NO: 13.

### EXAMPLE 6

### Isolation of lycopene β -cyclase cDNA from Pineapple

### ISOLATION OF LYCOPENE β-CYCLASE (LYC) cDNA FROM PINEAPPLE

Primer Design - Primers were designed based on published sequences of lycopene cyclase genes from different species. These primer were:

| **Primer** | **Sequence** | **SEQ ID NO** |
|---|---|---|
| CycD-F3** | ACKGGMTTTCKMGVTSYCTYG | **57** |
| Pcyc-R3 | TCCATGCCGAAGCAGAAGAACTC | **58** |
| ** where: M = A or C; K = G or T; V = A or C or G; S = C or G; and Y = C or T | | |

The initial isolation of the LYC cDNA used the identical strategy and protocols for RNA isolation, reverse transcription, PCR amplification, cloning and sequencing as described in Example 4 for the cloning of the ISO cDNA.

### ISOLATION OF PINEAPPLE LYC 3' AND 3' UTR cDNA FROM A PINEAPPLE FRUIT cDNA LIBRARY

Primer Design - Two primers were designed based on the cDNA sequence obtained from the previous step. These primers were:

| **Primer** | **Sequence** | **SEQ ID NO** |
|---|---|---|
| Lyc3'RACE-1 | CTTACCGGTAACGAGCTTTCGG | **59** |
| Lyc3'RACE-2 | GGAAGGATCTTTGGCCGATTG | **60** |

First and Second PCR Reactions - Phage suspension isolated from a pineapple fruit cDNA library was used as a template for the first PCR reaction using primer SEQ ID NO59 and universal forward primer. The second PCR reaction was performed using the first PCR product as a template, and the Lyc3'RACE-2 (SEQ ID NO: 60) and the T7 primers. The PCR program was performed as described in Step I.4. The PCR product cloning and sequencing was done as described in Step I.5.

### ISOLATION OF LCY 5' cDNA USING 5'-RACE METHOD

Primer Design - Two primers were designed based on the sequence obtained from the previous step. These primers were:

| **Primer** | **Sequence** | **SEQ ID NO** |
|---|---|---|
| Lyc5'RACE-1 | ATCTTATCCAGATCGAACGGGTGC | **61** |
| Lyc5'RACE-2 | GTACGCGACCTGGTATCCGGGAT | **62** |

First and Second PCR Reactions - Phage suspension isolated from a pineapple fruit cDNA library was used as a template for the first PCR reaction using primer Lyc5'RACE-1 (SEQ ID NO: 61) and universal reverse primer. A second PCR reaction was performed using the first PCR product as a template, and primer Lyc5'RACE-2 (SEQ ID NO: 62) and T3 primer. The PCR program was performed as described in Step I.4, and the PCR product was cloned and sequenced as described in Step I.5.

The sequencing of this product identified a 1653 base pair nucleic acid encoding a putative pineapple lycopene β-cyclase. This nucleotide sequence is provided in **FIG. 1** and SEQ ID NO: 14. The amino acid translation of the open reading frame contained in this nucleic acid is provided in **FIG.1** and SEQ ID NO: 15.

### EXAMPLE 7

### Isolation of Bromelain Inhibitor Gene Promoter Sequence from Pineapple

### ISOLATION OF BROMELAIN INHIBITOR GENE PROMOTER SEQUENCE USING INVERSE PCR METHOD

Primer Design - Six primers were designed based on a published bromelain cDNA EST sequence (Pineapple green mature fruit cDNA library *Ananas comosus* cDNA clone JBW005D04; GenBank Accession No. CO731100). These primers were:

| **Primer** | **Sequence** | **SEQ ID NO** |
|---|---|---|
| BRI5'-1 | ACTCGAGCGAGAAAATCAAGAC | **63** |
| BRI5'-2 | GTGCGAGCCGAGGACGGAC | **64** |
| BRI5'-3 | GACACTGCAACAAGAAGTAC | **65** |
| BRIiPCR1 | CTCATAAGAGAATAGTGGTTG | **66** |
| BRIiPCR2 | TGATATTATTTGATGTTTGCG | **67** |
| BRIiPCR3 | GTGTTGCTGCTCTTTCTGCATG | **68** |

Genomic DNA was extracted as described in Example 1. The genomic DNA was digested with SphI and religated as described in Example 2. Promoter sequence was isolated as described in Example 2.

The sequencing of the bromelain inhibitor gene promoter region is provided in **FIG.1** and SEQ ID NO: 16.

### EXAMPLE 8

### Isolation of Pineapple Meristem ACC synthase 3' Sequence

### ISOLATION OF PINEAPPLE MERISTEM ACC SYNTHASE 3' SEQUENCE USING TAIL PCR METHOD

Primer Design - Three primers were designed based on published sequence data (see, e.g., United States Patent No. 6,194,639, entitiled "ACC synthase genes from pineapple," to Botella et al., filed May 1, 1997). These primers were:

| **Primer** | **Sequence** | **SEQ ID NO** |
|---|---|---|
| ACC3'-1 | GCCAACATTACTGACTGAAAG | **69** |
| ACC3'-2 | CACAGGATCTTCTCTTCCGGC | **70** |
| ACC3'-3 | CTTCTGCTGGATGGACCTAC | **71** |

TAIL PCR reaction - 50 ng genomic DNA from MD-2 was used as a template and standard TAIL PCR reaction was performed. The TAIL PCR product was cloned and sequenced as described in Step I.5.

The sequence of the 1167 basepair pineapple meristem ACC synthase gene 3' region amplicon is provided in **FIG. 1** and SEQ ID NO: 17.

### EXAMPLE 9

### Expression Vector Constructions

### UBP1.5-GUS-NOS

The Ubp1.5 fragment (SEQ ID NO: 5) was cloned as a SalI-NcoI fragment upstream of GUS-NOS gene cassette in pCambia-1300 (CAMBIA; Center for the Application of Molecular Biology to International Agriculture, Camberra, Australia). The GUS sequence encodes the β-glucuronidase enzymatic reporter, and NOS refers to terminator sequence derived from the *Agrobacterium* nopaline synthase gene, as known in the art.

**Step IX.1** Primer Design - Two primers were designed to amplify the Ubp1.5 fragment by introducing the SalI site at the 5' end and the NcoI site at the 3' end of the DNA. These were:

| **Primer** | **Sequence** | **SEQ ID NO** |
|---|---|---|
| Forward primer Ubp1.5.5'Sal | GAGACCATGGGTTTTTGAAGTTTTCCCTTTTTTTTACTTTAG | **73** |
| Reverse primer Ubp3Ncol | GACTCCATGGCTGAAACAGATTGAGAAACACAGAAT | **74** |

**Step IX.2** PCR Amplification - The primers above were used in a PCR amplification reaction using standard PCR reagents and reaction conditions.

**Step IX.3** Cloning - The amplified Ubp1.5 fragment was digested with SalI and NcoI restriction enzymes and then ligated into pCambia-1301 digested with the same enzymes, in the following reaction mix:

| | |
|---|---|
| Ubp1.5 | ∼100 ng |
| PCambia1301 | ∼100 ng |
| 10X ligase buffer (New England BioLabs^{®}, Inc.) | 1 µl |
| T4 DNA ligase (New England BioLabs^{®}, Inc.) | 1 µl |
| Add water to 10 µl. | |

The reaction was incubated at room temperature for 2-3 hours. One µl of the resulting ligation product was used to transform *E. coli* DH5a strain by electroporation. The transformants were selected by plating onto LB agar plates containing kanamycin at 50 µg/ml.

### EHS 1.1-GUS-NOS

The EHS1.1-GUS-NOS vector was generated by cloning the EHS1.1 sequence (SEQ ID NO: 2) as a SalI-NcoI fragment upstream of the GUS-NOS gene cassette in pCambia-1300.

Two primers were designed to amplify the EHS 1.1 fragment by introducing a SalI site at the 5' end and an NcoI site at the 3' end of the DNA. These primers were:

| **Primer** | **Sequence** | **SEQ ID NO** |
|---|---|---|
| Forward primer EHSp5'-SalI | GTTAGTCGACTTCACGCAATGAGACTGCAGCC | **75** |
| Reverse primer EHSp3.1-NcoI | GTTACCATGGGTATCTATAGATCCTTGATCTC | **76** |

Following the PCR reaction, the EHS1.1 amplicon was cloned into pCambia-1300 as described above in Step IX.3.

### EHS1.7-GUS-NOS

The EHS1.7- GUS-NOS vector was generated by cloning an EHS1.7 amplicon (SEQ ID NO: 3) having SalI-NcoI ends upstream of the GUS-NOS cassette in pCambia 1300.

Two primers were design to amplify the EHS 1.7 fragment by introducing the SalI site at the 5' end and the NcoI site at the 3' end of the DNA. These primers were:

| **Primer** | **Sequence** | **SEQ ID NO** |
|---|---|---|
| Forward primer EHSextp5-Sal2 | GTTAGTCGACCTTTCCTTCCTGGCCGTCTC | **77** |
| Reverse primer EHSp3.1-NcoI | GTTACCATGGGTATCTATAGATCCTTGATCTC | **78** |

Following the PCR reaction, the EHS 1.7 PCR amplicon was cloned into pCambia-1300 as described above in Step IX.3.

### EHS 1.7UBP1.5-GUS-NOS

The EHS1.7Ubp1.5- GUS-NOS fusion-promoter vector was constructed by cloning a Ubp1.5 amplicon (SEQ ID NO: 5) as an NcoI-NcoI fragment between the EHS1.7 and GUS sequences in construct EHS 1.7- GUS-NOS (see above).

Two primers were designed to amplify the Upb1.5 sequence by introducing an NcoI site at the 5' end and an NcoI site at the 3' end of the DNA. These primer were:

| **Primer** | **Sequence** | **SEQ ID NO** |
|---|---|---|
| Forward primer Ubp1.5.5'Nco | GAGACCATGGGTTTTTGAAGTTTTCCCTTTTTTTTACTTTAG | **79** |
| Reverse primer EHSp3.1-NcoI | GTTACCATGGGTATCTATAGATCCTTGATCTC | **80** |

The amplified Ubp1.5 NcoI-NcoI fragment was cloned into construct EHS 1.7-GUS-NOS (see above) between the EHS 1.7 and GUS sequences as described above in Step IX.3.

### EHS 1.7Ubp1.5-ALS-ALS3'

The EHS1.7Ubp1.5-ALS-ALS3' vector was generated by cloning EHS1.7Ubp1.5 (SEQ ID NO: 6) upstream of the gene encoding acetolactase synthase (ALS, also known as *SurB*), which can serve as a selectable marker that confers resistance to chlorsulfuron (see, Firoozabady et al., "Transformation and regeneration of pineapple," Plant Cell, Tissue and Organ Culture 84(1):1-16 [2005]). Expression of the protein is also stabilized by the addition of ALS gene terminator sequence, termed ALS3'. The ALS-ALS3' sequences were obtained from a vector containing the genomic ALS gene sequence with adjacent 3' sequence. The ALS gene sequences are known in the art, see, for example, US Patent Serial No. 5,378,824 to Bedbrook et al.

The EHS1.7Ubp1.5 fragment amplification introduced a KpnI site at the 5' end and a SmaI site at the 3' end of the DNA fragment. The primers used in the amplification were:

| **Primer** | **Sequence** | **SEQ ID NO** |
|---|---|---|
| Forward primer EHSUbp5'Kpn | GAGAGGTACCCTTTCCTTCCTGGCCGTCTCC | **81** |
| Reverse primer EHS3'Sma | GAGACCCGGGCTGAAACAGATTGAGAAACACAGAATGAG | **82** |

The resulting PCR amplicon was cloned into pALS 12 upstream of the ALS-ALS3' gene cassette. The ligation reaction and cloning conditions were as described above in Step IX.3.

### EHS1.1-LYC SENSE-LS 1 INTRON-LYC ANTISENSE-BADH TERMINATOR

The EHS 1.1-LYC sense-Ls1 intron-LYC antisense-BADH terminator vector is an expression construct for use in gene suppression RNAi nucleic acid expression. This RNAi vector has the following components, in linear order, (i) the EHS1.1 promoter (SEQ ID NO: 2), (ii) approximately 615 base pairs (SEQ ID NO: 93) of lycopene β-cyclase (LYC) gene sequence derived from the LYC cDNA of SEQ ID NO: 14 in the sense orientation; (iii) the Ls1 gene intron (189 base pairs) from potato (Eckes et al., "Isolation and characterization of a light-inducible, organ-specific gene from potato and analysis of its expression after tagging and transfer into tobacco and potato shoots," Mol. Gen. Genet., 205:14-22 [1986]) that forms a spliceable intron loop; (iv) approximately 615 base pairs (SEQ ID NO: 93) of lycopene β-cyclase (LYC) gene sequence derived from the LYC cDNA of SEQ ID NO: 14 in the antisense orientation;; and (v) BADH terminator sequence.

The construct was built through five steps, as described below.

### Step 1 - Cloning of the Ls1 intron:

The Ls1 intron sequence (see Eckes et al., "Isolation and characterization of a light-inducible, organ-specific gene from potato and analysis of its expression after tagging and transfer into tobacco and potato shoots," Mol. Gen. Genet., 205:14-22 (1986), and GenBank Accession No. X04753) was amplified from pCambia-1301 template and a set of primers that added Pst1-BamHI sites at the 5' end and XhoI-SmaI sites at the 3'-end.

Primers were designed based on a known Ls 1 gene sequence (potato light-inducible tissue-specific ST-LS1 gene; GenBank Accession No. X04753). These primers were:

| **Primer** | **Sequence** | **SEQ ID NO** |
|---|---|---|
| Forward primer Ls1F | GGATCCAAGCTTGTACGTTTGTTTCTGCTTCTAC | **83** |
| Reverse primer Ls1R | GGATCCCTGCAGGATACCTAAACATCACCATG | **84** |

The resulting Ls1 intron PCR amplicon was cloned into the pGEM-Teasy vector (Promega™ Corp., WI, USA) as described in Step IX.3.

### Step 2. Cloning of LYC-sense sequence upstream of the Ls1 intron

(A) A fragment of the pineapple lycopene β-cyclase, gene was isolated as follows. Total RNA was isolated from commercial Del Monte Gold™ MD-2 variety pineapple fruit tissue (shell and fruit tissue) using TRI-Reagent^{®} solution (SIGMA^{®}, St. Louis, MO, USA) based on the manufacture's protocol. Degenerate primers were designed based on published lycopene cyclase gene sequences (*Zea mays* lycopene β-cyclase, Gen Bank Accession No. AY206862; *Citrus unshiu* lycopene cyclase, GenBank Accession No. AY166796; tomato lycopene β-cyclase, GenBank Accession No. X86452; pepper lycopene cyclase, GenBank Accession No. X86221). The primers used were:

| **Primer** | **Sequence** | **SEQ ID NO** |
|---|---|---|
| Forward primer CycD-F3 ** | ACKGGMTTTCKMGVTSYCTYG | **57** |
| Reverse primer Pcyc-R3 | TCCATGCCGAAGCAGAAGAACTC | **58** |
| ** where: M = A or C; K = G or T; V = A or C or G; S = C or G; and Y = C or T | | |

The pineapple lycopene β-cyclase gene was amplified from pineapple fruit total RNA by RT-PCR (Invitrogen™ reverse transcriptase) following the manufacture's protocol. The resulting PCR product was cloned into pGEM-Teasy vector (Promega™ Corp., WI, USA). The sequence of this amplified pineapple lycopene cyclase (LYC) partial cDNA sequence is shown in **FIG. 1** and SEQ ID NO: 93.

(B) Primers were designed to add PstI-KpnI sites at the 5' end and a BamHI site at the 3' end of the Lyc PCR amplicon. These primers were:

| **Primer** | **Sequence** | **SEQ ID NO** |
|---|---|---|
| Forward primer senseLyc5' | GAATCTGCAGGGTACCGATTACGGGCTTCTCTCGGTGC | **85** |
| Reverse primer senseLyc3' | GAATGGATCCGATTTCCATGCCGAAGCAGAAG | **86** |

The Lyc-sense fragment was amplified from the plasmid produced in Step 1 above using primers senseLyc5' and senseLyc3'. The resulting PCR amplicon was cloned into the construct described in Step 2(A) upstream of the Ls1 intron.

### Step 3. Cloning of LYC-antisense sequence downstream of the Ls1 intron

Primers were designed to add 5' SmaI-EcoRV sites at the 5' end and a XhoI site at the 3' end of the LYC amplicon. These primers were:

| **Primer** | **Sequence** | **SEQ ID NO** |
|---|---|---|
| Forward primer antiLyc5' | GAATCCCGGGGATATCGATTACGGGCTTCTCTCGGTGC | **87** |
| Reverse primer antiLyc3' | GAATCTCGAGGATTTCCATGCCGAAGCAGAAG | **88** |

LYC-antisense fragment was amplified from the plasmid produced in Step 2 using these primers, and the resulting amplicon was cloned downstream of the Ls1 intron in construct Step 2.

### Step 4. Cloning of the BADH terminator

(i) The sequence of the BADH cDNA was obtained. Using the cDNA as a template, primers were designed to amplify the BADH terminator adding an EcoRV site at the 5' end and a Smal site at the 3' end of the fragment. These primers were:

| **Primer** | **Sequence** | **SEQ ID NO** |
|---|---|---|
| Forward primer BADHt5' | GAATGATATCTTTTGTCCACGAAGCAGTTTTAATAAATGTGC | **89** |
| Reverse primer BADHt3' | GAATCCCGGGATAAGAACAAGAACAAGAGGGATGGGC | **90** |

(ii) The resultant fragment following amplification was cloned downstream of the Lyc-antisense sequence in the construct produced from Step 3.

### Step 5. Cloning of the EHS 1.1 promoter

(i) Primers were designed to introduce a PstI site at the 5' end and the KpnI site at the 3' end of the EHS1.1 fragment. These primers were:

| **Primer** | **Sequence** | **SEQ ID NO** |
|---|---|---|
| Forward primer EHSp.5'PstI | GAATCTGCAGCCTACAGGGAACAAGC | **91** |
| Reverse primer EHSp.3'KpnI | GAATGGTACCGTATCTATAGATCCTTGATCTCTTTGATCTCTC | **92** |

(ii) The resulting EHS 1.1 PCR amplicon was cloned upstream of the Lyc-sense sequence in the construct produced in Step 4.

### Step 6. Cloning of EHS1.1-Lyc sense-Ls1 intron-Lys antisense-BADH terminator cassette

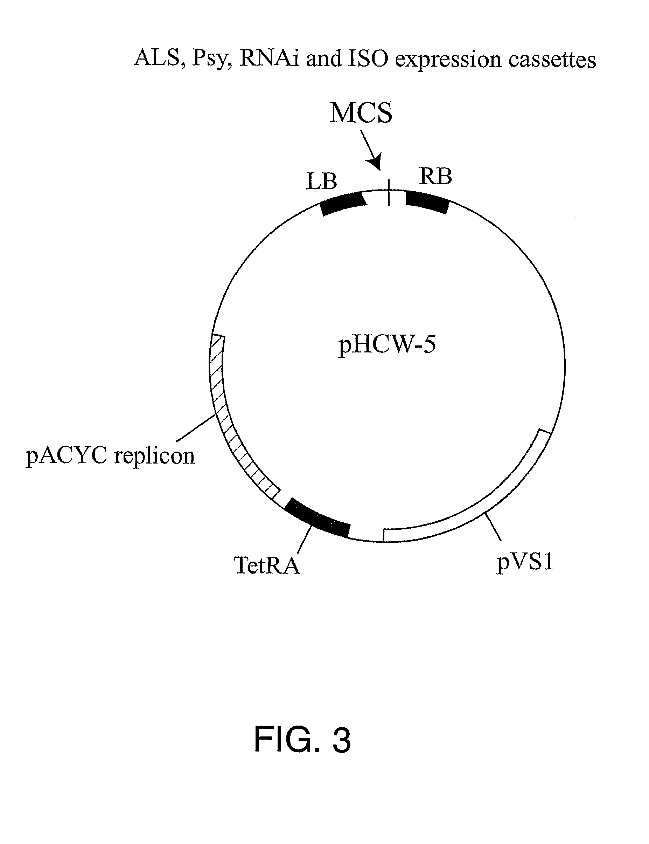

The complete EHS1.1-LYC sense-Ls1 intron-LYC antisense-BADH terminator cassette was isolated from the pGEM-Teasy (Promega™ Corp., WI, USA) vector backbone and cloned into pHCW-1 binary vector. The vector pHCW-1 is similar to the pHCW-5 vector shown in **FIG. 3****,** except without an MCS.

### EXAMPLE 10

### Promoter Activity Analysis Using Agrobacterium Gene Transfer

The activity of various expression vectors of the invention in pineapple and tobacco was assessed by a GUS activity assay following nucleic acid delivery by *Agrobacterium* transfer. Pineapple and tobacco model systems were studied.

### GUS ACTIVITY ASSAYS IN PINEAPPLE TISSUES USING AGROBACTERIUM GENE TRANSFER

Establishment of pineapple shoot cultures, preparation of source tissues for inoculation with *Agrobacterium tumefaciens,* pretreatment of explants for cocultivation with *Agrobacterium, Agrobacterium* culture and preparation, cocultivation on cocultivation medium and recovery were all done essentially as described in International Patent Application Publication No. WO 2004/053082 (hereby incorporated by reference in its entirety), except where noted below.

### Establishment of Shoot Cultures

The establishment of shoot cultures was as described in International Patent Application Publication No. WO 2004/053082 (hereby incorporated by reference in its entirety), except where slips and crowns of Del Monte Gold™ MD-2 variety pineapples grown in the field in Hawaii were used as explants. The core or the stem of the slip or crown (approximately 3cm x 3cm) was washed in water and mild detergent for one hour, then the core was incubated for 30 to 60 minutes in 100 ppm chlorin dioxide (ClO₂) solution with shaking.

### Pretreatment of Explants for Cocultivation with Agrobacterium

The pretreatment of explants for cocultivation with *Agrobacterium* was as described in International Patent Application Publication No. WO 2004/053082 (hereby incorporated by reference in its entirety), except where longitudinal sections were pretreated (cultured) on B3N.2 medium for 8-14 days, then leaf bases or stem sections (5-6 mm) were prepared, mixed with *Agrobacterium* for cocultivation and the mixture wounded (optionally vacuum infilterated). In some cases root sections (5-8 mm) were prepared from shoots that had been rooted in culture in 1/2MSNI medium.

### Agrobacterium tumefaciens Culture and Preparation

The culture and preparation of *Agrobacterium tumefaciens* was as described in International Patent Application Publication No. WO 2004/053082 (hereby incorporated by reference in its entirety), except that *Agrobacterium tumefaciens* strain GV3101 containing a vector reporting system was used for transformation. In some sapects, the vector system includes the *gus* reporter gene encoding β-glucuronidase (GUS).

### Cocultivation on Cocultivation Medium

The cocultivation of *Agrobacterium tumefaciens* was as described in International Patent Application Publication No. WO 2004/053082 (hereby incorporated by reference in its entirety), except that the cocultivation medium was ½ MS T2.2IBA0.1 + 500As +/- 1PVP (see media compositions described below).

### Recovery and GUS Staining

After cocultivation, tissues were transferred (approximately 20 pieces/plate) to recovery medium T2.2IBA0.1C500PVP1 for a recovery period of 12-18 days under low light condition (16 hrs/day). When the majority of explants exhibited morphogenic response (swelling of leaf base, formation of nodular tissues at leaf base) they were assayed for GUS expression using known protocols (see, Jefferson et al., EMBO J., 6:3901-3907 (1987); hereby incorporated by reference in its entirety).

### Results

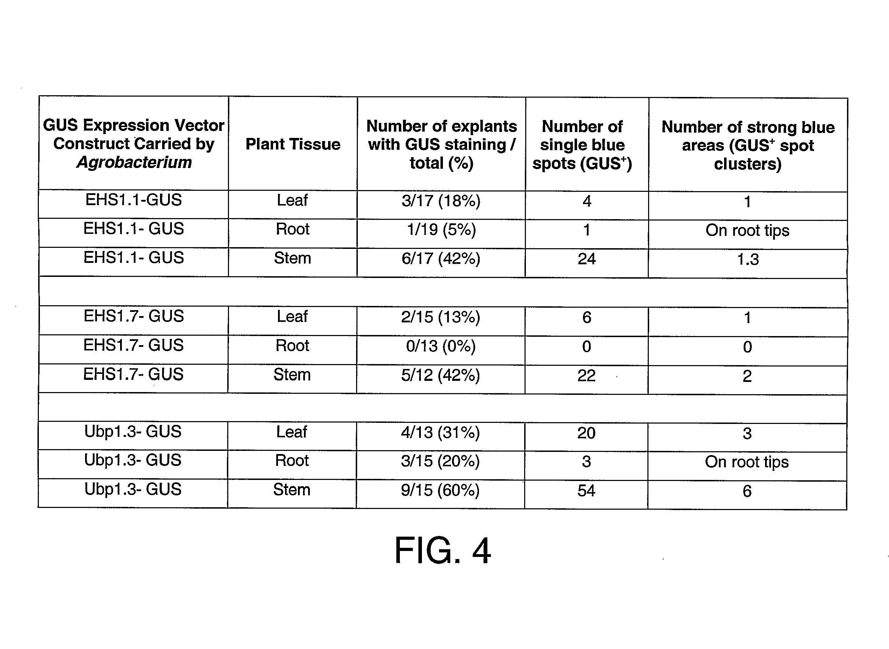

Results of the GUS expression assay using different expression vectors with promoter sequences of the invention are provided in **FIGS. 4****,** **5** and **6****.** In **FIG. 4****,** the activity of various promoter sequences in different pineapple tissues is examined. Three different promoters driving GUS expression were tested. These expression reporter constructs included the EHS1.1 (SEQ ID NO: 2), the EHS1.7 (SEQ ID NO: 3) and the Ubp1.3 (SEQ ID NO: 4) promoters. Each of these reporter constructs was delivered to plant tissues by *Agrobacterium*-mediated transfer using techniques known in the art.

As can be seen in **FIG**. **4**, each of these reporter constructs shows activity in the pineapple plants. Furthermore, as can be seen in all three measures of activity quantitation (number of plants with staining, number of spots, and number of blue spot clusters), any one reporter shows tissue-dependent variable activity. For example, the EHS promoters driving GUS expression showed activity in leaf and stem, but no activity or reduced activity in root. The Ubp1.3 promoter showed activity in all tissues tested. The EHS promoters EHS1.1 and EHS1.7 also showed activity in pineapple fruit (data not shown).

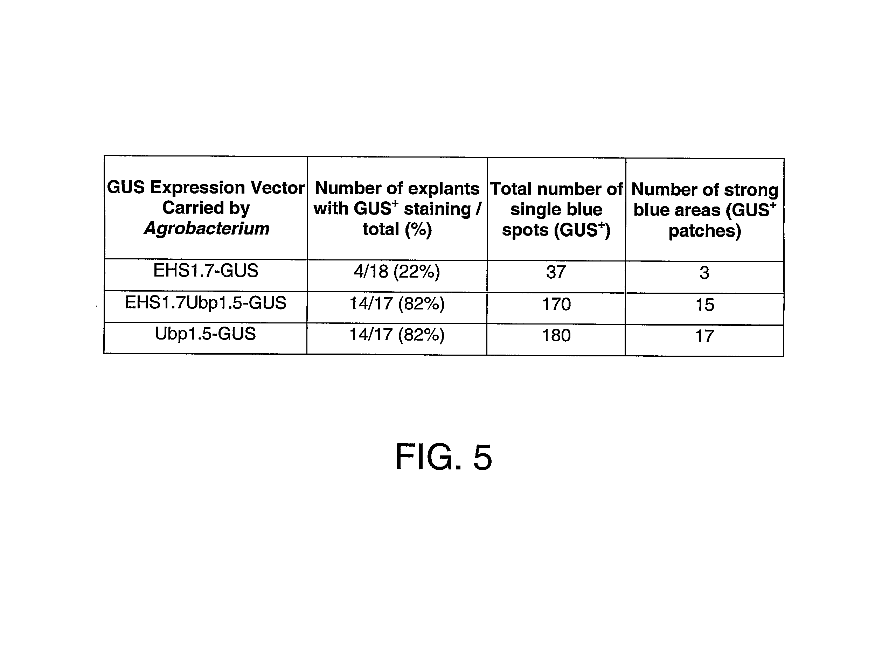

In **FIG. 5****,** the activity of various promoter constructs in Del Monte Gold™ MD-2 variety pineapple leaves is examined. This analysis included three reporters with three different promoter sequences. These were EHS1.7 (SEQ ID NO: 3), Ubp1.5 (SEQ ID NO: 5) and a fusion promoter consisting of both EHS1.7 and Ubp1.5 promoter sequences (fusion promoter SEQ ID NO: 6). As can be seen in **FIG. 5****,** each of these promoters is active in pineapple leaves.

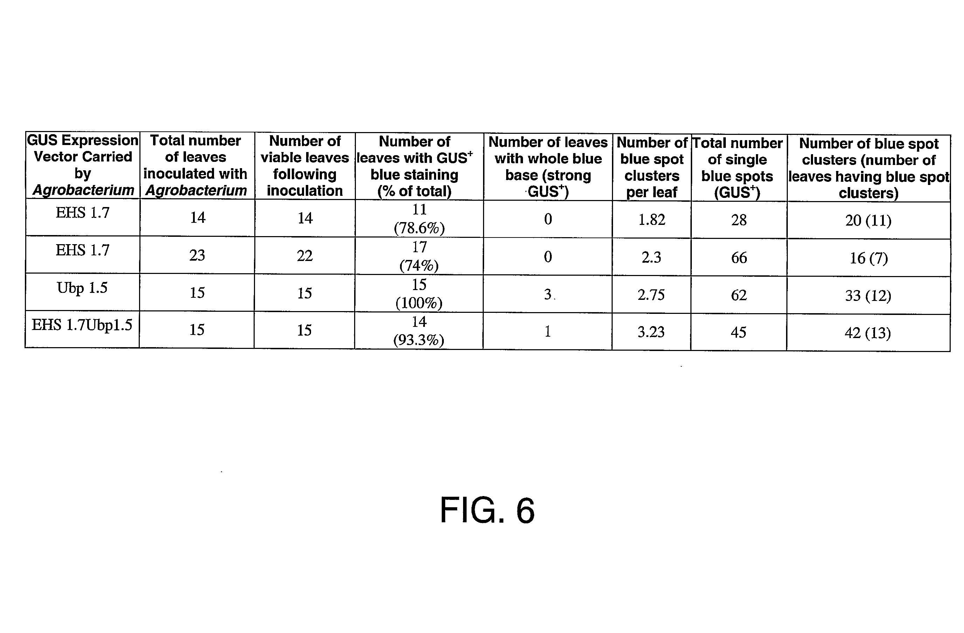

In **FIG. 6****,** the activity of various promoter sequences in Del Monte Gold™ MD-2 variety pineapple leaf explants is examined. This analysis included three reporters with three different promoter sequences. These were EHS1.7 (SEQ ID NO: 3), Ubp1.5 (SEQ ID NO: 5) and a fusion promoter consisting of both EHS1.7 and Ubp1.5 promoter sequences (fusion promoter SEQ ID NO: 6). The EHS1.7 reporter construct was used in a replicate experiment. As can be seen in **FIG. 6****,** each of these promoters is active in pineapple leaf explants.

### GUS ACTIVITY ASSAYS IN TOBACCO LEAF SECTIONS USING AGROBACTERIUM DELIVERY

The activities of various promoters of the invention used in GUS reporter constructs were assayed in tobacco leaf sections using a GUS activity assay following DNA delivery by *Agrobacterim*-mediated gene transfer using standard methods. Protocols for tobacco leaf transformation by *Agrobacterium* are well known in the art. See, e.g., Horsch et al., "Infection and transformation of leaf discs with Agrobacterium tumefaciens and regeneration of transformed plants," Science 227:1226-1231 (1985) and Plant Cell, Tissue and Organ Culture- Fundamental Methods (Springer Lab Manual), Gamborg and Phillips (editors), Springer-Verlag Telos, publ. 1995, see Chapter 15, pp.181-195 by Firoozabady and Kuehnle.

The tissues were assayed for GUS activity after three days of cocultivation with *Agrobacterium* containing the various reporter constructs that carry the EHS and Ubp promoters driving expression of the GUS gene product. Protocols for assaying GUS activity in tobacco leaf sections were adapted from Jefferson et al., EMBO J., 6:3901-3907 (1987).

The results of these assays are shown in Table 6.

**TABLE 6**

| **GUS expression vector construct carried by *Agrobacterium*** | **Total number of explants showing GUS⁺ staining following inoculation (total number of inoculated explants)** | **Total number of GUS⁺ single blue spots on all explants** | **Average number of GUS⁺ spots per explant** |
|---|---|---|---|
| EHS1.1-GUS | 11 (15) | 99 | 6.6 |
| EHS1.7-GUS | 13 (15) | 140 | 9.3 |
| Ubp1.3-GUS | 13 (17) | 60 | 3.5 |

As can be seen from this table, the EHS1.1, EHS1.7 and Ubp1.3 promoters (*i.e.,* SEQ ID NOS: 2, 3 and 4) are all clearly active in the tobacco explants. This result is significant in that it illustrates that these promoters can be used in multiple plant species, and indeed, in plant species that are divergent as monocotyledonous (pineapple) and dicotyledonous (tobacco) plants.

### EXAMPLE 11

### Promoter Activity Analysis Using DNA Delivery by Particle Bombardment

The activity of various reporter constructs comprising promoters/terminators of the invention in pineapple and pepper fruit was assessed by a GUS activity assay following nucleic acid delivery by particle bombardment. This example illustrates that the promoter/terminator sequences of the invention can be utilized in expression vectors that are delivered to plants by techniques in addition to *Agrobacterium* delivery, *e.g.,* using biolistics.

### GUS ACTIVITY ASSAYS IN PINEAPPLE FRUIT USING BIOLISTIC DELIVERY

Pineapple fruit has been used previously for testing gene expression by biolistics (Zhou et al., Plant Biotech. J., 1:463-4780 2003; which is incorporated by reference). Protocols for gene transfer in pineapple leaves, root and stem were adapted from known methods, *e.g*., Sanford et al., "Optimizing the biolistic process for different biological applications," Methods in Enzymology 217:483-509 (1993).

### Surface Sterilization of Pineapple Fruit

Pineapple fruit from an imported from a Costa Rica ornamental variety and Del Monte Gold™ MD-2 variety were used as targets for promoter activity analysis. Crown was removed and pineapple fruit was washed thoroughly in tap water with soap. The fruit was then soaked in or sprayed with 70 % (v/v) ethyl alcohol and dried for 1 to 2 minutes, followed by immersion in 2 % (v/v) NaOCl and incubated on the stirrer for 20 min with subsequent washing in DDW (double distilled water) three times. After washing, shell was cut off from the fruit and slices were made in transverse or longitudinal direction to the core of the fruit. Sections were made about 1-4 centimeter squares and 2-4 mm thick. Tissue pieces were blotted on filter paper, placed on the MSO medium solidified with 0.25 % Gelrite^{®}. The sections were arranged in the middle of 90 mm petri dishs, covering a circle area of 4 cm in diameter, and allowed to dry thoroughly for 30-45 min.

### Bombardment Conditions

Fruit pieces were bombarded using a particle delivery system (Bio-Rad^{®} Laboratories, Hercules, CA). Gold particles (Bio-Rad^{®}) particles with 1.0 µm diameter were used as microprojectiles. Bombardment was performed based on Sanford et al., "Optimizing the biolistic process for different biological applications," Methods in Enzymology 217:483-509 (1993), which is incorporated herein by reference. Seven shots were performed from each gold particles coating preparation.

The target (a 90 mm petri dish) was placed 7-10 cm from the point of particle discharge. Helium (working) pressure was tested in the range of 600 to 1350 psi and chamber vacuum pressure was 26.5-27 in Hg. Every target was bombarded either once or twice with the plasmid DNA (1 µg/µl) coated onto the 1 µm gold particles (60 mg/ml). After bombardment, all plates were placed for incubation in the growth room at 28°C ± 1°C.

DNA delivery was evaluated by GUS expression (Jefferson et al., EMBO J., 6:3901-3907 (1987); incorporated by reference). GUS expression was monitored 36 to 48 hours after bombardment. In order to suppress any endogenous GUS activity in plant tissues and to inhibit pathogen growth (e.g., bacteria and yeasts), 12 % (v/v) methanol was added to the basic GUS staining solution based on the protocol described in Kosugi et al., Plant Science 70:133-140 (1990), which is incorporated by reference. The reaction mixture was incubated for 12-16 hours at 37°C. All tissues were washed with 70 % (v/v) ethanol after removing the staining solution.

### Results

This bombardment DNA delivery used a GUS reporter construct driven by the Ubp1.5 promoter. Two ornamental pineapple fruits imported from Costa Rica and the Del Monte Gold™ MD-2 variety were tested. Transverse cuts of the fruits were used. Two red ornamental pineapple targets were used, while one MD-2 target was used. The two red ornamental pineapple targets showed a total of seven (7) GUS⁺ blue spots. The single MD-2 target showed one GUS⁺ blue spot. This experiment illustrates that the promoters Ubp1.5 promoter (SEQ ID NO: 5) can be used across multiple species, and furthermore, expression vectors that utilize the promoters/terminators of the invention can be used in conjunction with biolistic gene delivery.

### GUS ACTIVITY ASSAYS IN PINEAPPLE LEAVES USING BIOLISTIC DELIVERY

A similar experiment as described above was conduced using a GUS reporter vector driven by the EHS1.1 promoter(SEQ ID NO: 2) in binary vector backbone pCambia-1300 and using pineapple leaves from Del Monte Gold™ MD-2 variety as the biolistic target. Leaves were prepared essentially as they were prepared for Agrobacterium inoculation, e.g., as described in International Patent Application Publication No. WO 2004/053082. Two bombardment shots per used for each plate. A total of 68 young leaves were bombarded with DNA. Of those 68 leaves, 13 showed GUS⁺ blue spots. A total of 75 GUS⁺ blue spots were counted on all leaves.

This demonstrates that the promoters/terminators of the invention, e.g., EHS1.1 promoter (SEQ ID NO: 2), can be used to express genes in multiple tissues (e.g., leaves, fruit, root, stem), and furthermore, demonstrates that expression vectors that utilize the promoters/terminators of the invention can be used in conjunction with biolistic gene delivery.

### GUS ACTIVITY ASSAYS IN PEPPER FRUITS USING BIOLISTIC DELIVERY

A experiment using biolistic delivery of a GUS reporter vector driven by the EHS1.1 promoter (SEQ ID NO: 2) into a dicotyledonous bell pepper fruit was undertaken. Various biolistic conditions were utilized.

### Surface Sterilization of Bell Pepper Fruit

Yellow or dark green ripe bell pepper fruit was used in the biolistics and promoter activity assay. The bell pepper fruit was washed with soap, then sprayed with 70% ethanol, washed in 2% (v/v) NaOCl for 2 minutes with subsequent washing in double distilled water. Several pieces were cut from the fruit and placed on MSO solid medium with the endocarp on the medium surface and with removed exocarp side up. Cut pieces were allowed to dry thoroughly on the opened plate in a laminar flow hood for 40-50 minutes.

The GUS reporter gene cassette EHS 1.1-GUS-NOS driven by the EHS1.1 promoter (SEQ ID NO: 2) using the p-Cambia vector backbone was used in the analysis (reporter construct termed pHCWII-1).z

The particle bombardment was made at a distance of approximately 10 cm and at a pressures of 1350 psi. Two experimental condition were tested. These conditions are shown in Table 7.

**TABLE 7**

| | **Treatment** | **Number of GUS⁺ blue spots per target** |
|---|---|---|
| **Biolistic Condition 1** | The inner part of bell pepper fruit was mechanically exposed (without any further treatment of the inner part of the fruit). This exposed inner part of the fruit was the biolistic target surface. Biolistic targets were approx 2 cm x 2 cm sections (approx 2 or 3 sections total). Each experimental plate received two biolistic shots. | **4** |
| **Biolistic Condition 2** | Exocarp was removed from the bell pepper fruit. The exposed surface following exocarp removal was the biolistic target surface. Biolistic targets were approx 2 cm x 2 cm sections (approx 2 or 3 sections total). Each experimental plate received one biolistic shot. | **25** |

The effects of changing the biolistic psi on the expression of a reporter plasmid were also tested. The GUS reporter vector driven by the EHS1.1 promoter (SEQ ID NO: 2) was used in the analysis. Each plate received a single biolistic bombardment. The GUS expression results following two different psi values were tested, adn the results are shown in Table 8.

**TABLE 8**

| **Pressure, PSI** | **Number of GUS⁺ blue spots per target** |
|---|---|
| 1100 | 45 |
| 1350 | 24 |

These experiments using biolistic gene delivery in the dicot bell pepper illustrate that the promoters/terminators of the invention, e.g., EHS1.1 promoter (SEQ ID NO: 2), can be used to express genes in a wide range of species including monocot (e.g., pineapple) and dicot (e.g., bell pepper) fruit. Furthermore, this experiment again demonstates that expression vectors that utilize the promoters/terminators of the invention can be used in conjunction with biolistic gene delivery.

### EXAMPLE 12

### Expression Vector Backbone

A vector, termed pHCW, was constructed to facilitate *Agrobacterium-*mediated plant transformation. The pHCW vector uses a binary vector strategy, as known in the art. In the binary vector system, a plasmid containing the left and right borders of T-DNA from *Agrobacterium tumefaciens* Ti plasmid is generated, where the sequences to be transferred to the host plant cell is placed between the left and right borders. This plasmid is then transformed into *E*. *coli,* and in turn transferred by conjugation to an *A*. *tumefaciens* strain containing a helper plasmid that provides the *vir* functions. On infection of a plant, the activated *vir* functions recognize the left border sequence of the modified plasmid and transfers the DNA between the left and right borders to a plant chromosome.

Plasmid pHCW-5, shown in **FIG. 3****,** is a binary vector for plant transformation. As shown in **FIG. 3****,** RB and LB denote the T-DNA right and left borders respectively. Additional components of the plasmid include:

(i) the pVS1 replicon derived from *Pseudomonas aeruginosa,* which ensures replication in *Agrobacterium tumefaciens* (Itoh et al., "Genetic and molecular characterization of the Pseudomonas plasmid pVS1," Plasmid 11:206-220 [1984]);

(ii) a tetracycline resistance gene TetRA, which allows for selection of the binary plasmid in *Agrobacterium tumefaciens* and *Escherichia coli* (Waters et al., "The tetracycline resistance determinants of RP1 and Tn1721; nucleotide sequence analysis," Nucl. Acids Res., 11(17):6089-6105 [1983]);

(iii) the origin of replication from plasmid pACYC, which ensures replication in *Escherichia coli* (Chang et al., J. Bacteriology 134:1141-1156 [1979]); and

(iv) a multiple cloning site (MCS), to facilitate the introduction of different nucleic acids and nucleic acid cassettes.

### EXAMPLE 13

### Expression Vectors Used in the Production of Transgenic Pineapple Plants

Transgenic plants were generated using a variety of vectors containing genes and promoters/terminators of the present invention. These vectors are based on the pHCW vector backbone described in Example 12. Three examples of these expression constructs used to produce transgenic plants are described below.

### EHS1.2Ubp1.5-PPsyISO-Ubpter/EHS1.7Ubp1.5-ALS-ALS3'

This is an *Agrobacterium-vector* carrying a large cassette consisting of two subcassettes. In one subcassette, a fusion promoter (SEQ NO: 12) comprising the EHS1.2 sequence and the Ubp1.5 sequence drives the expression of PPsyISO, which is a tandem arrangement of the pineapple phytoene synthase (PSY) cDNA (SEQ ID NO: 11) and the pineapple carotenoid isomerase (ISO) cDNA (SEQ ID NO: 1), to produce the two respective recombinant proteins ISO and PSY (SEQ ID NOS: 9 and 13; respectively). Expression of the fusion protein is stabilized by the addition of the ubiquitin terminator sequence Ubpter (SEQ ID NO: 10).

In the second subcassette, a fusion promoter comprising the EHS 1.7 sequence and the Ubp1.5 sequence (fusion promoter SEQ ID NO: 6) drives the expression of the gene encoding acetolactase synthase (ALS), which confers resistance to chlorsulfuron (CS). Expression of the protein is stabilized by the addition of terminator sequence, ALS3', derived from the ALS gene (see, US Patent Serial No. 5,378,824 to Bedbrook et al.).

### EHS1.7-LYC-RNAi-Ubpter/EHS1.7Ubp1.5-ALS-ALS3'

This is a dual nucleic acid cassette. In one subcassette, a fusion promoter comprising the EHS 1.7 promoter sequence (SEQ ID NO: 3) drives the expression of a pineapple lycopene β-cyclase, (LYC) RNAi cassette similar to that described in Example 9. The present vector consists of the following, in linear order: (i) the EHS 1.7 promoter (SEQ ID NO: 3), (ii) lycopene β-cyclase, (LYC) gene sequence in the sense orientation; (iii) the Ls1 gene intron from potato that forms a spliceable intron loop; (iv) lycopene β-cyclase (LYC) gene sequence in the antisense orientation; and (v) ubiquitin terminator sequence Ubpter (SEQ ID NO: 10).

In the second subcassette, as described above, a fusion promoter comprising the EHS1.7 and Ubp1.5 sequences (fusion promoter SEQ ID NO: 6) drives the expression of the gene encoding ALS, which confers resistance to chlorsulfuron. Expression of the protein is stabilized by the addition of terminator sequence, ALS3', derived from the ALS gene.

### Ubp1.5-ALS-ALS3'/Ubp1.5-LYC-RNAi-Ubpter

This is another dual nucleic acid cassette. In the first subcassette, a promoter comprising the Ubp1.5 sequence (SEQ ID NO: 5) drives the expression of the ALS gene product. Expression of the protein is stabilized by the addition of terminator sequence, ALS3', derived from the ALS gene.

In the second subcassette, the Ubp1.5 sequence (SEQ ID NO: 5) drives the expression of a pineapple lycopene β-cyclase (LYC) RNAi cassette consisting of LYC sense and antisense sequences separated by an intervening potato Ls1 gene intron, followed by the addition of the ubiquitin terminator sequence Ubpter (SEQ ID NO: 10).

### EXAMPLE 14

### Production of Transgenic Pineapple Plants

Using vectors containing genes and promoters/terminators of the present invention, transgenic plants were produced as described in WO 2004/053082, or alternatively, according to the methods described below. For example, *Agrobacterium* carrying one of three expression constructs (described in Example 13) were used to generate transgenic pineapple plants.

In some of the protocols used herein to establish transgenic plants, the establishment of pineapple shoot cultures, preparation of source tissues for inoculation with *A. tumefaciens,* pretreatment of explants for inoculation with *Agrobacterium, Agrobacterium* culture and preparation, cocultivation, recovery, selection, plant regeneration, and micropropagation were all done essentially as described in International Patent Application Publication WO 2004/053082, except for the modifications described below.

### MODIFIED CULTURE REGIME

In some plant establishment protocols (for example, the protocols used to produce transgenic plants with the vectors described in Example 13), a culture regime outlined un Table 9 was used.

**TABLE 9**

| **Stage of production of transgenic plants** | **Liquid Culture Medium Liquid** | **Number of days** |
|---|---|---|
| Cocultivation with Agrobacterium | ½MST2.2I.1As500PVP1 | 3 |
| Recovery 1 | ½MST2.2I.1C500PVP1 | 4 |
| Recovery 2 | T2.2I.1PVP1C500 | 7 |
| Selection I | T2.2I.1PVP1C500CS.5 | 25 |
| Selection II | B1PVP1CEF300CS1 | 20 |
| Selection III | B1CEF200V100PVP1CS2 | 21 |
| Selection IV | B1CEF100V100AA.2CS3 | 20 |
| Selection V | B1CEF100V100AA.2CS5 | 20 |
| Selection VI | B1V50CS10 liquid* | 21 |
| Micropropagation | B.5CS10 liquid* | 21 |
| Rooting | N.51.5 liquid | 21 |
| * Culture medium is changed every 3 weeks. | | |

### RECOVERY, SELECTION AND PLANT REGENERATION.

Recovery was done in two steps; first, on ½ MST2.2I.1 PVP1C500 for 4-5 days; second, on T2.2I.1PVP1C500 for an additional 4-5 days. The explants were transferred to selection media, as shown, to produce fully transgenic plants. Regeneration efficiency of leaves under selection using the Ubp1.5-ALS-ALS3'/Ubp1.5-LYC-RNAi-Ubpter construct described in Example 13 is shown in Table 10.

**TABLE 10**

| initial no. of leaf explants | no. of shoots generated after 20 weeks in culture | total no. of shoots regenerated after 25 weeks | regeneration efficiency while under selection | no. of explants that regenerated shoots in 25 weeks (%) |
|---|---|---|---|---|
| 38 | 6 | 23 | 60% | 18 (47) |
| | | | | |
| **Control tissues not treated with *Agrobacterium*** | | | | |
| 24 | 0 | 0 | 0 | 0 |

Thus, as seen in Table 10, the nucleic cid sequences of the invention can be used to generate transgenic plants.

### EXAMPLE 15

### Media Formulations

Generally, culture media are designated with letters and numbers; letters referring to the media components used, followed by a number indicating the concentration of the particular component. For example, B2N2 is an MS media that contains 6-benzylamino purine (BA or B) and α-naphthalene acetic acid (NAA or N). More specific details relating to the media compositions referred to in these examples, including component concentrations, are provided below.

For tissue culture media, the pH should generally be in the range of about 5 to about 7.5, preferably about 5.6. The medium is used following sterilization by autoclaving, except for specific components that are filter-sterilized and then added after autoclaving.

**TABLE 11**

| **Abbrevations** | |
|---|---|
| **Abbreviation** | **Component** |
| AA | Ascorbic acid |
| B or BA | 6-Benzyladenine (6-Benzylaminopurine) |
| C or Carb | Carbenicillin |
| Cef | Cefotaxime |
| Cs | Chlorsulfuron |
| I or IBA | Indolebutyric acid |
| N or NAA | α-Naphthaleneacetic acid |
| PVP | Polyvinylpyrrolidone |
| T or TDZ | Thidiazuron |
| V or Vanc | Vancomycin |

### MS or MSO

| | |
|---|---|
| MS salts | 1X |
| B5 | 1X |
| vitaSucrose | 30 g/lmins |
| MES | 600 mg/l |
| Gelrite® | 2.5 g/l |
| pH | 5.7 |

### B1.5N.5

MS medium supplemented with:

| | |
|---|---|
| BA | 1.5mg/l |
| NAA | 0.5 mg/l |

### B1

MS medium supplemented with:

| | |
|---|---|
| BA | 1 mg/l |

### B3N.2

MS medium supplemented with:

| | |
|---|---|
| BA | 3 mg/l |
| NAA | 0.2 mg/l |

### N.5I.5 LIQUID

MS medium without Gelrite®, supplemented with:

| | |
|---|---|
| NAA | 0.5 mg/l |
| IBA | 0.5 mg/l |

### ½MST2.2I.1As500PVP1

½ MS salts and sugar, supplemented with:

| | |
|---|---|
| PVP | 1g/l |
| TDZ | 2.2 mg/l |
| IBA | 0.1 mg/l |
| Acetosyringone | 500 µM |

### T2.2I.1PVP1C500

MS medium supplemented with:

| | |
|---|---|
| PVP | 1g/l |
| TDZ | 2.2 mg/l |
| IBA | 0.1 mg/ |
| Carb | 500 mg/l |

### T2.2I.1PVP1C500CS.5

T2.2I.1PVP1C500 medium supplemented with:

| | |
|---|---|
| Chlorsulfuron | 0.5 µg/l |

### ½MST1I1.C500PVP1

MST1I0.1C500PVP1 medium supplemented with:
½ MS salts

### ½MST2.2I.1As500PVP1

T2.2I.1PVP1C500 medium supplemented with:
½ MS salts

### B1PVP1CEF300CS1

B1 medium supplemented with:

| | |
|---|---|
| PVP | 1g/l |
| Cef | 300 mg/l |
| PVP | 1 g/l |
| Chlorsulfuron | 1 µg/l |

### B1CEF200V100PVP1CS2

B1PVP1CEF300CS1 medium supplemented with:

| | |
|---|---|
| Cef | 200 mg/l |
| Vanc | 100 mg/l |
| Cs | 2 µg/l |

### B1CEF100V100AA.2CS3

B1CEF200V100PVP1CS2 medium supplemented with:

| | |
|---|---|
| Cef | 100 mg/l |
| Ascorbic acid | 0.2 mg/l |
| Cs | 3 µg/l |

### B1CEF100V100AA.2CS5

B1CEF100V100AA.2CS3 medium supplemented with:

| | |
|---|---|
| Cs | 5 µg/l |

### B1V50CS10

B 1 medium supplemented with:

| | |
|---|---|
| Vanc | 50 mg/l |
| Cs | 10 µg/l |

### B.5CS10

B 1 medium supplemented with:

| | |
|---|---|
| BA | 0.5 mg/l |
| Cs | 10 µg/l |

### N.5I.5

MSO medium supplemented with:

| | |
|---|---|
| ½ MS salts and sugar | |
| NAA | 0.5 mg/l |
| IBA | 0.5 mg/l |

While the foregoing invention has been described in some detail for purposes of clarity and understanding, it will be clear to one skilled in the art from a reading of this disclosure that various changes in form and detail can be made without departing from the true scope of the invention. For example, all the techniques and apparatus described above can be used in various combinations. All publications, patents, patent applications, and/or other documents cited in this application are incorporated by reference in their entirety for all purposes to the same extent as if each individual publication, patent, patent application, and/or other document were individually indicated to be incorporated by reference for all purposes.
The following numbered paragraphs (paras.) contain further statements of various aspects of the present invention:-
1. An isolated or recombinant nucleic acid comprising a polynucleotide sequence selected from the group consisting of:
   (a) a polynucleotide sequence of SEQ ID NO: 1-8, 10-12, 14, 16, 17, 93, complements thereof or unique subsequences thereof;
   (b) a polynucleotide sequence that hybridizes under highly stringent conditions to substantially an entire length of the polynucleotide sequence of (a); and
   (c) a polynucleotide sequence encoding a polypeptide comprising an amino acid sequence corresponding to: (i) SEQ ID NO: 9, 13 or 15, (ii) a conservative variant of SEQ ID NO: 9, 13 or 15, (iii) a unique subsequence of SEQ ID NO: 9, 13 or 15; or (iv) a polypeptide having at least 90% sequence identity with SEQ ID NO: 9, 13 or 15.
2. The nucleic acid of para. 1(a), wherein said polynucleotide sequence of SEQ ID NO: 2-8, 10, 12 or 16 comprises transcription regulatory activity.
3. The nucleic acid of para. 2, wherein said transcription regulatory activity comprises transcription promoter activity or transcription terminator activity.
4. The nucleic acid of para. 1(c), wherein said polypeptide comprises carotenoid isomerase activity, phytoene synthase activity or lycopene β-cyclase activity.
5. The polypeptide encoded by the polynucleotide sequence of para. 1(c).
6. A host cell comprising the nucleic acid of para. 1.
7. The host cell of para. 6, wherein said host cell is selected from a bacterial cell and a plant cell.
8. An expression cassette comprising the nucleic acid of para. 1.
9. A vector comprising the nucleic acid of para. 1.
10. The vector of para. 9, wherein said vector complises a plasmid or a virus.
11. The vector of para. 9, wherein said vector is an expression vector.
12. A method of producing a transformed plant cell, the method comprising transforming said plant cell using said vector of para. 9.
13. A plant cell transformed with a recombinant vector comprising a nucleic acid comprising a polynucleotide sequence selected from the group consisting of:
   (a) a polynucleotide sequence of SEQ ID NO: 1-8, 10-12, 14, 16, 17, 93, complements thereof or unique subsequences thereof;
   (b) a polynucleotide sequence that hybridizes under highly stringent conditions to substantially an entire length of the polynucleotide sequence of (a); and
   (c) a polynucleotide sequence encoding a polypeptide comprising an amino acid sequence corresponding to: (i) SEQ ID NO: 9, 13 or 15, (ii) a conservative variant of SEQ ID NO: 9, 13 or 15, (iii) a unique subsequence of SEQ ID NO: 9, 13 or 15; or (iv) a polypeptide having at least 90% sequence identity with SEQ ID NO: 9, 13 or 15.
14. A plant comprising said transformed plant cell of para. 13.
15. The plant cell of para. 13, wherein said plant cell is a monocotyledonous plant cell.
16. The plant cell of para. 13, wherein said plant cell is a pineapple cell.
17. The plant cell of para. 13, wherein said plant cell is a dicotyledonous plant cell.
18. The plant cell of para. 13, wherein said plant cell is derived from a plant selected from the genera: *Ananas, Musa, Vitis, Fragaria, Lotus, Medicago, Onobrychis, Trifolium, Trigonella, Vigna, Citrus, Carica, Persea, Prunus, Syragrus, Theobroma, Coffea, Linum, Geranium, Manihot, Daucus, Arabidopsis, Brassica, Raphanus, Sinapis, Atropa, Capsicum, Datura, Hyoscyamus, Lycopersicon, Nicotiana, Solanum, Petunia, Digitalis, Majorana, Mangifera, Cichorium, Helianthus, Lactuca, Bromus, Asparagus, Antirrhinum, Heterocallis, Nemesia, Pelargonium, Panicwn, Pennisetum, Ranunculus, Senecio, Salpiglossis, Cucurbita, Cucumis, Browaalia, Lolium, Malus, Apium, Gossypium, Vicia, Lathyrus, Lupinus, Pachyrhizus, Wisteria, Stizolobium, Agrostis, Phleum, Dactylis, Sorgum, Setaria, Zea, Oryza, Triticum, Secale, Avena, Hordeum, Saccharum, Poa, Festuca, Stenotaphrum, Cynodon, Coix, Olyreae, Phareae, Glycine, Pisum, Psidium, Passiflora, Cicer, Phaseolus, Lens,* and *Arachis.*
19. The plant cell of para. 13, wherein said nucleic acid comprises the polynucleotide sequence of (c), and wherein said transformed plant cell expresses the polypeptide encoded by the polynucleotide sequence of (c).
20. The plant cell of para. 19, wherein said expressed polypeptide modifies accumulation of one or more carotenoid in the transformed plant cell
21. The plant cell of para. 20, wherein said plant cells shows increased levels of β-carotene or lycopene.
22. The plant cell of para. 13, wherein said nucleic acid comprises the polynucleotide sequence of (a), (b), or (c), wherein said polynucleotide sequence homologously recombines with at least one promoter of at least one endogenous gene in the transformed plant cell.
23. The plant cell of para. 13, wherein said nucleic acid comprises the polynucleotide sequence of (a) or (b) operably linked to a nucleic acid segment that encodes an RNA molecule and/or a protein molecule, wherein said polynucleotide of (a) is selected from a polynucleotide sequence of SEQ ID NO: 2-8, 10, 12, 16, complements thereof or unique subsequences thereof.
24. The plant cell of para. 23, wherein said nucleic acid segment confers resistance on the transformed cell to one or more of: insects, drought, nematodes, viral disease, bacterial disease or herbicides.
25. The plant cell of para. 23, wherein said nucleic acid segment comprises at least one sense nucleic acid segment that corresponds to at least a portion of at least one endogenous gene.
26. The plant cell of para. 23, wherein said nucleic acid segment comprises at least one sense nucleic acid segment that corresponds to at least a portion of at least one exogenous gene.
27. The plant cell of para. 23, wherein said nucleic acid segment comprises at least one antisense nucleic acid segment that corresponds to at least a portion of at least one endogenous gene.
28. The plant cell of para. 23, wherein said nucleic acid segment encodes at least one polypeptide transcription factor.
29. A method of producing a vector, the method comprising operably linking an isolated or recombinant nucleic acid to an extra-chromosomal element, said nucleic acid comprising a polynucleotide sequence selected from the group consisting of:
   (a) a polynucleotide sequence of SEQ ID NO: 1-8, 10-12, 14, 16, 17, 93, complements thereof or unique subsequences thereof;
   (b) a polynucleotide sequence that hybridizes under highly stringent conditions to substantially an entire length of the polynucleotide sequence of (a); and
   (c) a polynucleotide sequence encoding a polypeptide comprising an amino acid sequence corresponding to: (i) SEQ ID NO: 9, 13 or 15, (ii) a conservative variant of SEQ ID NO: 9, 13 or 15, (iii) a unique subsequence of SEQ ID NO: 9, 13 or 15; or (iv) a polypeptide having at least 90% sequence identity with SEQ ID NO: 9, 13 or 15.
30. The method of para. 29, wherein said extrachromosomal element is capable of replication in a bacterial cell or a plant cell.

## Claims

1. An isolated or recombinant nucleic acid comprising a polynucleotide sequence selected from the group consisting of:
(a) a polynucleotide sequence of SEQ NO: 16 or a complement or conservative variant thereof;
(b) a polynucleotide sequence that hybridizes under highly stringent conditions to substantially the entire length of a polynucleotide sequence of (a);
(c) a polynucleotide sequence that comprises at least 100 contiguous nucleotides of SEQ NO: 16; and,
(d) a polynucleotide sequence that comprises a subsequence having at least 80% sequence identity to the polynucleotide of (a) or (c).

2. The isolated or recombinant nucleic acid of claim 1, wherein the polynucleotide sequence comprises transcription regulatory activity and/or transcription promoter activity.

3. The isolated or recombinant nucleic acid of claim 1 or claim 2, wherein said isolated or recombinant nucleic acid comprises a sequence that encodes a polypeptide comprising carotenoid isomerase activity, phytoene synthase activity or, lycopene β-cyclase activity.

4. The isolated or recombinant nucleic acid of claim 1, wherein the nucleic acid comprises a first promoter sequence selected from the group consisting of:
(a) a polynucleotide sequence of SEQ ID NO: 16 or a complement or conservative variant thereof;
(b) a polynucleotide sequence that hybridizes under highly stringent conditions to substantially the entire length of a polynucleotide sequence of (a);
(c) a polynucleotide sequence that comprises at least 100 contiguous nucleotides of SEQ ID NO: 16; and
(d) a polynucleotide sequence that comprises a subsequence having at least 80% sequence identity to the polynucleotide of (a) or (c); and wherein the nucleic acid comprises a second promoter sequence selected from the group consisting of:
(e) a polynucleotide sequence of SEQ NO: 6 or a complement or conservative variant thereof;
(f) a polynucleotide sequence that hybridizes under highly stringent conditions to substantially the entire length of a polynucleotide sequence of (e);
(g) a polynucleotide sequence that comprises at least 100 contiguous nucleotides of SEQ ID NO: 6; and,
(h) a polynucleotide sequence that comprises a subsequence having at least 80% sequence identity to the polynucleotide of (e) or (g).

5. The isolated or recombinant nucleic acid of claim 4, wherein the first and second promoter sequences each comprises transcription promoter activity; wherein said SEQ ID NO: 16 directs expression of a first expressible nucleic acid encoded by the recombinant or isolated nucleic acid and said SEQ ID NO: 6 directs expression of a second expressible nucleic acid encoded by the recombinant or isolated nucleic acid when said isolated or recombinant nucleic acid is expressed in a cell.

6. The isolated or recombinant nucleic acid of claim 1 or claim 4, further comprising a polynucleotide sequence selected from the group consisting of:
a) a polynucleotide sequence of SEQ ID NO: 14 or a complement or conservative variant thereof;
b) a polynucleotide sequence that hybridizes under highly stringent conditions to substantially the entire length of a polynucleotide sequence of (a);
c) a polynucleotide sequence that comprises at least 100 contiguous nucleotides of SEQ ID NO: 14;
d) a polynucleotide sequence that encodes an amino acid sequence of SEQ ID NO: 15 or a conservative variant of SEQ ID NO: 15; and,
e) a polynucleotide sequence that comprises a subsequence having at least 80% sequence identity to the polynucleotide of (a) or (c).

7. The isolated or recombinant nucleic acid of any one of claims 1, 4, or 6, further comprising a polynucleotide sequence selected from the group consisting of:
a) a polynucleotide sequence of SEQ ID NO: 10 or a complement or conservative variant thereof;
b) a polynucleotide sequence that hybridizes under highly stringent conditions to substantially the entire length of a polynucleotide sequence of (a);
c) a polynucleotide sequence that comprises at least 100 contiguous nucleotides of SEQ ID NO: 10; and,
d) a polynucleotide sequence that comprises a subsequence having at least 80% sequence identity to the polynucleotide of (a) or (c).

8. The isolated or recombinant nucleic acid of claim 1, wherein the nucleic acid further comprises one or more nucleotide sequences selected from the group consisting of:
a) the sequence of SEQ ID NO: 1-8, 10-12, 14, 17, or 93;
b) a sequence that is at least 80% identical to at least 100 contiguous nucleotides of SEQ ID NO: 1-8, 10-12, 14, 17, or 93;
c) a nucleotide sequence that encodes an amino acid sequence of SEQ ID NO:9, SEQ ID NO: 13, or SEQ ID NO: 15 or conservative variants thereof; and
d) a sequence complementary to any one of (a)-(c) or a or conservative variant of (a) or (b).

9. A recombinant cell comprising the isolated or recombinant nucleic acid according to any one of claims 1 to 8.

10. The recombinant cell of claim 9, wherein the recombinant nucleic acid comprises a polynucleotide sequence that encodes a polypeptide that, when expressed in the recombinant cell, modifies accumulation of one or more carotenoid in the recombinant cell; wherein the cell shows increased levels of β-carotene or lycopene; or wherein the recombinant nucleic acid confers resistance on the cell to one or more of: insects, drought, nematodes, viral disease, bacterial disease, or herbicides.

11. The recombinant cell of claim 9, wherein the nucleic acid comprises at least one sense nucleic acid that corresponds to at least a portion of at least one endogenous gene; wherein the nucleic acid comprises at least one sense nucleic acid that corresponds to at least a portion of at least one exogenous gene; or wherein the nucleic acid comprises at least one antisense nucleic acid that corresponds to at least a portion of at least one endogenous gene.

12. The recombinant cell according to any one of claims 9 to 11, wherein the cell is a recombinant bacterial cell or a recombinant plant cell.

13. The recombinant cell of claim 12, wherein the plant cell is a monocotyledonous plant cell or a dicotyledonous plant cell.

14. The recombinant cell of claim 13, wherein the monocotyledonous plant cell is a pineapple cell.

15. The recombinant cell of claim 12, wherein the recombinant plant cell is derived from a plant selected from the genera: *Ananas, Musa, Vitis, Fragaria, Lotus, Medicago, Onobrychis, Trifolium, Trigonella, Vigna, Citrus, Carica, Persea, Prunus, Syragrus, Theobroma, Coffea, Linum, Geranium, Manihot, Daucus, Arabidopsis, Brassica, Raphanus, Sinapis, Atropa, Capsicum, Datura, Hyoscyamus, Lycopersicon, Nicotiana, Solanum, Petunia, Digitalis, Majorana, Mangifera, Cichorium, Helianthus, Lactuca, Bromus, Asparagus, Antirrhinum, Heterocallis, Nemesia, Pelargonium, Panicum, Pennisetum, Ranunculus, Senecio, Salpiglossis, Cucurbita, Cucumis, Browaalia, Lolium, Malus, Apium, Gossypium, Vicia, Lathyrus, Lupinus, Pachyrhizus, Wisteria, Stizolobium, Agrostis, Phleum, Dactylis, Sorgum, Setaria, Zea, Oryza, Triticum, Secale, Avena, Hordeum, Saccharum, Poa, Festuca, Stenotaphrum, Cynodon, Coix, Olyreae, Phareae, Glycine, Pisum, Psidium, Passiflora, Cicer, Phaseolus, Lens,* and *Arachis.*

16. The recombinant cell according to any one of claims 12 to 15, wherein said recombinant cell is a recombinant plant cell and wherein the isolated or recombinant nucleic acid homologously recombines with at least one promoter of at least one endogenous gene in the transformed plant cell.

17. An expression cassette comprising the isolated or recombinant nucleic acid according to any one of claims 1 to 8.

18. A vector comprising the isolated or recombinant nucleic acid according to any one of claims 1 to 8 or the expression cassette of claim 17.

19. The vector of claim 18, wherein the vector comprises a plasmid or a virus and/or wherein said vector is an expression vector.

20. A plant comprising the recombinant cell according to any one of claims 12 to 16 wherein said recombinant cell is a recombinant plant cell; a plant comprising the expression cassette of claim 17; or a plant comprising the vector according to claim 18 or 19.
